# EUROPÄISCHE PATENTSCHRIFT

(11) **EP 2 723 344 B1**
(45) Veröffentlichungstag und Bekanntmachung des Hinweises auf die Patenterteilung: **07.10.2015**
(21) Anmeldenummer: 12756362.5
(22) Anmeldetag: 19.08.2012
(51) Int. Cl.: A61P 1/00, A61P 11/00, A61P 11/06, A61K 31/045, A61K 31/137, A61K 31/164, A61K 31/35, A61K 31/4174, A61K 31/4178, A61K 31/505, A61K 31/573, A61K 31/592, A61K 31/593, A61K 31/728

(54) **MONOTERPENHALTIGES KOMBINATIONSTHERAPEUTIKUM**
MONOTERPENE-CONTAINING AGENT FOR COMBINATION THERAPY
AGENT DE POLYTHÉRAPIE CONTENANT DES MONOTERPÈNES

(30) Priorität: 19.08.2011 DE 102011111111
(43) Veröffentlichungstag der Anmeldung: 30.04.2014
(73) Patentinhaber: Maria Clementine Martin Klosterfrau Vertriebsgesellschaft mbH, 50670 Köln (DE)
(72) Erfinder: GREVE, Harald, 40545 Düsseldorf (DE)
(74) Vertreter: Strehlke, Ingo Kurt
(86) Internationale Anmeldenummer: PCT/EP2012/003523
(87) Internationale Veröffentlichungsnummer: WO 2013/026556

(56) Entgegenhaltungen:
- WO-A1-01/46132
- WO-A1-2009/032887
- WO-A1-2010/025821
- WO-A2-2007/036802
- WO-A2-2008/039409

## Beschreibung

Die vorliegende Erfindung betrifft das medizinische Gebiet der prophylaktischen bzw. therapeutischen Behandlung von insbesondere entzündlichen Atemwegserkrankungen oder von insbesondere entzündlichen Erkrankungen des Verdauungstraktes.

Insbesondere betrifft die vorliegende Erfindung ein Kombinationstherapeutikum, welches sich insbesondere zur Verwendung bei der prophylaktischen bzw. therapeutischen Behandlung von insbesondere entzündlichen Atemwegserkrankungen, insbesondere bronchopulmonalen Erkrankungen, oder zur Verwendung bei der prophylaktischen bzw. therapeutischen Behandlung von insbesondere entzündlichen Erkrankungen des Verdauungstraktes, insbesondere des Darms, eignet.

Darüber hinaus betrifft die vorliegende Erfindung die Verwendungen des erfindungsgemäßen Kombinationstherapeutikums, insbesondere für die vorgenannten Anwendungen.

Schließlich betrifft die vorliegende Erfindung einen Vitamin D-Rezeptoragonisten (VDA) zur Verwendung bei der prophylaktischen bzw. therapeutischen Behandlung von Entzündungen bzw. von Erkrankungen der Atemwege bzw. von entzündlichen Erkrankungen des Verdauungstraktes, insbesondere des Darms.

Unter dem Begriff "Atemwegserkrankungen", wie er auch im Rahmen der vorliegenden Erfindung verwendet wird, ist eine allgemeine Bezeichnung zu verstehen, welche sämtliche, insbesondere entzündliche Erkrankungen der oberen wie unteren Atemwege bezeichnet, wobei hierunter akute wie chronische Krankheitszustände zu subsumieren sind. Beispiele für Atemwegserkrankungen der oberen Atemwege sind z. B. Entzündungen der Nebenhöhlen (z. B. Rhinosinusitis), während Beispiele für Atemwegserkrankungen der unteren Atemwege z. B. Asthma bronchiale, Bronchitis und COPD sind.

Der Begriff "bronchopulmonale Erkrankungen", wie er gleichermaßen auch im Rahmen der vorliegenden Erfindung verwendet wird, ist eine generische Bezeichnung insbesondere für sämtliche entzündliche wie nichtentzündliche Erkrankungen der unteren Atemwege (d. h. der bronchialen wie pulmonalen Atemwege), hierunter insbesondere Asthma bronchiale, Bronchitis sowie chronische obstruktive Lungenerkrankungen (sogenannte "COPD" bzw. "Chronic Obstructive Pulmonary Disease"), und wird im Rahmen der vorliegenden Erfindung synonym zu dem Begriff der sogenannten "Atemwegserkrankungen der unter Atemwege" verwendet.

Asthma bronchiale, oft vereinfachend auch nur als Asthma bezeichnet, ist eine chronische entzündliche Erkrankung der Atemwege mit dauerhaft bestehender bronchialer Überempfindlichkeit bzw. Hyperreaktivität, Entzündung der Bronchien sowie mangelnder bronchialer Reinigung (Clearance), wobei infolge der Bronchialobstruktion anfallsweise Atemnot auftreten kann, wobei grundsätzlich zwischen nichtallergischem (intrinsischem) und allergischem (extrinsischem) Asthma differenziert wird; daneben kennt man sowohl Mischtypen von allergischem und nichtallergischem Asthma als auch Mischtypen von Asthma und COPD. Je nach Schweregrad und den damit einhergehenden Krankheitssymptomen klassifiziert man gemäß den sogenannten Leitlinien nach GINA 2006 ("Global Initiative for Asthma") die verschiedenen Asthma-Krankheitsstadien von GINA I bis IV, wobei die Stufe 1 intermittierendes Asthma, die Stufe 2 leichtgradiges Asthma, die Stufe 3 mittelgradiges Asthma und schließlich die Stufe 4 schweres Asthma bezeichnet. In der neuen Klassifikation (GINA 2007) wird dagegen die Asthmakontrolle in den Vordergrund gestellt, und es wird somit unterschieden zwischen kontrolliertem, partiell kontrolliertem und unkontrolliertem Asthma.

Als Bronchitis wird dagegen allgemein die Entzündung der Bronchien, insbesondere der Bronchialschleimhaut, bezeichnet, wobei zwischen akuter Bronchitis einerseits und chronischer Bronchitis andererseits unterschieden wird. Die chronische Bronchitis ist gemäß Weltgesundheitsorganisation (WHO) definiert als Husten und Auswurf an den meisten Tagen während mindestens drei Monaten in zwei aufeinanderfolgenden Jahren und gehört zu den häufigsten chronischen Erkrankungen überhaupt (ca. 15 bis 25 %) mit infolgedessen großer Relevanz aus gesundheitsökonomischer Sicht. Im Fall der chronisch obstruktiven Bronchitis besteht eine dauerhafte Bronchialobstruktion, die sich meistens aus einer chronischen Bronchitis entwickelt.

Der Begriff der chronischen obstruktiven Lungenerkrankung bzw. COPD ("*Chronic Obstructive Pulmonary Disease"),* wie er auch im Rahmen der vorliegenden Erfindung verwendet wird, stellt einen Sammelbegriff für die chronisch obstruktive Bronchitis und das Lungenemphysem dar, wobei der Begriff "obstruktiv" das typische Merkmal der dauerhaften Bronchialverengung charakterisiert. Dabei klassifiziert man je nach Schweregrad und den damit einhergehenden Krankheitssymptomen gemäß den sogenannten Leitlinien nach GOLD ("*Global Initiative for Chronic Obstructive Lung Disease"*) die verschiedenen COPD-Krankheitsstadien von GOLD I bis IV.

Bei der Behandlung von bronchopulmonalen Erkrankungen, insbesondere von Atemwegserkrankungen der vorgenannten Art, kommen bei mildem bis mittlerem Schweregrad oftmals topische bzw. lokale, insbesondere inhalative bzw. inhalierbare Atemwegstherapeutika zum Einsatz. Hierzu zählen beispielsweise inhalative Bronchodilatatoren und Bronchospasmolytika, wie inhalative beta-2-Sympathomimetika, inhalative Anticholinergika, inhalative Kortikosteroide oder dergleichen. Infolge ihrer nur topischen bzw. lokalen Wirkung können die vorgenannten inhalativen Atemwegstherapeutika oftmals nicht die gewünschte therapeutische Wirkung entfalten, so dass zumeist relativ hohe Dosismengen verabreicht werden müssen, um den gewünschten therapeutischen Erfolg zu erzielen, oder aber in schwerwiegenderen Fällen systemische Therapeutika, insbesondere auf Kortikosteroidbasis, bedarfsweise oder sogar dauerhaft hinzugezogen werden müssen. Infolge der hohen einzusetzenden und erforderlichen Dosismengen werden zudem vielfach unerwünschte Nebenwirkungen beobachtet. Bisweilen wird auch eine nicht ausreichende Sensitivität der vorgenannten inhalativen Atemwegstherapeutika in Bezug auf die zu behandelnden Atemwegserkrankungen beobachtet. Schließlich ist mit einer rein topischen, insbesondere inhalativen Therapierung der vorgenannten Atemwegserkrankungen insbesondere der Nachteil verbunden, dass die Anwendung inhalativer Atemwegstherapeutika nicht immer zu einer ausreichenden Deposition in den peripheren Atemwegen führt, da kleinste Atemwege, wie beispielsweise die terminalen und respiratorischen Bronchioli, im Allgemeinen durch eine inhalative Therapie nicht erreicht werden können, insbesondere bei Einschränkungen in der Lungenfunktion, wie z. B. bei schwerer COPD.

So kommen beispielsweise sowohl beim Asthma bronchiale als auch bei der COPD - neben einer Grundtherapie mit sogenannten Bronchodilatatoren - ab einem gewissen Schweregrad auch topische bzw. lokale, insbesondere inhalative bzw. inhalierbare Kortikosteroide zum Einsatz. Daneben kommen bei schwerem Asthma bronchiale entsprechend GINA IV zusätzlich auch systemische, insbesondere perorale Kortikosteroide zum Einsatz.

Inhalierbare bzw. inhalative Kortikosteroide, insbesondere inhalierbare bzw. inhalative Glukokortikoide, welche synonym auch als "Inhalative Corticosteroids" oder einfach nur mit dem Akronym "ICS" bezeichnet werden, gehören zu den wichtigsten Therapeutika zur topischen bzw. lokalen, insbesondere inhalativen Behandlung entzündlicher Atemwegserkrankungen, insbesondere bei Asthma bronchiale und COPD. Das Wirkprinzip besteht bei regelmäßiger Inhalation in einer primär topischen bzw. lokalen Deposition in den Atemwegen, verbunden mit einer zugleich effektiven Entzündungshemmung durch relativ kleine Steroidmengen.

Auf lokaler bzw. topischer Ebene reduzieren inhalierbare Kortikosteroide, insbesondere Glukokortikoide, die Atemwegsentzündung durch die Hemmung von Zytokinen und Arachidonsäure-Metaboliten (AA-Metaboliten), welche aus aktivierten Atemwegsepithelzellen und distalen, die Atemwege auskleidenden, sogenannten alveolaren Makrophagen freigesetzt werden. In Abhängigkeit von der inhalierten Noxe und der genetischen Disposition gelangen durch die verschiedenen, vorgenannten chemotaktisch und vasodilatierend wirkenden Mediatoren unterschiedliche weiße Blutzellen in die Atemwege und verursachen eine entweder eosinophile Zellinfiltration im Fall des Asthma bronchiale oder eine primär granulozytäre Zellinfiltration im Fall der COPD. Diese Zellinfiltration ist als wichtige Determinante zur Entwicklung der Atemwegsentzündung bei Asthma bronchiale und der Bronchitis bekannt.

Nach den aktuellen internationalen und nationalen Therapierichtlinien werden jedoch ICS nicht in den Frühstadien der Atemwegserkrankungen eingesetzt, sondern insbesondere erst bei mildem persistierendem Asthma (GINA II) und bei mittel- bis schwergradiger COPD (GOLD III und IV), wobei zumeist eine dauerhafte Therapie erforderlich ist. Mit dieser anerkannten Therapiestrategie werden die zunehmend bekannten Nebenwirkungen von ICS sowie eine nicht ausreichende Sensitivität von ICS bei COPD berücksichtigt. Aus diesem Grunde bleibt die Therapie mit ICS nur der mittel- und schwergradigen COPD vorbehalten, die jedoch nicht den Progress bei COPD, sondern nur die Abnahme von Exazerbationen beeinflussen kann.

Naturgemäß ist für den klinischen Erfolg einer topischen Therapie mit inhalativen Kortikosteroiden, insbesondere Glukokortikoiden, der Grad der Deposition und insbesondere auch die Steroidverteilung in den peripheren Atemwegen von direkter Bedeutung. Aber selbst bei optimalem Einsatz verschiedenster Atemhilfen für Dosieraerosole und Pulverpräparate ist der Therapieerfolg nicht nur durch die Fähigkeit des Patienten, optimal zu inhalieren, sondern vielmehr primär durch das Inhalationsprinzip aufgrund der nicht ausreichenden Behandlung peripherer Atemwege limitiert. Wesentliche Ursachen hierfür sind somit die nicht ausreichende Steroiddeposition in den kleinen Atemwegen (<10⁻⁸ mol/l) und die entsprechend höhere Steroiddeposition auf den Schleimhäuten des Mundes und der Luftröhre. Nach Resorption gelangen hierdurch regelmäßig kleinere Steroidmengen unerwünschtermaßen auch in die Blutbahn und verursachen typische Steroidnebenwirkungen, wie z. B. Hemmung der Kortisolproduktion, Entwicklung einer Osteoporose, Kataraktbildung etc. Diese zunehmend bekannt werdenden Nebenwirkungen limitieren daher bislang auch den therapeutischen Einsatz von ICS bei leichteren Formen der COPD, obgleich ICS, je nach Schweregrad der Atemwegserkrankung, gemäß den Therapierichtlinien noch höher dosiert und in Kombination mit weiteren Therapeutika bis hin zu oralen Glukokortikoiden über einen Zeitraum von zwei bis drei Wochen empfohlen werden. Die eigentliche Ursache hierfür ist auch auf eine nicht immer optimale Wirkeffizienz der eingesetzten Therapeutika zurückzuführen.

Neben den vorgenannten Therapieansätzen werden, mehr oder weniger traditionell, zur symptomatischen Behandlung beispielsweise bronchitischer Beschwerden und zur Erleichterung des Abhustens bei Hypersekretion, insbesondere bei Erkältungskrankheiten, auch inhalativ anzuwendende ätherische Öle bzw. Ölgemische für einen kürzeren Zeitraum eingesetzt. Hierbei handelt es sich jedoch um eine nichtkausale Therapie, welche insbesondere nur bei leichtgradigen, vor allem akuten Atemwegserkrankungen zum Einsatz kommt, jedoch bei chronischen und insbesondere schwergradigen Atemwegserkrankungen allenfalls unterstützend angewendet werden kann.

Darüber hinaus wird unter dem Begriff "Erkrankungen des Verdauungstraktes", wie er auch im Rahmen der vorliegenden Erfindung verwendet wird, eine allgemeine Bezeichnung verstanden, welche insbesondere entzündliche Erkrankungen der oberen wie unteren Abschnitte des Verdauungstraktes, insbesondere des Darms, bezeichnet, wobei hierunter akute wie chronische Krankheitszustände zu subsumieren sind. Beispiele für entzündliche Erkrankungen des Darms sind insbesondere chronisch entzündliche Darmerkrankungen (*Inflammatory Bowel Disease,* IBD), wie *Morbus Crohn* und *Colitis ulcerosa.*

Bei *Morbus Crohn* handelt es sich um chronisch-granulomatöse Entzündungen. Bevorzugt befallen sind der untere Dünndarm *(terminales Ileum)* und Dickdarm (*Colon*), seltener die Speiseröhre (Ö*sophagus*)*.* Charakterisierend für *Morbus Crohn* ist der diskontinuierliche, segmentale Befall der Darmschleimhaut, es können also gleichzeitig mehrere Darmabschnitte erkrankt sein, die durch gesunde Abschnitte voneinander getrennt sind. Typische Symptome des *Morbus Crohn* sind Bauchschmerzen und Durchfall. Die Schmerzen treten besonders oft im rechten Unterbauch und oft nach dem Essen oder vor dem Stuhlgang auf. Die Durchfälle sind manchmal blutig. Die Beschwerden treten für gewöhnlich in sogenannten Schüben auf. Ein derartiger Schub dauert meist mehrere Wochen an.

Die *Colitis ulcerosa* (Cu, *ulcerative colitis*) ist durch einen entzündlichen Befall des Mastdarms und des Dickdarms gekennzeichnet. Anders als beim *Morbus Crohn* breitet sich die der Erkrankung zugrundeliegende Entzündung kontinuierlich vom Mastdarm beginnend aus, also von anal nach oral. Die Entzündung als solche ist in der Regel auf die Darmschleimhaut beschränkt.

Glukokortikoide sind die wichtigsten Medikamente bei der Behandlung von chronisch entzündlichen Darmerkrankungen und insbesondere zur Behandlung der akuten Schübe des *Morbus Crohn.* Die therapeutisch und vorrangig systemisch bzw. peroral applizierten Glukokortikoide führen selbst in schweren Fällen noch bei der Hälfte aller Patienten zu einer Remission. Bei einem leichten bis mittelgradigen Schub kann eine gewisse Verbesserung erreicht werden. Jedoch ist der Therapieerfolg nicht immer ausreichend, und zudem treten oftmals gravierende, insbesondere systemische Nebenwirkungen auf.

Die WO 2007/036802 A2 betrifft pharmazeutische Dosierformen von Vitamin D-Analoga, wie beispielsweise Calcitriol, Verfahren zu deren Herstellung sowie deren jeweilige Verwendung. Die in diesem Zusammenhang beschriebenen Dosierformen sind insbesondere für die Behandlung von Autoimmunerkrankungen vorgesehen.

Die WO 01/46132 A1 betrifft Verfahren zur Behandlung von entzündlichen Erkrankungen des Darmtraktes, wobei diese Verfahren die alleinige Applikation von biologisch aktiven Vitamin D-Komponenten umfassen, was zu einer Linderung der Symptome der genannten Erkrankungen des Darms führen soll.

Gemäß der WO 2008/039409 A2 werden Verfahren zur Behandlung und Vorbeugung von Zuständen der Immunschwäche oder Infektionskrankheiten allgemein beschrieben, wobei die Verfahren die Applikation von Vitamin D in Kombination mit weiteren Antioxidantien, wie beispielsweise Vitamin E oder Zink, umfassen.

Die WO 2009/032887 A1 betrifft oral zu verabreichende Zusammensetzungen sowie ein Behandlungsverfahren für Infektionen der Atemwege, im Rahmen dessen einem Menschen eine Zusammensetzung verabreicht wird, welche pro Dosiereinheit 450 IU bis 500.000 IU Cholecalciferol enthält.

Die WO 2010/025821 A1 betrifft die Verwendung eines Monoterpens einerseits sowie eines weiteren Atemwegstherapeutikums andererseits zur Behandlung von Atemwegserkrankungen.

Der vorliegenden Erfindung liegt daher die Aufgabe zugrunde, die zuvor geschilderten Nachteile des Standes der Technik zumindest teilweise zu vermeiden oder aber wenigstens abzuschwächen.

Insbesondere soll im Rahmen der vorliegenden Erfindung eine verbesserte und/oder effizientere Therapie zur Behandlung von Atemwegserkrankungen, insbesondere bronchopulmonalen Erkrankungen, bzw. zur Behandlung von entzündlichen Darmerkrankungen bereitgestellt werden.

Weiterhin soll im Rahmen der vorliegenden Erfindung eine verbesserte Wirkeffizienz, insbesondere einhergehend mit einer möglichen Dosisreduktion, bzw. ein breiteres Anwendungsspektrum lokal bzw. topisch, insbesondere inhalativ applizierbarer Atemwegstherapeutika der vorgenannten Art (wie z. B von inhalativen Bronchodilatatoren und/oder Bronchospasmolytika, einschließlich Sympathomimetika, Phosphodiesterasehemmern, Parasympatholytika und/oder Vagolytika, Anticholinergika, Kortikosteroiden etc.) insbesondere im Hinblick auf eine Therapie zur Behandlung von Atemwegserkrankungen ermöglicht bzw. erreicht werden.

Gleichermaßen soll im Rahmen der vorliegenden Erfindung eine verbesserte Wirkeffizienz, insbesondere auch einhergehend mit einer möglichen Dosisreduktion, bzw. ein breiteres Anwendungsspektrum von insbesondere systemisch bzw. peroral applizierbaren Therapeutika (wie z. B. von systemischen Kortikosteroiden etc.) insbesondere im Hinblick auf eine Therapie zur Behandlung von Darmentzündungen ermöglicht bzw. erreicht werden.

Zur Lösung der zuvor angeführten Aufgabe schlägt die vorliegende Erfindung ein Kombinationstherapeutikum, insbesondere pharmazeutisches Kombinationstherapeutikum, nach Anspruch 1; weitere, insbesondere vorteilhafte Ausgestaltungen des erfindungsgemäßen Kombinationstherapeutikums sind Gegenstand der diesbezüglichen Unteransprüche.

Bei sämtlichen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoffe oder dergleichen stets 100 % bzw. 100 Gew.-% resultieren. Dies versteht sich für den Fachmann aber von selbst.

Im Übrigen gilt, dass der Fachmann anwendungsbezogen oder einzelfallbedingt von den nachfolgend aufgeführten Zahlen-, Bereichs- oder Mengenangaben abweichen kann, ohne dass er den Rahmen der vorliegenden Erfindung verlässt.

Zudem gilt, dass sämtliche im Folgenden genannten Werte- bzw. Parameterangaben oder dergleichen grundsätzlich mit genormten bzw. standardisierten oder explizit angegebenen Bestimmungsverfahren oder anderenfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Die Anmelderin hat nun überraschenderweise herausgefunden, dass die zuvor geschilderten Aufgaben dadurch in effektiver Weise gelöst werden, dass erfindungsgemäß ein Kombinationstherapeutikum, insbesondere pharmazeutisches Kombinationstherapeutikum, vorzugsweise zur Verwendung bei der prophylaktischen und/oder therapeutischen Behandlung von insbesondere entzündlichen Atemwegserkrankungen, insbesondere bronchopulmonalen Erkrankungen, oder zur Verwendung bei der prophylaktischen und/oder therapeutischen Behandlung von insbesondere entzündlichen Erkrankungen des Verdauungstrakts, insbesondere des Darms, bereitgestellt wird. Das erfindungsgemäße Kombinationstherapeutikum zeichnet sich dabei dadurch aus, dass das Kombinationstherapeutikum-insbesondere in jeweils wirksamen, vorzugsweise pharmazeutisch wirksamen Mengen - mindestens einen Vitamin D-Rezeptoragonisten (VDA) einerseits und 1,8-Cineol andererseits umfasst.

Denn die Anmelderin hat in völlig überraschender Weise gefunden, dass auf Basis der erfindungsgemäßen Kombination mit dem gezielten Einsatz eines Vitamin D-Rezeptoragonisten (VDA) eine synergistische Wirkverstärkung in Bezug auf 1,8-Cineol vorliegt und auf dieser Basis ein wirkeffizientes Kombinationstherapeutikum für die angeführten Indikationen bereitgestellt wird, welches zu einem verbesserten Therapieerfolg bzw. zu einer Dosisreduktion der eingesetzten Wirkkomponenten, insbesondere auch des Monoterpens, sowie zu einem schnelleren Abklingen der Erkrankung führt. In diesem Zusammenhang führt in völlig unerwarteter Weise auch eine weiterführende Anwendung bzw. Co-Medikation des erfindungsgemäßen Kombinationstherapeutikums insbesondere mit sogenannten Leitlinientherapeutika für die jeweils zugrundeliegende Erkrankung zu deutlich verbesserten Therapieerfolgen, wobei es im Rahmen der vorliegenden Erfindung völlig überraschend gelungen ist, die Wirkeffizienz der jeweils eingesetzten Therapeutika, beispielsweise von Kortikosteroiden, signifikant zu verbessern. Hierauf wird im Nachfolgenden noch im Detail eingegangen. In diesem Zusammenhang kommt dem erfindungsgemäßen Kombinationstherapeutikum selbst insbesondere auch eine antientzündliche Wirkweise zu, welche durch den Einsatz des Vitamin D-Rezeptoragonisten maßgeblich gesteigert wird.

Der im Rahmen der vorliegenden Erfindung völlig überraschend aufgefundene und in den nachfolgenden Ausführungsbeispiele belegte synergistische Effekt, welcher mit dem erfindungsgemäßen Kombinationstherapeutikums einhergeht, kann insbesondere - ohne sich auf diese Theorie beschränken zu wollen - darauf zurückgeführt werden, dass die Expression von Vitamin D-Rezeptoren durch Vitamin D bzw. den Vitamin D-Rezeptoragonisten (VDA) selbst reguliert wird. Demnach ist der Vitamin D-Rezeptor bei Vitamin D-Mangelzuständen, welche oftmals bei den zugrundeliegenden Indikationen vorliegen, herunterreguliert und kann durch Vitamin D hochreguliert werden. Die therapeutische Verabreichung von Vitamin D bzw. des in Rede stehenden Vitamin D-Rezeptoragonisten (VDA) bewirkt somit im Rahmen der vorliegenden Erfindung - ohne sich auf diese Theorie beschränken zu wollen-insbesondere in den therapeutisch angeführten Mengen eine Hochregulation des Vitamin D-Rezeptors, welche durch den weiteren Wirkstoff bzw. die weitere Wirkkomponente des erfindungsgemäßen Kombinationstherapeutikums noch weiterführend verstärkt wird. Denn der weitere Wirkstoff, insbesondere in Form des Monoterpens, führt - gleichermaßen ohne sich auf diese Theorie beschränken zu wollen - insbesondere auch zu einer Hemmung von sogenannten COX II-metaboliten, insbesondere Prostaglandinen, welche insbesondere bei Infekten sowie akuten und chronischen Entzündungen gebildet werden und eine Herabregulierung von Vitamin D-Rezeptoren induzieren können. Durch die Hemmung von COX II-Metaboliten kann somit auch auf diesem Weg der Vitamin D-Rezeptor hochreguliert werden, und zwar insbesondere auch insofern, als seine Herunterregulation verhindert wird. Zudem hemmt Vitamin D - gleichermaßen ohne sich auf diese Theorie beschränken zu wollen - sowohl das Protein MKP-1 als auch p-38-MAPK. Insofern führt Vitamin D auch zu einer Beeinflussung insbesondere des MAP-Kinase-Signaltransduktionsweg, welcher unter anderem entzündliche Prozesse im Körper induzieren kann. Die gezielte Wirkkombination der dem erfindungsgemäßen Kombinationstherapeutikum zugrundeliegenden Wirkkomponenten kann auch auf dieser Ebene zu dem in völlig unerwarteter Weise aufgefunden synergistischen Effekt führen.

Durch die erfindungsgemäß vorgesehene weiterführende Kombination bzw. Begleittherapie beispielsweise mit Steroiden, insbesondere Kortikosteroiden, wird-gleichermaßen ohne sich auf diese Theorie beschränken zu wollen -, bedingt durch die Steroidgabe, eine Verminderung der Prostaglandinsynthese neben einer Hochregulation von Steroidrezeptoren, welche im Allgemeinen für immunmodulatorische Prozesse eine Rolle spielen, erzielt, was einen weiteren positiven Einfluss auf die Verringerung entzündlicher Prozesse hat. Auf Basis der gezielten Wirkstoffkombination wird somit in völlig unerwarteter Weise ein synergistischer Effekt hinsichtlich der Wirkeffizienz des erfindungsgemäßen Kombinationstherapeutikums bereitgestellt.

Üblicherweise sind gesunde Personen bei ausreichender Sonnenexposition grundsätzlich unabhängig von einer zusätzlichen Aufnahme von Vitamin D über die Nahrung, da unter Sonneneinfluss bzw. UV-Strahlung ausgehend von der Vorläufersubstanz von Vitamin D, nämlich 7-Dehydroxycholesterol, in der Haut Prävitamin D₃ entsteht. Aus dem instabilen Prävitamin D₃ entsteht nach etwa 48 Stunden Calcitriol, welches eine physiologisch aktive Form von Vitamin D₃ darstellt. Darüber hinaus können ausgehend von 7-Dehydroxycholesterol zum Schutz vor einer Vitamin D₃-Überproduktion Lumisterol und Tachysterol gebildet werden. Ein Mangel oder das Meiden von Sonne aber auch bestimmte Erkrankungen erfordern mitunter jedoch eine nahrungsabhängige Aufnahme von pflanzlichem Vitamin D₂ bzw. Ergocalciferol bzw. von tierischem Vitamin D₃ bzw. Cholecalciferol.

Da im Körper die aktive Form von Vitamin D erst durch enzymatische Prozesse bereitgestellt wird, stellt der körpereigene Vitamin D-Metabolismus ein komplexes Netzwerk auf Basis zahlreicher aufeinanderfolgender Stoffwechselschritte dar. Im Rahmen des Vitamin D-Metabolismus erreichen die verschiedenen Vitamin D-Metabolite, insbesondere gebunden an das Vitamin D-bindende Protein (VDB), die Leber oder werden im Fettgewebe aufgrund ihrer Lipophilie gespeichert. In der Leber werden Vitamin D-Metabolite durch die 25-Hydroxylase (CYP2R1) zu Calcidiol (25-Hydroxyvitamin D bzw. 25-OH D) mit höherer Bindungsaffinität zu dem Vitamin D-Rezeptor (synonym auch als Vitamin D₃-Rezeptor bezeichnet) und mit einer Halbwertszeit von etwa 2,5 Wochen metabolisiert. Die Konversionsrate kann dabei 0,1 bis 0,2 %/10⁶ Zellen/Stunde betragen. Der Spiegel von Calcidiol entspricht der sonnen- und/oder diätbedingten Vitamin D₃-Menge (100 bis 200 IU/Tag) und nimmt im Sommer im Mittel um 12 ng/ml zu.

Durch die 1-alpha-Hydroxylase entsteht im Rahmen des Vitamin D-Metabolismus schließlich der aktive Metabolit Calcitriol (1,25-Dihydroxyvitamin D bzw. 1,25-DOH D) in der Niere und anderen Zellen. Calcitriol besitzt im Vergleich zu Calcidiol mitunter ein stärkeres Potential zur Hypercalcämie. Die Aktivität der 1-alpha-Hydroxylase und somit die renale Bildung von Calcitriol wird durch verschiedene Hormone, insbesondere Parathormon, Oestrogene, Calcitonin, Wachstumshormone und Prolaktin, sowie durch ein Absinken des Phosphatspiegels, insbesondere Hypophosphatämie, stimuliert. Durch Calcium und Calcitriol selber hingegen wird die 1-alpha-Hydroxylase gehemmt. Neben den Zellen der Niere wird die 1-alpha-Hydroxylase auch in anderen Zellen, wie Makrophagen oder Granulomen, exprimiert. Im Gegensatz zu den Zellen der Nieren haben in den übrigen Zellen jedoch Parathormon, Calcium und Vitamin D-Metabolie keinen Einfluss auf deren Aktivität. Nebenwirkungen von Glukokortikoiden, Chloroquin oder Ketoconazol hingegen sind allgemein mit einer Reduktion von Calcitriol verbunden. Bei Adipositas liegt häufig ein Mangel an Calcidiol aufgrund seiner Lipophilie vor, wobei dennoch zwischen Ursache und Folge des Vitamin D-Mangels auch bei Adipositas nicht sicher differenziert werden kann.

Vitamin D-Rezeptoren (VDR) werden von verschiedenen Zellen exprimiert, beispielsweise von Monozyten, Makrophagen, Thrombozyten, Lymphozyten sowie auch Epithelzellen. Folglich kommen Vitamin D-Rezeptoren (VDR) unter anderem in den Atemwegen sowie im Darm vor. Die Expression des Vitamin D-Rezeptors (VDR) für Calcitriol wird dabei durch Calcitriol selbst kontrolliert. Insbesondere ist ein übermäßiger Anstieg der Calcitriolkonzentration mitunter mit einer Hemmung bzw. Verringerung der Expression von Vitamin D-Rezeptoren (VDR) verbunden. Liegt Calcitriol in einer Konzentration von 10⁻⁷ mol/l vor, erfolgt eine 9-fache Expression, wohingegen die Anwesenheit einer höheren Calcitriolkonzentration von 10⁻⁶ mol/l lediglich zu einer 1,6-fachen Expression von VDR führt.

Im Gegensatz dazu werden Vitamin D-Rezeptoren (VDR) für Calcidiol weder durch Calcidiol selbst noch durch Calcitriol herunterreguliert, was insbesondere im Hinblick auf den therapeutischen Einsatz von Calcitriol bzw. Calcidiol zu berücksichtigen ist.

Insgesamt ist die Rolle dieser Rezeptoren weitgehend unerforscht. Unter anderem wird von einer Involvierung in die Zunahme der intrazellulären CalciumKonzentration, die Phospolipase C-Aktivität, die Regulation der DNA-Transkription und die RNA-Synthese mit einer höheren Bindungsaffinität für Calcitriol im Vergleich zu Calcidiol vermutet. In verschiedenen Zellmodellen wurde zudem eine zunehmende Zelldifferenzierung und eine Hemmung der Zellproliferation durch Calcitriol beschrieben und es existieren Hinweise, dass auch Calcidiol hierbei eine Rolle spielt.

Als ungefährer Tagesbedarf von Vitamin D bzw. Vitamin D₃ werden 600 IU empfohlen und bei älteren Patienten oder bei erhöhtem Osteoporoserisiko 800 IU bzw. 4.000 IU als maximale tägliche Aufnahme, um einen Calcidiol-Spiegel von mindestens 20 ng/ml zu erreichen. Andere Empfehlungen zur Behandlung der Osteoporose gehen von einer täglichen Aufnahme von 2.000 IU aus, um einen Plasmaspiegel von > 30 ng/ml sicherzustellen. Hierbei mangelt es jedoch an gesicherten Erfahrungen, um die Entwicklung verschiedener Erkrankungen bei Überdosierung bis hin zur Arteriosklerose und Bauchspeicheldrüsenkarzinomen zu vermeiden. Das US-*Institute of Medicine* empfiehlt in diesem Zusammenhang eine orale Calcitriol-Aufnahme von 50.000 IU/Woche über einen Zeitraum von acht Wochen. Anschließend erfolgt eine Senkung der Calcitriol-Zufuhr auf 50.000 IU alle zwei bis vier Wochen. Alternativ wird die Gabe von 100.000 IU über einen Zeitraum von drei Monaten angeführt.

Insgesamt existiert eine Vielzahl von Erkrankungen, welche mit einem Vitamin D-Mangel assoziiert sind. Dazu gehören überwiegend chronische Erkrankungen, wie verschiedene Tumorerkrankungen, Autoimmunerkrankungen, sowie Infektionserkrankungen, beispielsweise Tuberkulose, kardiovaskuläre Erkrankungen, wie die koronare Herzerkrankung und Bluthochdruck, *Morbus Crohn, Colitis ulcerosa,* Arthritis, Psoriasis und Nierenerkrankungen. Auch Behandlungen mit bestimmten Therapeutika, insbesondere Kortikosteroiden und Antiepileptika, führen zu einem Vitamin D bzw. Vitamin D₃-Mangel als Nebenwirkung. Weiterhin können auch psychische Erkrankungen, wie Depressionen oder Schizophrenie, mit einem Vitamin D bzw. Vitamin D₃-Mangel assoziiert sein. Bei allen vorgenannten Erkrankungen kann im Allgemeinen also ein Mangel an Calcitriol nachgewiesen werden, wobei bislang nicht geklärt ist, ob der Mangel an Vitamin D bzw. Vitamin D₃ die Ursache oder die Folge der Erkrankung ist.

Ein Mangelzustand an Calcitriolwird üblicherweise durch gleichzeitige Bestimmung des Präkusors oder Metaboliten Calcidiol bestimmt.

Es wird angenommen, dass - ohne sich auf diese Theorie beschränken zu wollen - der Wirkmechanismus von Vitamin D auf einer Bindung von Calcidiol bzw. Calcitriol an den Vitamin D-Rezeptor (VDR) basiert. Dieser Komplex bindet an das Vitamin D-Response-Element in der Promotorregion des Vitamin D-Reponsegenes, so dass es zu einer Zunahme der durch die RNA-Polymerase vermittelten Transkription des Vitamin D-Reponsegenes kommt. Infolge der verstärkten Transkription des Vitamin D-Reponsegenes werden verschiedene immunrelevante Vorgänge vermittelt bzw. induziert, unter anderem die Aktivierung der CD4-Rezeptoren von T-Helferzellen, die Hemmung der von T-Helferzellen produzierten Zytokinen (Interferon-gamma, Interleukin-2, Interleukin-5), welche insbesondere zu Entzündungsreaktionen führen, und eine Stimulation von Interleukin-4. Darüber hinaus kann auch eine Hemmung von Interferon-gamma bei gleichzeitiger Stimulation von Interleukin-4, Interleukin-5 und Interleukin-10 auftreten. Auch kann eine Hemmung der Interleukin-12 stimulierten Produktion von Interferon-gamma sowie eine Hemmung von Interleukin-4 und Interleukin-13 durch Calcitriol erfolgen. Aus den vorstehenden Ausführungen ist ersichtlich, dass Vitamin D, insbesondere Calcitriol, an verschiedenen Immunantworten beteiligt ist.

Darüber hinaus wird auch angenommen, dass Calcidiol eine Rolle für Prozesse des Immunsystems haben könnte. Im Rahmen der vorliegenden Erfindung wurde gefunden, dass Calcidiol und Calcitriol die Produktion des im Zusammenhang mit Entzündungsreaktionen gebildeten Tumornekrosefaktor-alpha (TNF-alpha) hemmen, wobei Calcidiol die TNF-alpha-Bildung bis zu dreimal stärker hemmt als Calcitriol und somit überraschenderweise eine nochmals stärkere Entzündungshemmung bewirkt. Darüber hinaus wurde überraschenderweise gefunden, dass auch Calcidiol in synergistischer Weise mit verschiedenen Leitlinientherapeutika interagiert und diese in ihrer Wirkung verstärkt. Für Calcitriol ist in diesem Zusammenhang beachtlich, dass Calcitriol in höheren Konzentrationen mitunter eine Downregulation von Vitamin D-Rezeptoren (VDR) induziert, so dass die Wirkung von Calcitriol insbesondere bei höheren Konzentrationen von Calcitriol abgeschwächt sein kann. Dennoch kommt auch Calcitriol im Rahmen der vorliegenden Erfindung eine hohe Bedeutung zur Behandlung der zugrundeliegenden Erkrankungen zu.

Auf dieser Grundlage wird im Rahmen der vorliegenden Erfindung auch eine Co-Medikation von Vitamin D bzw. Vitamin D-Metaboliten, insbesondere Calcidiol und/oder Calcitriol, vorzugsweise Calcidiol, in Kombination mit verschiedenen entzündungshemmenden, bronchodilatatorischen und/oder antiallergischen Leitlinientherapeutika vorgeschlagen.

Im Rahmen der vorliegenden Erfindung steht vorrangig die entzündungshemmende Wirkung von Vitamin D im Vordergrund. Erfindungsgemäß von Bedeutung ist dabei einerseits die entzündungshemmende Wirkung von Vitamin D an sich, welche in völlig überraschender Weise im Rahmen eines synergistischen, antientzündlichen Effektes durch eine Kombination mit weiteren Wirkstoffen, insbesondere einem Monoterpen, gesteigert werden kann. Im Rahmen der vorliegenden Erfindung wird in diesem Zusammenhang insbesondere eine Art Zusatztherapie bzw. Co-Medikation zur Verbesserung der klinischen Effekte bzw. eine Reduktion des Medikamentenbedarfs auch im Rahmen von Leitlinientherapien bereitgestellt.

Bei Patienten mit chronisch obstruktiver Atemwegserkrankung (COPD) ist oftmals ein Vitamin D-Mangel vorhanden. In diesem Zusammenhang hat sich gezeigt, dass der Calcidiol-Spiegel im Blut mit dem Schweregrad der Lungenfunktionseinschränkung (FEV1) und somit auch mit dem Schweregrad der COPD (GOLD-Stadien) korreliert.

Was im Speziellen den Zusammenhang zwischen Autoimmunerkrankungen und Vitamin D-Mangel anbelangt, wird vermutet, dass bei verschiedenen Autoimmunerkrankungen ein niedriger Vitamin D-Spiegel durch eine chronische, intrazelluläre Infektion verursacht wird, welche zu einer Fehlfunktion des Vitamin D-Rezeptors führt und auf diese Weise wiederum die Empfänglichkeit für sekundär auftretende Infektionen erhöht. Dies wird insbesondere für die Krankheiten *Psoriasis vulgaris, Lupus erythematodes,* rheumatoide Arthritis, Sarkoidose, Uveitis und weitere vermutet. Weiterhin sind Patienten mit chronisch polypoider Rhinosinusitis und einer Pilz-allergischen Rhinosinusitis teilweise von Vitamin D-Mangelzuständen betroffen, welche auch mit Nasenpolypen, einer vermehrten ossären Erosion und einer Verminderung von dendritischen Zellen assoziiert sind. Häufig tritt ein Vitamin D-Mangel auch bei verschiedenen Formen chronisch-entzündlichen Darmerkrankungen (IBD), insbesondere bei *Morbus Crohn* (CD) und *Colitis ulcerosa* (UC), auf. Erfindungsgemäß wurde in völlig überraschender Weise gefunden, dass auf Basis des erfindungsgemäßen Kombinationstherapeutikums nicht nur eine effiziente Wirksteigerung bzw. Dosisreduzierung von weiteren Wirkstoffen, wie sie beispielsweise im Rahmen von Leitlinientherapien eingesetzt werden, erreicht werden kann, sondern dass auch den zuvor mit den zugrundeliegenden Krankheiten aufgefundenen Vitamin D-Mangelzuständen in effektiver Weise entgegengetreten werden kann, was einen weiteren zentralen Vorteil der vorliegenden Erfindung darstellt.

Im Stand der Technik wird jedoch im Zusammenhang mit Vitamin D-Mangelerkrankungen, welche vorwiegend aus Beobachtungsstudien und teilweise aus Interventionsstudien bei malignen Erkrankungen, wie Colon-Karzinomen, stammen, zunehmend Empfehlungen nationaler und internationaler Fachverbände ausgesprochen, den Vitamin D-Spiegel nicht routinemäßig zu bestimmen. Insbesondere wird von unnötigen Vitamin D-Supplementierungen abgeraten. So empfiehlt das *U.S. Institute of Medicine* (IOM) im Januar 2011, dass für die Knochengesundheit eine Vitamin D-Konzentration von 20 ng/ml für 97,5 % der Bevölkerung völlig auseichend ist. Bislang wurde eine Vitamin D-Konzentration von 30 ng/ml empfohlen. Darüber hinaus wird weiterhin argumentiert, dass "Nahrungsergänzungsmittel und Vitamin D-Zusätze zu Lebensmitteln aus medizinischer Sicht nur dann sinnvoll sind, wenn weitere Risikofaktoren für eine Osteoporose vorliegen-beispielsweise bei älteren Menschen oder bei verminderter Knochendichte". In gleicher Weise warnt auch die Deutsche Gesellschaft für Endokrinologie, dass "eine Überversorgung Gesundheitsrisiken berge und die Einnahme von Vitamin D-Präparaten nur in ärztlich begründeten Fällen notwendig ist", insbesondere vor dem Hintergrund, dass der Organismus ungefähr 80 % des benötigten Vitamin D selbst bildet. Auch ausgehend von den obigen Ausführungen ist die erfindungsgemäß vorgeschlagene gezielte Therapie für die angeführten Erkrankungen auf Basis von Vitamin D dem Fachmann gerade nicht nahegelegt.

Denn erfindungsgemäß wurden nunmehr in völlig überraschender Weise spezifische Wirkeffekte von Vitamin D, insbesondere Calcidiol und/oder Calcitriol, gefunden, welche die erfindungsgemäßen speziellen Verwendungen im Rahmen von Wirkeffizienzsteigerungen und Dosisreduktionen begleitender Therapeutika bzw. pharmakologischer Wirksubstanzen ermöglichen.

In diesem Zusammenhang wurde überraschenderweise auch gefunden, dass Calcidiol, der Präkusor und Metabolit von Calcitriol, eine primäre und stärker ausgebildete sowie eigenständige steroidartige und entzündungshemmende Wirkung besitzt als Calcitriol bzw. Vitamin D in seiner physiologisch aktiven Form selbst, so dass Cacidiol eine erfindungsgemäß besonders bevorzugte Wirkkomponente in Form des Vitamin D-Rezeptoragonisten (VDA) darstellt.

Insbesondere übersteigt die entzündungshemmende Wirkung von Calcidiol die Wirkung von Calcitriol etwa um das dreifache, so dass Calcidiol, wie zuvor angeführt, im Rahmen des erfindungsgemäßen therapeutischen Einsatzes, insbesondere als Kombinationstherapeutikum zur Wirkungssteigerung von Leitlinientherapeutika, als eine der bevorzugten Substanzen anzuführen ist. Dessen ungeachtet kommt jedoch auch Calcitriol im Hinblick auf die vorliegende Erfindung eine große Rolle als Vitamin D-Rezeptoragonist (VDA) zu.

Für den therapeutischen Einsatz von Calcitriol und/oder Calcidiol bzw. einer Mischung aus beiden vorgenannten Substanzen zur Erhöhung insbesondere lokaler, antientzündlicher Vitamin D-Konzentrationen und somit der Wirksamkeit, wird erfindungsgemäß zur Gewährleistung einer optimalen Verfügbarkeit ein spezielles Therapiekonzept im Allgemeinen zur primären Lokaltherapie einerseits zur Behandlung von Atemwegserkrankungen, insbesondere in Form eines Nasensprays; zur inhalativen Therapie, insbesondere in Form eines Pulvers und/oder Dosieraerosols; und andererseits zur Behandlung entzündlicher Darmerkrankungen (*Morbus Crohn, Colitis ulcerosa*), insbesondere in Form von Pellets oder gelöst bzw. suspendiert in Öl in dünndarmlöslichen Kapseln, zur lokalen Darmtherapie- und der Systemtherapie bei assoziierter Systementzündung bereitgestellt.

Erfindungsgemäß kann das Kombinationstherapeutikum für die Therapie mit Vitamin D somit in nichtbeschränkender Weise in Form einer insbesondere inhalierbaren Lösung oder Suspension auf Basis eines Pulvers oder Sprays bereitgestellt werden, insbesondere verbunden mit dem Vorteil, unabhängig vom Körpergewicht und der Lipophilie des Wirkstoffs, auf Basis von Vitamin D eine lokale Schleimhautentzündung, z. B. der Atemwege, direkt zu behandeln. Durch diesen Ansatz werden auch gegebenenfalls vorliegende Vitamin D-Nebenwirkungen der oralen Therapie, insbesondere die etwaige Ausbildung einer Hypercalcämie, vermieden. Gegenstand der vorliegenden Erfindung - gemäß einem ersten Aspekt der vorliegenden Erfindung - ist somit ein pharmazeutisches Kombinationstherapeutikum zur Verwendung bei der prophylaktischen und/oder therapeutischen Behandlung von entzündlichen Atemwegserkrankungen und/oder bronchopulmonalen Erkrankungen oder zur Verwendung bei der prophylaktischen und/oder therapeutischen Behandlung von entzündlichen Erkrankungen des Darms,
wobei das Kombinationstherapeutikum - in jeweils pharmazeutisch wirksamen Mengen - umfasst:
(a) mindestens einen Vitamin D-Rezeptoragonisten (VDA) und
(b) 1,8-Cineol.

Die Begriffe "Kombinationstherapeutikum" bzw. "pharmazeutisches Kombinationstherapeutikum" oder dergleichen, wie sie im Rahmen der vorliegenden Erfindung verwendet werden, sind sehr umfassend zu verstehen und bezeichnen nicht nur pharmazeutische Präparate bzw. Pharmazeutika, sondern auch sogenannte Medizinprodukte, homöopathische Mittel, Nahrungsergänzungsmittel oder dergleichen.

Weiterhin werden unter den Begriffen "Vitamin D-Rezeptoragonist" bzw. "Vitamin D-Rezeptoragonist (VDA)", wie sie im Rahmen der vorliegenden Erfindung verwendet werden, insbesondere solche Substanzen verstanden, welche imstande sind, mit den zugrundeliegenden Vitamin D-Rezeptoren insbesondere nach Art eines Liganden in Wechselwirkung zu treten bzw. mit den zugrundeliegenden Vitamin D-Rezeptoren zu interagieren, so dass infolge der zugrundeliegenden Wechselwirkung bzw. Interaktion eine Aktivierung der in Rede stehenden Vitamin D-Rezeptoren erfolgt. Insbesondere kann die Wechselwirkung bzw. Interaktion des erfindungsgemäß eingesetzten Vitamin D-Rezeptoragonisten mit den Vitamin D-Rezeptoren auf Basis einer Bindung, insbesondere auf Basis des sogenannten "Schlüssel/Schloss-Prinzips" erfolgen, infolgedessen eine Aktivierung der zugrundeliegenden Vitamin D-Rezeptoren mit nachfolgender Induktion stoffwechselspezifischer Vorgänge erfolgen kann. Hierbei kann es insbesondere vorgesehen sein, dass die erfindungsgemäß eingesetzte Substanz als solche, d. h. ohne weiterführende Modifizierung bzw. Verstoffwechselung, mit den Vitamin D-Rezeptoren zu interagieren imstande ist. Gleichermaßen umfassen die Begriffe "Vitamin D-Rezeptoragonist" bzw. "Vitamin D-Rezeptoragonist (VDA)", auch solche Substanzen, welche selbst nicht unmittelbar zur Wechselwirkung, insbesondere Bindung, mit bzw. an den Vitamin D-Rezeptor imstande sind, sondern gewissermaßen als Vorläufersubstanzen bzw. Präkursoren zur Bereitstellung eines Liganden für den Vitamin D-Rezeptor fungieren. Dabei kann die Modifizierung bzw. Umwandlung der eingesetzten Vorläufer insbesondere auf Basis stoffwechselspezifischer Vorgänge im Körper erfolgen.

Im Rahmen der vorliegenden Erfindung können als Vitamin D-Rezeptoragonisten insbesondere den natürlichen bzw. körpereigenen Metaboliten des körpereigenen Vitamin D-Stoffwechsels entsprechende bzw. hierzu chemisch zumindest im Wesentlichen identische bzw. wirkidentische Substanzen auf Basis synthetischer Verbindungen eingesetzt werden, wobei es sich diesbezüglich, wie nachfolgend noch definiert, insbesondere um 25-Hydroxy-Vitamin D₃ bzw. 25-Hydroxy-Vitamin D (Calcidiol) und/oder 1,25-Dihydroxy-Vitamin D₃ bzw. 1,25-Dihydroxy-Vitamin D (Calcitriol) handelt. In diesem Zusammenhang ist es im Rahmen der vorliegenden Erfindung gleichermaßen in nicht beschränkender Weise möglich, die entsprechenden Vorläufersubstanzen bzw. Präkursoren bzw. Speicherformen der in Rede stehenden Substanzen, wie beispielsweise 7-Dehydrocholesterol, Vitamin D₃ (Cholecalciferol) bzw. entsprechende inaktive Speicherprodukte, wie Lumisterol bzw. Tachysterol, einzusetzen, auch wenn dies erfindungsgemäß weniger bevorzugt ist. Insbesondere kann der Vitamin D-Rezeptoragonist auch in Form von Vitamin D₃ bzw. Cholecalciferol als solchem eingesetzt werden, welches nach entsprechender Verabreichung bzw. Applikation - ohne sich auf diese Theorie beschränken zu wollen - im Körper zu den entsprechend aktiven Liganden, insbesondere Calcidiol und/oder Calcitriol, metabolisiert bzw. verstoffwechselt wird.

Im Rahmen der vorliegenden Erfindung ist es, wie zuvor ausgeführt, gleichermaßen möglich, synthetische Analoga von Vitamin D₃ sowie dessen Vorläufern bzw. Präkursoren als Vitamin D-Rezeptoragonisten einzusetzen. Insbesondere kommt im Rahmen der vorliegenden Erfindung auch die Verwendung von Vitamin D₃-Analoga, wie Doxercalciferol, Alphacalciferol, Calcipotriol bzw. Dihydrotachystyrol, als Vitamin D-Rezeptoragonist in Betracht.

Gleichermaßen kommt im Rahmen der vorliegenden Erfindung auch die Verwendung von Vitamin D₂ bzw. Ergocalciferol sowie dessen Analoga in Betracht.

Bei den erfindungsgemäß eingesetzten Vitamin D-Rezeptoragonisten handelt es sich insbesondere um Substanzen, welche in Form von sogenannten Secosteroiden vorliegen können.

Weiterhin beziehen sich die im Rahmen der vorliegenden Erfindung verwendeten Begriffe "Vitamin D-Rezeptor" bzw. "Vitamin D-Rezeptor (VDR)" auf insbesondere zur Familie der Steroidrezeptoren vom Typ II gehörenden ligandenaktivierte Transkriptionsfaktoren bzw. Rezeptoren, welche insbesondere eine Affinität zu Liganden aus der Vitamin D-Gruppe aufweisen, wobei die in Rede stehenden Rezeptoren insbesondere eine hohe (Bindungs-)Affinität zu 25-Hydroxy-Vitamin D (Calcidiol) und/oder 1,25-Dihydroxy-Vitamin D (Calcitriol) aufweisen. Die in Rede stehenden Rezeptoren, welche erfindungsgemäß sozusagen als Zielstruktur bzw. *Target* für die eingesetzten Liganden fungieren, stellen somit im Allgemeinen insbesondere zu der Familie der Steroidrezeptoren gehörende Rezeptoren in Form von ligandenaktivierten Transkriptionsfaktoren dar, die infolge ihrer Aktivierung bestimmte Zielgene zu aktivieren oder zu hemmen im Stande sind, und so den Stoffwechsel insbesondere auch im Hinblick auf die Steuerung entzündlicher Prozesse beeinflussen können.

Ausgehend von der hohen Bindungsaffinität des Vitamin D-Rezeptors zu Calcidiol bzw. Calcitritol resultiert auch dessen Funktion, nämlich die Vermittlung der physiologischen Funktionen von Vitamin D in den zugrundeliegenden biologischen Strukturen, insbesondere Zielzellen. Ohne sich auf diese Theorie beschränken zu wollen, bilden Vitamin D-Rezeptoren nach Aktivierung durch Anbindung des Liganden ein Heterodimer mit dem sogenannten Retinoid-X-Rezeptor, wobei das resultierende Heterodimer als regulatives Element für die Expression von Zielgenen des Vitamin D-Rezeptors dient. Insbesondere kommt Vitamin D-Rezeptoren eine wichtige Funktion in der Regulation von Genen bzw. Genprodukten zu, welche für Immun-antworten, insbesondere entzündliche Prozesse unter Prostaglandinausschüttung sowie für proliferative Prozesse von Bedeutung sind.

Im Rahmen der vorliegenden Erfindung kann es vorgesehen sein, dass (a) der Vitamin D-Rezeptoragonist (VDA) ausgewählt ist aus der Gruppe von Verbidungen der Vitamin D-Gruppe, insbesondere Vitamin D₃ (Cholecalciferol) und/oder Vitamin D₂ (Ergocaliferol), vorzugsweise Vitamin D₃ (Cholecalciferol).

Gleichermaßen kann es im Rahmen der vorliegenden Erfindung vorgesehen sein, dass (a) der Vitamin D-Rezeptoragonist (VDA) ausgewählt ist aus der Gruppe von insbesondere physiologischen Vitamin D₃-Metaboliten und/oder insbesondere physiologischen Vitamin D₃-Präkursoren, vorzugsweise aus der Gruppe von insbesondere physiologischen Vitamin D₃-Metaboliten. Insbesondere kann (a) der Vitamin D-Rezeptoragonist (VDA) ausgewählt sein aus der Gruppe von insbesondere synthetischen Vitamin D₃-Analoga, insbesondere Tacalcitol (1,24-Dihydroxy-Vitamin D₃).

Erfindungsgemäß werden im Hinblick auf die Behandlung von insbesondere entzündlichen Erkrankungen der oberen Atemwege bzw. von insbesondere entzündlichen Erkrankungen des Darms besonders gute Ergebnisse erhalten, wenn der Vitamin D-Rezeptoragonist (VDA) ausgewählt ist aus der Gruppe von 25-Hydroxy-Vitamin D₃ (Calcidiol) und/oder 1,25-Dihydroxy-Vitamin D₃ (Calcitriol), vorzugsweise 25-Hydroxy-Vitamin D₃ (Calcidiol). Gemäß einer erfindungsgemäß besonders bevorzugten Ausführungsform ist (a) der Vitamin D-Rezeptoragonist (VDA) die Substanz 25-Hydroxy-Vitamin D₃ (Calcidiol).

Bei 25-Hydroxy-Vitamin D₃, welches synonym auch als 25-OH-Vitamin D₃ bzw. 25-OH D₃ bzw. 25-Hydroxycholecalciferol bezeichnet wird, handelt es sich insbesondere um eine Substanz des Vitamin D-Stoffwechsels mit Ligandenfunktion in Bezug auf Vitamin D-Rezeptoren. Im menschlichen Körper kann Calcidiol ausgehend von Vitamin D₃ durch Hydroxylierung von Vitamin D₃ insbesondere in der Leber gebildet werden.

Weiterhin handelt es sich bei 1,25-Dihydroxy-Vitamin D₃ bzw. Calcitriol, synonym auch als 1,25-(OH)₂-Vitamin D₃, 1,25-DOH D₃ bzw. 1α,25(OH)₂-Cholecalciferol bezeichnet, um eine weitere Substanz des Vitamin D₃-Stoffwechsels, wobei Calcitriol aus einer weiterführenden Hydroxylierung von Calcidiol resultieren kann.

Erfindungsgemäß kann es vorgesehen sein, dass (a) der Vitamin D-Rezeptoragonist (VDA) eine Kombination von 25-Hydroxy-Vitamin D₃ (Calcidiol) und 1,25-Dihydroxy-Vitamin D₃ (Calcitriol) ist.

In diesem Zusammenhang hat es sich als besonders vorteilhaft erwiesen, wenn das Kombinationstherapeutikum nach der Erfindung (i) 25-Hydroxy-Vitamin D₃ und (ii) 1,25-Dihydroxy-Vitamin D₃ in einem gewichtsbezogenen Mengenverhältnis von [(i) : (ii)] im Bereich von 1.000 : 1 bis 1 : 100, insbesondere im Bereich von 500: 1 bis 1 : 10, vorzugsweise im Bereich von 100 : 1 bis 1 : 5, bevorzugt im Bereich von 50 : 1 bis 1 : 1, besonders bevorzugt im Bereich von 10 : 1 bis 1 : 1, aufweist.

Gemäß einer erfindungsgemäß weiter bevorzugten Ausführungsform kann der Vitamin D-Rezeptoragonist (VDA) 25-Hydroxy-Vitamin D₃ (Calcidiol) sein.

Im Allgemeinen kann die Menge an Vitamin D-Rezeptoragonist (VDA) in dem erfindungsgemäßen Kombinationstherapeutikum in weiten Bereichen variieren. Insbesondere kann es im Rahmen der vorliegenden Erfindung vorgesehen sein, insbesondere um eine ausreichende therapeutische Wirksamkeit zu gewährleisten, dass das Kombinationstherapeutikum (a) den Vitamin D-Rezeptoragonisten (VDA), insbesondere 25-Hydroxy-Vitamin D₃ (Calcidiol) und/oder 1,25-Dihydroxy-Vitamin D₃ (Calcitriol), in einer Menge im Bereich von 0,0001 Gew.-% bis 5 Gew.-%, insbesondere im Bereich von 0,0005 Gew.-% bis 2 Gew.-%, vorzugsweise im Bereich von 0,001 Gew.-% bis 1,5 Gew.-%, bevorzugt im Bereich von 0,005 Gew.-% bis 1 Gew.-%, besonders bevorzugt im Bereich von 0,01 Gew.-% bis 0,75 Gew.-%, ganz besonders bevorzugt im Bereich von 0,05 Gew.-% bis 0,5 Gew.-%, bezogen auf das Kombinationstherapeutikum, enthält.

Zur Gewährleistung einer hohen Wirksamkeit des erfindungsgemäßen Kombinationstherapeutikums kann es im Rahmen der vorliegenden Erfindung vorgesehen sein, dass das Kombinationstherapeutikum (a) den Vitamin D-Rezeptoragonisten (VDA), insbesondere 25-Hydroxy-Vitamin D₃ (Calcidiol) und/oder 1,25-Dihydroxy-Vitamin D₃ (Calcitriol), in einer Menge im Bereich von 0,025 µg bis 500 µg, insbesondere im Bereich von 0,05 µg bis 400 µg, vorzugsweise im Bereich von 0,1 µg bis 300 µg, bevorzugt im Bereich von 1 µg bis 200 µg, besonders bevorzugt im Bereich von 10 µg bis 100 µg, bezogen auf eine Dosier- und/oder Applikationseinheit des Kombinationstherapeutikums, enthält.

Im Rahmen der vorliegenden Erfindung ist es zudem vorgesehen, dass als Monoterpen in Kombination mit dem Vitamin D-Rezeptoragonist (VDA) 1,8-Cineol eingesetzt wird.

Als erfindungsgemäß besonders wirksam hat es sich erwiesen, das Monoterpen 1,8-Cineol als isolierte bzw. einzelne Wirksubstanz einzusetzen (d. h. also keine Mischung verschiedener Monoterpene oder keine ätherischen Öle bzw. Ölgemische zu verwenden), selbstverständlich zusammen mit einem geeigneten pharmazeutischen Träger bzw. Exzipienten und gegebenenfalls zusammen mit weiteren, üblichen pharmazeutischen Hilfsstoffen und/oder Additiven. Dennoch ist erfindungsgemäß grundsätzlich nicht ausgeschlossen, zwei oder mehrere Monoterpene gemeinsam, insbesondere in Mischung, einzusetzen, obwohl dies erfindungsgemäß weniger bevorzugt ist.

Die Terpene sind eine stark heteorogene und sehr große Gruppe von chemischen Verbindungen, die sich biosynthetisch von Isopren- bzw. Isopentenyleinheiten ableiten lassen, wobei die Biosynthese über aktivierte Formen dieser Moleküle, nämlich dem Dimethylallylpyrophosphat (DMAPP) und dem Isopentenylpyrophosphat (IPP) erfolgt; die Einheiten bestehen aus fünf Kohlenstoffatomen (C₅-Einheiten). Es sind über 8.000 Terpene und über 30.000 der nahen verwandten Terpenoide bekannt. Die Terpene zählt man in der Systematik der organischen Chemie zu den Lipiden (sekundäre Naturstoffe). Der gemeinsame Baustein aller Terpene ist das Isopren. Die Terpene zählen zu den sekundären Pflanzenstoffen.

Es sind über 900 Monoterpene bekannt. Alle werden durch Monoterpensynthasen aus Geranylpyrophosphat (2,6-Dimethyloctan) synthetisiert. Im Rahmen der vorliegenden Erfindung kommen bevorzugt mono- und bicyclische Monoterpene zum Einsatz. Die meisten monocyclischen Monoterpene, die sich von p-Menthan ableiten lassen, weisen ein Cyclohexangerüst auf, während die Bicyclen Caran, Thujan, Pinan, Bornan und Fenchan, aber weiter gefasst auch Isobornylan und Isocamphan, die wichtigsten Stammverbindungen der bicyclischen Monoterpene sind.

Das Menthol wiederum - synonym auch als 2-Isopropyl-5-methylcyclohexanol oder aber 5-Methyl-2-(1-methylethyl)-cyclohexan-1-ol bezeichnet - ist ein monocyclischer Monoterpen-Alkohol, wohingegen das 1,8-Cineol zu den bicyclischen Epoxy-Monoterpenen, genauer gesagt zu den Limonenoxiden, gehört.

Synonyme Bezeichnungen für 1,8-Cineol mit der chemischen Summenformel C₁₀H₁₈O sind Eucalyptol, Limonen-1,8-oxid, 1,8-Epoxy-p-menthan oder 1,3,3-Trimethyl-2-oxabicyclo[2.2.2]octan. Es handelt sich um eine farblose Flüssigkeit mit würzigem, campherähnlichem Geruch mit einem Schmelzpunkt von +1,5 °C und einem Siedepunkt von 176 bis 177 °C, welche in Wasser unlöslich, aber mit den meisten organischen Lösemitteln mischbar ist.

Natürlich kommt 1,8-Cineol als Hauptbestandteil des Eukalyptusöls (Eukalyptusöl enthält bis zu 85 Gew.-% 1,8-Cineol), aber auch in anderen Pflanzen vor, so z. B. in Minze, Heilsalbei, Thymian, Basilikum und im Teebaum. Da-rüber hinaus ist 1,8-Cineol beispielsweise in Niaouli-, Juniperus-, Piper-, Cannabis-, Kajeput-, Salbeiöl, Myrtenöl und anderen ätherischen Ölen enthalten.

Technisches 1,8-Cineol, welches im Allgemeinen 99.6-%ig bis 99,8-%ig ist, wird im Allgemeinen durch fraktionierte Destillation von Eukalyptusöl gewonnen.

1,8-Cineol wird insbesondere als Expektorans bei Bronchialkatarrhen und anderen Atemwegserkrankungen vorwiegend in der Veterinärmedizin, aber darüber hinaus auch als Aromastoff in der Parfümindustrie angewendet. Darüber hinaus wird 1,8-Cineol in der Zahnmedizin bei der Revision von Wurzelfüllungen verwendet.

In physiologischer Hinsicht wirkt 1,8-Cineol in den oberen und unteren Atemwegen, insbesondere in der Lunge und den Nebenhöhlen, schleimlösend und bakterizid. Außerdem hemmt es bestimmte Neurotransmitter, welche für die Verengung von Bronchien verantwortlich sind. Bei chronisch-obstruktiven Lungenerkrankungen und Asthma bronchiale kann durch Gabe von reinem 1,8-Cineol die Lungenfunktion verbessert werden. Aufgrund seiner kortikosteroidartigen Wirkung wird es im Fall schwerwiegender Atemwegserkrankungen als Substitut zu oder in Co-Medikation mit Kortikosteroiden unter systemischer Applikation angewendet. 1,8-Cineol kann grundsätzlich sowohl topisch, z. B. inhalativ, oder aber systemisch, insbesondere in Form von Kapseln, angewendet werden.

Als Wirkstoff besitzt 1,8-Cineol also schleimlösende wie entzündungshemmende Wirkungen. Bei systemischer Anwendung wird 1,8-Cineol leicht resorbiert und gelangt über die Blutbahn in den Atmungsorganen zur Wirkung. 1,8-Cineol kann auf diese Weise beispielsweise entzündliche Sekrete sowie zähen Schleim in den Luftwegen verflüssigen und entzündlichen Prozessen in den Atemwegen entgegenwirken. Ein Sekretstau wird so verhindert, das Abhusten erleichtert, die Funktion der für die Reinigung zuständigen Flimmerhärchen in den Bronchien und der Nase unterstützt und somit die Durchlüftung der Atemwege verbessert. Im Bereich der oberen Luftwege schwinden die Behinderung der Nasenatmung bei Schnupfen und die Benommenheit des Kopfes.

Für weitergehende Einzelheiten zu dem Wirkstoff 1,8-Cineol kann beispielsweise verwiesen werden auf Römpp Chemielexikon, Georg Thieme Verlag, Stuttgart/New York, 10. Auflage, Band 1, 1996, Seite 752, Stichwort: "Cineol", sowie die dort referierte Literatur.

Im Rahmen der vorliegenden Erfindung wurde in völlig überraschender Weise eine synergistische Steigerung der Wirkeffizienz von 1,8-Cineol infolge der gemeinsamen Verabreichung mit dem Vitamin D-Rezeptoragonisten (VDA) gefunden, wie insbesondere nachfolgend auch auf Basis der Ausführungsbeispiele noch im Detail geschildert.

Erfindungsgemäß kann die Menge an (b) 1,8-Cineol in dem erfindungsgemäßen Kombinationstherapeutikum in weiten Bereichen variieren. Um eine ausreichende Wirkeffizienz zu erreichen, ist es jedoch erfindungsgemäß bevorzugt, wenn das Kombinationstherapeutikum das Monoterpen in einer Menge im Bereich von 0,1 Gew.-% bis 95 Gew.-%, insbesondere im Bereich von 0,5 Gew.-% bis 90 Gew.-%, vorzugsweise im Bereich von 1 Gew.-% bis 85 Gew.-%, bevorzugt im Bereich von 2 Gew.-% bis 80 Gew.-%, besonders bevorzugt im Bereich von 5 Gew.-% bis 75 Gew.-%, bezogen auf das Kombinationstherapeutikum, enthält.

Insbesondere sollte das Kombinationstherapeutikum nach der Erfindung (b) 1,8-Cineol in einer Menge im Bereich von 5 mg bis 1.000 mg, vorzugsweise im Bereich von 10 mg bis 750 mg, bevorzugt im Bereich von 50 mg bis 500 mg, besonders bevorzugt im Bereich von 75 mg bis 300 mg, bezogen auf eine Dosier- und/oder Applikationseinheit des Kombinationstherapeutikums, enthalten.

Im Hinblick auf die Wirksamkeit des erfindungsgemäßen Kombinationstherapeutikums für die oben angeführten Indikationen kommt auch dem mengenspezifischen Verhältnis von (a) Vitamin D-Rezeptoragonist (VDA) einerseits und (b) 1,8-Cineol andererseits eine große Bedeutung zu. In diesem Zusammenhang ist es erfindungsgemäß vorteilhaft, wenn das Kombinationstherapeutikum (a) den Vitamin D-Rezeptoragonisten (VDA) und (b) 1,8-Cineol in einem gewichtsbezogenen Mengenverhältnis von [(a) : (b)] im Bereich von 1 : 100.000 bis 1 : 1, insbesondere im Bereich von 1 : 10.000 bis 1 : 10, vorzugsweise im Bereich von 1 : 1.000 bis 1 : 100, aufweist.

Gemäß einer weiteren erfindungsgemäßen Ausführungsform kann das Kombinationstherapeutikum mindestens einen mit (a) dem Vitamin D-Rezeptoragonisten (VDA) und/oder (b) 1,8-Cineol mischbaren und/oder hierin löslichen, insbesondere bei 20 °C und Atmosphärendruck flüssigen, physiologisch unbedenklichen Träger (Exzipienten) enthalten. In diesem Zusammenhang kann der Träger ausgewählt sein aus der Gruppe von fetten Ölen, bevorzugt Triglyceriden, besonders bevorzugt mittelkettigen Triglyceriden (*Medium Chain Triglycerides,* MCT), ganz besonders bevorzugt Triglyceriden mit C₆-C₁₂-Fettsäureresten. In diesem Zusammenhang kann zudem der Träger in einem (a) Vitamin D-Rezeptoragonist (VDA)/Träger-Mengenverhältnis und/oder in einem (b) 1,8-Cineol /Träger-Mengenverhältnis, unabhängig voneinander, im Bereich von 1.000 : 1 bis 1 : 1.000, insbesondere im Bereich von 100 : 1 bis 1 : 100, eingesetzt sein.

Der Träger bzw. Exzipient ist selbst pharmakologisch nicht wirksam, bildet aber mit den Wirkstoffen, insbesondere mit dem Vitamin D-Rezeptoragonisten (VDA) und/oder dem Monoterpen, eine vorzugsweise homogene Mischung oder Lösung, was erfindungsgemäß von Vorteil ist.

Der Begriff der Öle, wie er erfindungsgemäß für die vorstehenden Träger bzw. Exzipienten verwendet wird, ist eine Sammelbezeichnung für Flüssigkeiten, welche sich nicht mit Wasser mischen lassen. Der Begriff der fetten Öle, wie er in diesem Zusammenhang erfindungsgemäß verwendet wird, bezeichnet speziell Fette, d. h. Gemische von Fettsäuretriglyceriden, welche bei Raumtemperatur (insbesondere 20 °C) und Atmosphärendruck flüssig sind; insbesondere bezeichnet dieser Begriff Ester des dreiwertigen Alkohols Glycerin (Propan-1,2-3-triol) mit drei, meist verschiedenen, überwiegend geradzahligen und unverzweigten aliphatischen Monocarbonsäuren, den so genannten Fettsäuren. Verbindungen dieser Art werden auch Triglyceride genannt (nach IUPAC-Empfehlung: Triacylglycerine). Triglyceride, synonym auch als Glycerol-Triester bezeichnet, sind also dreifache Ester des dreiwertigen Alkohols Glycerin mit drei Säuremolekülen, wobei die Vorsilbe "*tri*" auf drei Acyl-Säurereste, die mit Glycerin verestert sind, verweist.

Speziell mittelkettige Triglyceride, wie sie erfindungsgemäß bevorzugt als Träger bzw. Exzipient für den Wirkstoff 1,8-Cineol zum Einsatz kommen, sind insbesondere halbsynthetische neutrale Glycerinester von gesättigten, im Allgemeinen unverzweigten Monocarbonsäuren mittlerer Kettenlänge (d. h. C₆-C₁₂). Insbesondere bezeichnet der Begriff der mittelkettigen Triglyceride Gemische von Triglyceriden gesättigter Fettsäuren, hauptsächlich Caprylsäure (Octansäure) und Caprinsäure (Decansäure). Mittelkettige Triglyceride können im Allgemeinen aus Öl hergestellt werden, das aus dem festen und getrockneten Teil des Endosperms von *Cocos nucifera* L. und/oder aus dem getrockneten Endosperm von *Elaeis guineenses* Jacq. extrahiert wird. Für weitergehende Einzelheiten zu dem Begriff der mittelkettigen Triglyceride kann beispielsweise verwiesen werden auf die Monographie Ph. Eur. 6., Ausgabe, Grundwerk 2008, Seiten 4224 bis 4226, sowie auf die Zeitschrift für Ernährungswissenschaft, Band 13, Heft 1/2, 1973, Seiten 6 ff., D. Sailer et al. "Mittelkettige Triglyceride - Klinische Physiologie und Anwendung*"*)*.*

Zur weiterführenden Steigerung bzw. individuellen Anpassung bzw. Maßschneiderung der Wirksamkeit des erfindungsgemäßen Kombinationstherapeutikums im Hinblick auf die zugrundeliegende Erkrankungen kann es zudem vorgesehen sein, dass das Kombinationstherapeutikum nach der Erfindung (c) mindestens einen weiteren pharmakologisch wirksamen Inhaltsstoff und/oder Wirkstoff umfasst.

In diesem Zusammenhang kann es erfindungsgemäß vorgesehen sein, dass (c) der pharmakologisch wirksame Inhaltsstoff und/oder Wirkstoff ein insbesondere systemisch applizierbares und/oder systemisches, vorzugsweise peroral applizierbares Kortikosteroid, insbesondere Glukokortikosteroid, ist. In diesem Zusammenhang kann das Glukokortikosteroid ausgewählt sein aus der Gruppe von Dexamethason, Prednison, Prednisolon, Betamethason, Rimexolon, Paramethason, Hydrocortison und deren physiologisch verträglichen Derivaten, insbesondere Salzen und Estern, sowie Mischungen und Kombinationen der vorgenannten Verbindungen.

Erfindungsgemäß kann es auch vorgesehen sein, dass (c) der pharmakologisch wirksame Inhaltsstoff und/oder Wirkstoff ausgewählt ist aus der Gruppe von Antiphlogistika, insbesondere nichtsteroidalen Antiphlogistika, vorzugsweise Acetylsalicylsäure und/oder Paracetamol; beta-Sympathomimetika, vorzugsweise beta-2-Sympathomimetika; Parasympatholytika und/oder Vagolytika; und Anticholinergika; sowie Mischungen und Kombinationen der vorgenannten Verbindungen. In diesem Zusammenhang kommt - ohne sich auf diese Theorie beschränken zu wollen-insbesondere den nichtsteroidalen Antiphlogistika eine wichtige Rolle in der Hemmung von COX II-Metaboliten, insbesondere Prostaglandinen, zu, was zu einer weiteren Steigerung der Wirksamkeit des erfindungsgemäßen Kombinationstherapeutikum führt.

Insbesondere sollte das Kombinationstherapeutikum nach der Erfindung (c) den weiteren pharmakologisch wirksamen Inhaltsstoff und/oder Wirkstoff in einer Menge im Bereich von 0,001 Gew.-% bis 10 Gew.-%, insbesondere im Bereich von 0,01 Gew.-% bis 5 Gew.-%, vorzugsweise im Bereich von 0,1 Gew.-% bis 2 Gew.-%, bezogen auf das Kombinationstherapeutikum, enthalten.

Erfindungsgemäß kann es gleichermaßen vorgesehen sein, dass das Kombinationstherapeutikum nach der Erfindung (d) mindestens ein weiteres Vitamin umfasst. In diesem Zusammenhang kann das Vitamin ausgewählt sein aus der Gruppe von Vitamin A, Vitamin C und Vitamin E, insbesondere Vitamin C und Vitamin E. Zudem kann das erfindungsgemäße Kombinationstherapeutikum mindestens einen weiteren Inhaltsstoff aufweisen, insbesondere ausgewählt aus der Gruppe von Verarbeitungshilfsstoffen, Stabilisatoren, insbesondere organischen Lösemitteln, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

Was das erfindungsgemäße Kombinationstherapeutikum weiterhin anbelangt, so kann dieses topisch applizierbar sein und/oder appliziert werden. Gleichermaßen kann das Kombinationstherapeutikum nach der Erfindung systemisch, insbesondere peroral applizierbar sein und/oder appliziert werden.

In diesem Zusammenhang kommt die topische Applizierung des Kombinationstherapeutikums nach der Erfindung insbesondere in Bezug auf die diesbezügliche Verwendung zur Behandlung von insbesondere entzündlichen Atemwegserkrankungen in Betracht. In diesem Zusammenhang ist der Begriff des topisch, insbesondere inhalativ zu applizierenden Kombinationstherapeutikums erfindungsgemäß sehr breit zu verstehen, was insbesondere sämtliche dem Fachmann zur Behandlung von entzündlichen Atemwegserkrankungen bekannten Darreichungsformen umfasst, welche sich für die topische, insbesondere inhalative Anwendung eignen.

Weiterhin kommt die systemische Verabreichung des erfindungsgemäßen Kombinationstherapeutikums insbesondere im Hinblick auf die Behandlung von entzündlichen Erkrankungen des Verdauungstrakts, insbesondere des Darms, in Betracht. Eine systemische Applikation kommt jedoch grundsätzlich auch für die Behandlung von insbesondere entzündlichen Atemwegserkrankungen in Betracht, insbesondere im Hinblick auf eine systemische Aufnahme der Wirkkomponenten mit entsprechender Beeinflussung von lokalen und systemischen (Krankheits-)Prozessen. In diesem Zusammenhang kann das Kombinationstherapeutikum insbesondere in Form von dünndarmlöslichen Tabletten bzw. Kapseln oder aber in Form von sogenannten Pellets verabreicht werden, was insbesondere im Hinblick auf die Behandlung von entzündlichen Darmerkrankungen zu hohen Wirkstoffkonzentrationen direkt am Wirkort führt.

Denn insbesondere Monoterpene werden nach systemischer Gabe, insbesondere in Form von magensaftresistenten, aber dünndarmlöslichen Kapseln, sehr gut aufgenommen und entsprechend ihren physikalischen Eigenschaften in den Alveolen freigesetzt und gelangen somit in die Ausatmungsluft. Hierdurch können die Monoterpene gerade auch in den kleinsten, peripher gelegenen Atemwegen - im Unterschied zu inhalativen Atemwegstherapeutika allein - eine entzündungshemmende Wirkung vermitteln, insbesondere in Kombination mit den erfindungsgemäß eingesetzten Vitamin D-Rezeptoragonisten (VDA).

Im Allgemeinen kann das Kombinationstherapeutikum nach der Erfindung in Tagesdosen von (a) 10 µg bis 1.000 µg, insbesondere 20 µg bis 800 µg, vorzugsweise 30 µg bis 500 µg, Vitamin D-Rezeptoragonist (VDA) verabreicht werden. Insbesondere kann das Kombinationstherapeutikum zur Verabreichung in einer Tagesdosis von (a) 10 µg bis 1.000 µg, insbesondere 20 µg bis 800 µg, vorzugsweise 30 µg bis 500 µg, Vitamin D-Rezeptoragonist (VDA) hergerichtet sein. Zudem kann das Kombinationstherapeutikum in Tagesdosen von (b) 50 mg bis 3.000 mg, insbesondere 100 mg bis 1.500 mg, vorzugsweise 300 mg bis 1.000 mg, Monoterpen verabreicht werden. Insbesondere kann das Kombinationstherapeutikum zur Verabreichung in einer Tagesdosis von (b) 50 mg bis 3.000 mg, insbesondere 100 mg bis 1.500 mg, vorzugsweise 300 mg bis 1.000 mg, Monoterpen hergerichtet sein.

Die vorgenannten Tagesdosen können sich vorteilhafterweise auf eine, zwei oder mehrere Einzelgaben verteilen. Zudem liegt es im Ermessen des Fachmanns, von den oben genannten Tagesdosen abzuweichen, insbesondere im Hinblick auf die zugrundeliegende Indikation und Schwere der Erkrankung. Dies versteht sich für den Fachmann aber von selbst.

Was das erfindungsgemäße Kombinationstherapeutikum weiterhin anbelangt, so kann dieses, wie zuvor angeführt, in einer peroral, insbesondere inhalativ applizierbaren Form, vorzugsweise in Suspensions- und/oder Pulverform, bevorzugt als Inhalations- und/oder Rachensprayformulierung bzw. Aerosolspray zur Inhalation und/oder Applikation im Rachenraum, vorliegen.

Alternativ kann es erfindungsgemäß auch vorgesehen sein, dass, wie zuvor angeführt, das Kombinationstherapeutikum nach der Erfindung in einer peroral applizierbaren Darreichungsform, insbesondere in Form von Kapseln, Pellets und/oder Tabletten, vorzugsweise in Form von Kapseln oder Pellets, bevorzugt Kapseln, vorliegt. Zudem liegt es im Rahmen der vorliegenden Erfindung, wenn das Kombinationstherapeutikum in Form einer peroralen, magensaftresistenten, aber dünndarmlöslichen Darreichungsform, insbesondere Kapseln und/oder Pellets, bevorzugt Kapseln, vorliegt.

Erfindungsgemäß kann das Kombinationstherapeutikum nach der Erfindung zur prophylaktischen und/oder therapeutischen Behandlung, insbesondere Kombinationstherapie und/oder Co-Medikation, von Atemwegserkrankungen, insbesondere bronchopulmonalen Erkrankungen und/oder zur prophylaktischen und/oder therapeutischen Behandlung, insbesondere Kombinationstherapie und/oder Co-Medikation, von insbesondere entzündlichen Erkrankungen des Verdauungstraktes, insbesondere des Darms, eingesetzt werden.

In diesem Zusammenhang kann das Kombinationstherapeutikum nach der Erfindung (a) den Vitamin D-Rezeptoragonisten (VDA) einerseits und (b) 1,8-Cineol andererseits, gegebenenfalls gemeinsam mit (c) dem weiteren pharmakologisch wirksamen Inhaltsstoff und/oder Wirkstoff und/oder (d) dem weiteren Vitamin und/oder dem weiteren Inhaltsstoff, in einer gemeinsamen und/oder einzigen Zusammensetzung aufweisen.

Im Rahmen der vorliegenden Erfindung kann es auch vorgesehen sein, dass das erfindungsgemäße Kombinationstherapeutikum, insbesondere wie zuvor definiert, bevorzugt zur Verwendung bei der prophylaktischen und/oder therapeutischen Behandlung von insbesondere entzündlichen Atemwegserkrankungen, insbesondere bronchopulmonalen Erkrankungen, oder zur Verwendung bei der prophylaktischen und/oder therapeutischen Behandlung von insbesondere entzündlichen Erkrankungen des Verdauungstraktes, insbesondere des Darms, wobei das Kombinations-therapeutikum - insbesondere in jeweils wirksamen, vorzugsweise pharmazeutisch wirksamen Mengen - mindestens einen Vitamin D-Rezeptoragonisten (VDA) einerseits und 1,8-Cineol andererseits umfasst, in Form eines Kits (*"Kit of Parts"*) vorliegt bzw. (a) den Vitamin D-Rezeptoragonisten (VDA) einerseits und (b) 1,8-Cineol andererseits räumlich voneinander getrennt, insbesondere in voneinander verschiedenen Zusammensetzungen, aufweist. Dies kann für die weiteren Inhalts- bzw. Wirkstoffe in entsprechender Weise gelten. Diesbezüglich kommen die jeweils zuvor beschriebenen Applikationsformen für die jeweiligen Komponenten, unabhängig voneinander, in Betracht.

Im Rahmen einer Co-Medikation, beispielsweise mit Kortikosteroiden, kann es erfindungsgemäß in nicht beschränkender Weise vorgesehen sein, dass einerseits der Vitamin D-Rezeptoragonist (VDA), insbesondere gemeinsam mit dem Monoterpen, systemisch, insbesondere peroral (= *erste und zweite systemisch applizier-bare Komponente*), beispielsweise in Form von dünndarmlöslichen Kapseln, verabreicht wird und andererseits - gemeinsam bzw. in Kombination hiermit oder aber zeitlich versetzt hierzu - mindestens ein topisch applizierbares, insbesondere inhalatives Atemwegstherapeutikum, insbesondere Kortikosteroid, (= *topische applizier-bare Komponente)* insbesondere zu Zwecken der prophylaktischen und/oder therapeutischen Behandlung von Atemwegserkrankungen, insbesondere bronchopulmonalen Erkrankungen, verabreicht wird.

Im Rahmen einer Co-Medikation, beispielsweise mit Kortikosteroiden, kann es erfindungsgemäß in nicht beschränkender Weise zudem vorgesehen sein, dass einerseits der Vitamin D-Rezeptoragonist (VDA), insbesondere gemeinsam mit dem Monoterpen, systemisch, insbesondere peroral (= *erste und zweite systemisch applizierbare Komponente*), beispielsweise in Form von dünndarmlöslichen Kapseln, verabreicht wird und andererseits - gemeinsam bzw. in Kombination hiermit oder aber zeitlich versetzt hierzu - mindestens ein systemisch applizierbares, insbesondere peroral applizierbares Kortikosteroid (= *zweite systemisch applizierbare Komponente)* insbesondere zu Zwecken der prophylaktischen und/oder therapeutischen Behandlung von insbesondere entzündlichen Darmerkrankungen, verabreicht wird. Die ersten und zweiten systemisch applizierbaren Komponenten können auch in einer gemeinsamen bzw. einzigen Zusammensetzung vorliegen bzw. verabreicht werden. Gleichermaßen können auch die erste und die zweite systemisch applizierbare Komponente in voneinander verschiedenen Zusammensetzungen verabreicht werden.

Das erfindungsgemäße Kombinationstherapeutikum, wie zuvor definiert, eignet sich zur Verwendung bei der prophylaktischen und/oder therapeutischen Behandlung von insbesondere entzündlichen Atemwegserkrankungen, insbesondere bronchopulmonalen Erkrankungen, bzw. zur Herstellung eines Arzneimittels zur prophylaktischen und/oder therapeutischen Behandlung von insbesondere entzündlichen Atemwegserkrankungen, insbesondere bronchopulmonalen Erkrankungen, wobei das Kombinationstherapeutikum - insbesondere in jeweils wirksamen, vorzugsweise pharmazeutisch wirksamen Mengen - mindestens einen Vitamin D-Rezeptoragonisten (VDA) einerseits und 1,8-Cineol andererseits umfasst.

Das erfindungsgemäße Kombinationstherapeutikum, wie zuvor definiert, eignet sich zur Verwendung bei der prophylaktischen und/oder therapeutischen Behandlung von insbesondere entzündlichen Erkrankungen des Verdauungstraktes, insbesondere des Darms, bzw. zur Herstellung eines Arzneimittels zur prophylaktischen und/oder therapeutischen Behandlung von insbesondere entzündlichen Erkrankungen des Verdauungstraktes, insbesondere des Darms, wobei das Kombinationstherapeutikum - insbesondere in jeweils wirksamen, vorzugsweise pharmazeutisch wirksamen Mengen - mindestens einen Vitamin D-Rezeptoragonisten (VDA) einerseits und 1,8-Cineol andererseits umfasst.

Darüber hinaus betrifft die vorliegende Erfindung die Verwendung eines Vitamin D-Rezeptoragonisten (VDA) zur Herstellung eines Arzneimittels zur prophylaktischen und/oder therapeutischen Behandlung von insbesondere entzündlichen Atemwegserkrankungen, insbesondere bronchopulmonalen Erkrankungen, oder zur Herstellung eines Arzneimittels zur prophylaktischen und/oder therapeutischen Behandlung von insbesondere entzündlichen Erkrankungen des Verdauungstraktes, insbesondere des Darms. Erfindungsgemäß wird der Vitamin D-Rezeptoragonist (VDA) zusammen mit 1,8-Cineol verabreicht und/oder eingesetzt werden.

Weiterhin kann das erfindungsgemäße Kombinationstherapeutikum, wie zuvor definiert, mit mindestens einem weiteren Therapeutikum zur prophylaktischen und/oder therapeutischen Behandlung, insbesondere Kombinationstherapie und/oder Co-Medikation, von Atemwegserkrankungen, insbesondere bronchopulmonalen Erkrankungen, und/oder zur prophylaktischen und/oder therapeutischen Behandlung, insbesondere Kombinationstherapie und/oder Co-Medikation, von insbesondere entzündlichen Erkrankungen des Verdauungstraktes, insbesondere des Darms, verwendet werden, wobei das Kombinationstherapeutikum - insbesondere in jeweils wirksamen, vorzugsweise pharmazeutisch wirksamen Mengen-mindestens einen Vitamin D-Rezeptoragonisten (VDA) einerseits und 1,8-Cineol andererseits umfasst.

Diesbezüglich kann das weitere Therapeutikum ein topisch applizierbares Atemwegstherapeutikum, insbesondere ein inhalatives Atemwegstherapeutikum, sein, insbesondere im Hinblick auf die Behandlung von insbesondere entzündlichen Erkrankungen der Atemwege, vorzugsweise bronchopulmonalen Erkrankungen.

In diesem Zusammenhang ist es erfindungsgemäß bevorzugt, wenn das topisch applizierbare, insbesondere inhalative Atemwegstherapeutikum aus Bronchodilatatoren und Bronchospasmolytika ausgewählt ist. Der Begriff "Bronchodilatatoren", wie er erfindungsgemäß verwendet wird, bezeichnet insbesondere solche Substanzen, welche die Bronchien und Bronchiolen erweitern bzw. dilatieren und auf diese Weise den Atemwegswiderstand herabsetzen, wohingegen der Begriff der "Bronchospasmolytika", wie er erfindungsgemäß verwendet wird, solche Substanzen bezeichnet, welche den Bronchialmuskeltonus herabsetzen und zum Teil die Freisetzung von Mediatorsubstanzen aus den Mastzellen hemmen und die mukoziliäre Clearance, d. h. den insbesondere vom respiratorischen Epithel getragenen Selbstreinigungsmechanismus der Bronchien, steigern.

In diesem Zusammenhang kann das topisch applizierbare, insbesondere inhalative Atemwegstherapeutikum ausgewählt sein aus der Gruppe von Bronchodilatatoren und Bronchospasmolytika. Insbesondere kann das topisch applizierbare, insbesondere inhalative Atemwegstherapeutikum ausgewählt sein aus der Gruppe von (i) Kortikosteroiden, insbesondere Glukokortikoiden, (ii) Sympathomimetika, insbesondere beta-Sympathomimetika, vorzugsweise beta-2-Sympathomimetika; (iii) Phosphodiesterasehemmern; (iv) Parasympatholytika und/oder Vagolytika; (v) Anticholinergika; sowie Mischungen und Kombinationen der vorgenannten Verbindungen. Besonders bevorzugt kann das topisch applizierbare, insbesondere inhalative Atemwegstherapeutikum ausgewählt sein aus der Gruppe von Kortikosteroiden, insbesondere Glukokortikoiden, beta-2-Sympathomimetika und Anticholinergika sowie deren Mischungen und Kombinationen.

Gemäß einer erfindungsgemäß bevorzugten Ausführungsform kann das topisch applizierbare, insbesondere inhalative Atemwegstherapeutikum ein Kortikosteroid, insbesondere Glukokortikoid, sein, vorzugsweise ausgewählt aus der Gruppe von Beclometason, Mometason, Budesonid, Flunisolid, Fluticason, Triamcinolon und deren physiologisch verträglichen Derivaten, insbesondere Salzen und Estern, sowie Mischungen und Kombinationen der vorgenannten Verbindungen. In diesem Zusammenhang kann das Kortikosteroid in Tagesdosen von 50 bis 1.000 µg, insbesondere 75 bis 800 µg, besonders bevorzugt 100 bis 600 µg, verabreicht werden. Insbesondere kann das Kombinationstherapeutikum zur Verabreichung des Kortikosteroids in einer Tagesdosis von 50 bis 1.000 µg, insbesondere 75 bis 800 µg, besonders bevorzugt 100 bis 600 µg, hergerichtet sein.

Auch diesbezüglich kann von den zuvor angeführten Tagesdosen im Hinblick auf die konkrete Indikation und Schwere der Erkrankung abgewichen werden, was dem Fachmann an sich wohlbekannt ist.

Die Kortikosteroide sind eine Gruppe von ca. 50 in der Nebennierenrinde gebildeten Steroidhormonen sowie chemisch vergleichbaren synthetischen Stoffen, wobei alle Kortikosteroide aus dem Ausgangsstoff Cholesterin entstehen und als gemeinsames Grundgerüst das Progesteron (Delta-4-Pregnen-3,20-Dion) aufweisen. Die Kortikosteroide lassen sich nach ihrer biologischen Wirkung bzw. ihrem Bildungsort in drei Gruppen einteilen, nämlich Mineralokortikoide, Glukokortikoide und Androgene. Die erfindungsgemäß bevorzugt eingesetzten Glukokortikoide zählen somit zu den Kortikosteroiden.

Gemäß einer weiteren erfindungsgemäßen Ausführungsform kann das topisch applizierbare, insbesondere inhalative Atemwegstherapeutikum ein Sympathomimetikum, insbesondere beta-Sympathomimetikum, vorzugsweise beta-2-Sympathomimetikum, sein. Insbesondere kann das beta-Sympathomimetikum ausgewählt sein aus der Gruppe von kurzwirkenden Betamimetika (SABA), insbesondere Albuterol, Fenoterol, Hexoprenalin, Levalbuterol, Metaproterenol, Orciprenalin, Pirbuterol, Reproterol, Salbutamol und/oder Terbutalin, und langwirkenden Betamimetika (LABA), insbesondere Salmeterol und/oder Formoterol, sowie Mischungen und Kombinationen der vorgenannten Verbindungen.

Der Begriff der Sympathomimetika kennzeichnet insbesondere solche Substanzen, welche die Wirkung des Sympathikus nachahmen. Speziell die beta-Sympathomimetika (synonym auch als "Betamimetika" bezeichnet) besitzen überwiegend Wirkung auf die beta-Rezeptoren. Ganz speziell die erfindungsgemäß bevorzugten beta-2-Sympathomimetika führen an der glatten Muskulatur (beta-2-Rezeptoren) zur Erschlaffung und besitzen bronchospasmolytische Wirkung.

Bei den erfindungsgemäß bevorzugt eingesetzten inhalativen beta-2-Sympathomimetika kann es sich entweder um kurzwirkende Betamimetika (SABA = short acting beta agonists) oder um langwirkende Betamimetika (LABA = long acting beta agonists) handeln. Beispiele für kurzwirkende Betamimetika (SABA) sind insbesondere Albuterol, Fenoterol, Hexoprenalin, Levalbuterol, Metaproterenol, Orciprenalin, Pirbuterol, Reproterol, Salbutamol und/oder Terbutalin. Beispiele für langwirkende Betamimetika (LABA) sind insbesondere Salmeterol und/oder Formoterol.

Gemäß einer weiteren erfindungsgemäßen Ausführungsform kann es vorgesehen sein, dass das topisch applizierbare, insbesondere inhalative Atemwegstherapeutikum ein Anticholinergikum ist, insbesondere aus der Gruppe von Ipratropium, Tiotropium und/oder deren physiologisch verträglichen Derivaten, vorzugsweise Salzen, besonders bevorzugt Ipratropiumbromid und/oder Tiotropiumbromid, sowie Mischungen und Kombinationen der vorgenannten Verbindungen.

Anticholinergika sind solche Substanzen, welche die Wirkung von Acetylcholin unterdrücken, wobei die in Rede stehenden Substanzen gleichermaßen bronchospasmolytische Wirkungen aufweisen.

Gleichermaßen kann es erfindungsgemäß vorgesehen sein, dass mindestens ein weiterer systemischer, insbesondere peroraler Wirkstoff appliziert wird, insbesondere ausgewählt aus der Gruppe von systemischen Phosphodiesterasehemmern, insbesondere Theophyllin; systemischen Leukotrienrezeptorantagonisten, insbesondere Montelukast, Zaforlukast und Pranlukast; systemischen Kortikosteroiden; Antiphlogistika, insbesondere nichtsteroidalen Antiphlogistika, vorzugsweise Acetylsalicylsäure und/oder Paracetamol; sowie deren Mischungen und Kombinationen.

Erfindungsgemäß kann es zudem vorgesehen sein, dass zusätzlich mindestens ein weiterer systemischer, insbesondere peroraler Wirkstoff appliziert wird, insbesondere ausgewählt aus der Gruppe von systemischen Phosphodiesterasehemmern, insbesondere Theophyllin; systemischen Leukotrienrezeptorantagonisten, insbesondere Montelukast, Zaforlukast und Pranlukast; systemischen Kortikosteroiden; Antiphlogistika, insbesondere nichtsteroidalen Antiphlogistika, vorzugsweise Acetylsalicylsäure und/oder Paracetamol; sowie deren Mischungen und Kombinationen.

Es versteht sich für den Fachmann von selbst, dass im Rahmen der erfindungsgemäßen Konzeption die vorgenannten topischen bzw. inhalativen Atemwegstherapeutika auch miteinander kombiniert werden können - und dies auch im Rahmen einer gemeinsamen Zusammensetzung in Kombination mit dem (a) Vitamin D-Rezeptoragonisten (VDA) einerseits und (b) 1,8-Cineol andererseits.

Darüber hinaus kann es im Rahmen der vorliegenden Erfindung vorgesehen sein, dass das weitere Therapeutikum ein systemisch applizierbares Therapeutikum, insbesondere ein peroral applizierbares Therapeutikum, ist. In diesem Zusammenhang kann das Therapeutikum mindestens ein systemisch applizierbares, vorzugsweise peroral applizierbares Kortikosteroid, insbesondere Kortikosteroid, vorzugsweise Glukokortikosteroid, aufweisen. Diesbezüglich kann das Glukokortikosteroid ausgewählt sein aus der Gruppe von Dexamethason, Prednison, Prednisolon, Betamethason, Rimexolon, Paramethason, Hydrocortison und deren physiologisch verträglichen Derivaten, insbesondere Salzen und Estern, sowie Mischungen und Kombinationen der vorgenannten Verbindungen.

Die Verabreichung derartiger systemischer Kortikoide kommt insbesondere im Rahmen der Behandlung von entzündlichen Erkrankungen des Verdauungstraktes, insbesondere des Darms, in Betracht, ist jedoch nicht hierauf beschränkt, da die Verabreichung derartiger systemischer Kortikoiden grundsätzlich auch im Rahmen der Behandlung von bronchopulmonale Erkrankungen in Betracht kommt.

Im Rahmen der erfindungsgemäßen Konzeption können beliebige bronchopulmonale Erkrankungen bzw. Atemwegserkrankungen behandelt werden. Insbesondere ist die Atemwegserkrankung, insbesondere die bronchopulmonale Erkrankung eine entzündliche oder nichtentzündliche, insbesondere entzündliche Erkrankung der oberen oder unteren Atemwege. Insbesondere kann es sich bei der bronchopulmonalen Erkrankung bzw. Atemwegserkrankung um eine entzündliche, insbesondere infektexazerbierte bzw. steroidpflichtige Atemwegserkrankung handeln.

Beispielsweise kann es sich bei der Atemwegserkrankung, insbesondere bronchopulmonalen Erkrankung, um Asthma bronchiale oder Bronchitis handeln.

Weiterhin kann die Atemwegserkrankung, insbesondere die bronchopulmonale Erkrankung, eine chronische obstruktive Lungenerkrankung (COPD) sein, insbesondere eine chronische obstruktive Bronchitis oder ein Lungenemphysem.

Des Weiteren kann die Atemwegserkrankung, insbesondere die bronchopulmonale Erkrankung, eine tabakrauchinduzierte, insbesondere nikotininduzierte akute oder chronische Atemwegserkrankung, insbesondere entzündliche Atemwegserkrankung, sein.

Im Rahmen der erfindungsgemäßen Konzeption können auch Frühformen von COPD, insbesondere im Stadium nach GOLD 0 oder I, oder eine Frühform des Asthma bronchiale, insbesondere im Stadium 0 oder I nach GINA 0 oder I, sein. Insbesondere können im Rahmen der vorliegenden Erfindung Frühformen von COPD, insbesondere im Stadium 0 oder I nach GOLD, oder Frühformen des Asthma bronchiale, insbesondere im Stadium nach GINA 0 oder I, behandelt werden, und auf diese Weise kann eine Exazerbationsprophylaxe vor und nach erfolgter Exazerbation bzw. eine Verhinderung und Verlangsamung des Krankheitsprogresses vor oder nach erfolgter Exazerbation erreicht werden.

Darüber hinaus kann die Atemwegserkrankung eine Erkältungserkrankung oder ein grippaler Infekt sein. Gleichermaßen kann die Atemwegserkrankung Rhinitis und/oder Sinusitis sein.

Gemäß einer alternativen erfindungsgemäßen Ausführungsform kann die entzündliche Erkrankung des Verdauungstraktes, insbesondere des Darms, *Morbus Crohn* und/oder *Colitis ulcerosa* und/oder eine chronisch entzündliche Darmerkrankung (*Inflammatory Bowel Disease,* IBD) sein.

Das erfindungsgemäßen Kombinationstherapeutikum eignet sich darüber hinaus auch zur insbesondere synergistischen Wirkungssteigerung mindestens eines topisch applizierbaren, insbesondere inhalativen Atemwegstherapeutikums bei der prophylaktischen und/oder therapeutischen Behandlung von Atemwegserkrankungen, insbesondere bronchopulmonalen Erkrankungen.

Darüber hinaus eignet sich das erfindungsgemäße Kombinationstherapeutikum auch zur insbesondere synergistischen Steigerung der antiinflammatorischen und/oder antioxidativen Wirkung topischer, insbesondere inhalativer Kortikosteroide, insbesondere Glukokortikoide.

Gleichermaßen eignet sich das Kombinationstherapeutikum nach der Erfindung zur Dosisreduktion von topisch applizierbaren, insbesondere inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen.

Gemäß einer weiteren erfindungsgemäßen Ausführungsform eignet sich das Kombinationstherapeutikum nach der Erfindung auch zur Induktion und/oder Verstärkung des steroidpermissiven Effektes von topisch applizierbaren, insbesondere inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen.

Zudem eignet sich das erfindungsgemäße Kombinationstherapeutikum auch zur Vermeidung oder Reduktion eines Gewöhnungseffektes von beta-Sympathomimetika bei der prophylaktischen und/oder therapeutischen Behandlung von Atemwegserkrankungen, insbesondere bronchopulmonalen Erkrankungen, insbesondere unter Dauertherapie bei allen Schweregraden von COPD und Asthma bronchiale.

Zudem eignet sich das erfindungsgemäße Kombinationstherapeutikum nach der Erfindung auch in Kombination mit mindestens einem topisch applizierbaren, insbesondere inhalativen Atemwegstherapeutikum, insbesondere in Kombination mit einem inhalativen Kortikosteroid, zur Reduktion des Bedarfs oder zum Ersatz systemischer Kortikosteroide oder anderer antiinflammatorisch und/oder immunsuppressiv wirkender systemischer Substanzen bei der prophylaktischen und/oder therapeutischen Behandlung von Atemwegserkrankungen, insbesondere bronchopulmonalen Erkrankungen.

Gemäß einer noch weiterführenden erfindungsgemäßen Ausführungsform eignet sich das Kombinationstherapeutikum nach der Erfindung auch in Kombination mit mindestens einem topisch applizierbaren, insbesondere inhalativen Atemwegstherapeutikum, insbesondere in Kombination mit einem inhalativen Kortikosteroid und gegebenenfalls einem inhalativen beta-2-Sympathomimetikum, zur Optimierung der Basistherapie bei Asthma bronchiale und COPD.

Insbesondere betrifft die vorliegende Erfindung auch die Behandlung der systemischen Organbeteiligung bei sämtlichen Schwereformen von COPD.

Insbesondere eignet sich das erfindungsgemäße Kombinationstherapeutikum auch zur Modulation und Hemmung COPD-abhängiger und/oder COPD-unabhängiger Alterungsprozesse, insbesondere mit dem Ziel der Reduktion von Morbidität, der Erhöhung der Lebensqualität und/oder Lebenserwartung.

Insbesondere eignet sich das erfindungsgemäße Kombinationstherapeutikum auch zur kombinierten antiinflammatorischen und/oder antioxidativen Therapie von persistierenden und/oder progredienten Atemwegserkrankungen, insbesondere entzündlichen Atemwegserkrankungen, nach Aufgabe des Rauchens, gegebenenfalls unter Co-Medikation mit anderen Atemwegstherapeutika, insbesondere mit dem Ziel der Retardierung der Emphysementwicklung, der respiratorischen Insuffizienz und/oder der Entwicklung peripherer Atemwegsobstruktionen.

Das erfindungsgemäße Kombinationstherapeutikum eignet sich insbesondere bei der Verwendung zur gegenseitigen Wirkverstärkung (a) des Vitamin D-Rezeptoragonisten (VDA) einerseits und (b) 1,8-Cineol andererseits, insbesondere für die prophylaktische und/oder therapeutische Behandlung saisonaler Vitamin D₃-Mangelzustände, insbesondere winterlicher Vitamin D₃-Mangelzustände, vorzugsweise zur Besserung der Infektabwehr und zur verbesserten entzündungshemmenden Therapie der Atemwege, insbesondere mit dem Ziel, akute und chronische Exazerbationen bei chronisch obstruktiver Bronchitis, Asthma und/oder chronisch entzündlichen und/oder allergischen Erkrankungen, insbesondere bei chronischer Rhinosinusitis und/oder allergischer Rhinitis, der oberen Atemwege zu reduzieren.

Insbesondere eignet sich das Kombinationstherapeutikum nach der Erfindung zur insbesondere synergistischen Intensivierung der antiinfektiösen, antiallergischen, entzündungshemmenden und/oder antioxidativen Wirkung von Monoterpenen, insbesondere von 1,8-Cineol. In diesem Zusammenhang kann die perorale Darreichungsform, bezogen auf die Applikation- bzw. Dosiseinheit, den Vitamin D-Rezeptoragonisten in Mengen von 10 µg bis 15 µg und das Monoterpen in Mengen von 200 mg bis 300 mg enthalten.

Gleichermaßen eignet sich das erfindungsgemäße Kombinationstherapeutikum zur lokalen Applikation und/oder Verabreichung, insbesondere nasalen und/oder inhalativen Applikation und/oder Verabreichung, oder zur systemischen Applikation und/oder Verabreichung zur prophylaktischen und/oder therapeutischen Behandlung von viralen und/oder bakteriellen Erkältungserkrankungen, insbesondere in Kombination mit mindestens einer weiteren Wirksubstanz, vorzugsweise mit Vitaminen, insbesondere Vitamin C und oder Vitamin E, Acetylsalicylsäure und/oder mindestens einem weiteren nicht-steroidalen Antiphlogistikum, insbesondere ausgewählt aus der Gruppe der NSAIDS, oder Paracetamol, insbesondere zur Verstärkung der Wirksamkeit der in Kombination eingesetzten Wirksubstanzen, insbesondere mit dem Ziel der Verbesserung und Reduktion des Krankheitsverlaufs von Erkältungskrankheiten.

Darüber hinaus eignet sich das erfindungsgemäße Kombinationstherapeutikum nach der Erfindung auch zur Co-Medikation mit insbesondere nasal-topischen Glukokortikosteroiden, insbesondere zur Intensivierung und/oder Verbesserung der Wirksamkeit und/oder zur Wirkeffizienzsteigerung von Leitlinientherapien für obere Atemwegserkrankungen, insbesondere der akuten, allergischen, chronischen und/oder chronisch rezidivierenden polypoiden Rhinitis und/oder Rhinosinusitis.

Zudem eignet sich das Kombinationstherapeutikum zur Verwendung bei der Co-Medikation zur Intensivierung und/oder Verbesserung der Wirksamkeit und/oder zur Wirkeffizienzsteigerung von Leitlinientherapien, insbesondere auf Basis topisch-nasaler Steroide und/oder alpha-1-Sympathomimetika, für Erkrankungen der oberen Atemwege, insbesondere akuter, allergischer und/oder chronischer Rhinitis und/oder Rhinosinusitis, und/oder zur Verwendung bei der zur Co-Medikation zur Intensivierung und/oder Verbesserung der Wirksamkeit und/oder zur Wirkeffizienzsteigerung von Leitlinientherapien, insbesondere auf Basis von LABA, ICS (inhalatives Kortikosteroid), LAMA (long acting muscarinic antagonist), LABA in Kombination mit ICS, LABA und LAMA, LABA in Kombination mit ICS und LAMA), für Erkrankungen der unteren Atemwege, insbesondere Asthma, COPD und/oder chronische Lungenemphyseme, durch Zigarettenrauch oder Feinpartikel oder andere Umweltnoxen induzierte und/oder assoziierte Atemwegserkrankungen, insbesondere mit dem Ziel einer Induktion und/oder Verstärkung von steroidpermissiven Effekten, insbesondere um den progressiven Verlauf von Obstruktionen und/oder Emphysemen, Exazerbationen und/oder den Steroidbedarf zu reduzieren. Insbesondere kann das Kombinationstherapeutikum in diesem Fall als dünndarmlösliche Kapsel vorliegen.

Gleichermaßen eignet sich das erfindungsgemäße Kombinationstherapeutikum zur Verwendung bei der Co-Medikation und/oder als Zusatztherapie, insbesondere in Form eines Kits, mit mindestens einem weiteren Wirkstoff, wobei der Wirkstoff ausgewählt ist aus der Gruppe von systemischen Glukokortikosteroiden (SCS), intravenösen Substitutionstherapien mit alpha-1-Antitrypsin, IgG-Immunglobulinen, insbesondere bei AK-Mangelsyndrom, Phosphodiesteraseinhibitoren, insbesondere Roflumilast und/oder Theophyllin, zur prophylaktischen und/oder therapeutischen Behandlung von Erkrankungen der oberen Atemwege, insbesondere akuter, allergischer und/oder chronischer Rhinitis und/oder Rhinosinusitis, und/oder zur prophylaktischen und/oder therapeutischen Behandlung von Erkrankungen der unteren Atemwege, insbesondere Asthma, COPD und/oder chronischen Lungenemphysemen, und/oder zur prophylaktischen und/oder therapeutischen Behandlung von durch Zigarettenrauch, Feinpartikel und/oder durch andere Umweltnoxen induzierten und/oder hiermit assoziierten Atemwegserkrankungen, insbesondere mit dem Ziel, die Therapieeffekte von Leitlinientherapien, wie zuvor definiert, zu intensivieren und/oder zu verbessern, und/oder mit dem Ziel einer Induktion und/oder Verstärkung von steroidpermissiven Effekten, insbesondere um den progressiven Verlauf von Obstruktionen und/oder Emphysemen, Exazerbationen und/oder den Steroidbedarf zu reduzieren. Diesbezüglich kann auch eine Substitutionstherapie mit alpha-1-Antitrypsin bei einer Applikation des Vitamin D-Rezeptoragonisten (VDA) von etwa 250 mg bis 375 mg pro Woche in Betracht kommen.

Weiterhin eignet sich das erfindungsgemäße Kombinationstherapeutikum zur Kompensation niedriger Wirkstoffspiegel von Vitamin D und/oder niedriger Wirkstoffspiegel von Monoterpenen, insbesondere wobei das Kombinationstherapeutikum als Kit vorliegt, für die Prophylaxe, Therapie und/oder Retardierung des progressiven Verlaufs bei insbesondere schweren entzündlichen Darmerkrankungen, insbesondere chronisch-aktiven entzündlichen Darmerkrankungen, und/oder für die Reduktion von Exazerbationen, schwerer COPD, insbesondere des Lungenemphysems mit oder ohne respiratorischer Insuffizienz, schwerem Asthma, schweren Infektionserkrankungen, allergischen Reaktionen, zur Entzündungshemmung und Steroideinsparung, Osteoporose, Herzkreislauferkrankungen, Arteriosklerose, malignen Erkrankungen und Retardierung von Alterungsprozessen.

In diesem Zusammenhang eignet sich das erfindungsgemäße Kombinationstherapeutikum auch zur Erhöhung der körpereigenen Vitamin D-Synthese bzw. zu einer Erhöhung des körpereigenen Vitamin D-Spiegels, insbesondere durch orale Dosierung von dünndarmlöslichen Kapseln.

Darüber hinaus eignet sich das erfindungsgemäße Kombinationstherapeutikum auch zur Verwendung bei der prophylaktischen oder therapeutischen Behandlung von oberen Atemwegsobstruktionen sowie von profibrotischen Entzündungsreaktionen, insbesondere bei schlafbezogenen Atmungsstörungen, insbesondere des obstruktiven und zentralen Schlafapnoesyndroms. Auch diesbezüglich kommen insbesondere dünndarmlösliche Kapseln des erfindungsgemäßen Kombinationstherapeutikums in Betracht.

Gleichermaßen eignet sich das erfindungsgemäße das Kombinationstherapeutikum zur Intensivierung und/oder Verbesserung der Wirksamkeit und/oder zur Wirkeffizienzsteigerung von Leitlinientherapien von chronisch entzündlichen Darmerkrankungen, insbesondere des Reizdarmsyndroms, *Morbus Crohn* und *Colitis ulcerosa,* insbesondere mit dem Ziel der Prophylaxe, insbesondere Exazerbationsprophylaxe, Remissionserhaltung und Erhöhung der Entzündungshemmung bei Kindern und Erwachsenen, insbesondere wobei das Kombinationstherapeutikum als Kit vorliegt.

Insbesondere besteht ein Fokus des erfindungsgemäßen Kombinationstherapeutikums in der Reduktion akuter, infektiöser, allergisch oder chronisch bedingter Rezidivhäufungen mit Besserung der Therapierefraktärität durch eine kombinierte Behandlung von Lokal- und/oder Systementzündung insbesondere bei Reizdarmsyndromen, insbesondere bei entzündlichen Darmerkrankungen, wie *Inflammatory Bowel Disease.*

Gemäß einem wiederum weiteren Aspekt der vorliegenden Erfindung betrifft die vorliegende Erfindung einen Vitamin D-Rezeptoragonisten (VDA), insbesondere 25-Hydroxy-Vitamin D₃ (Calcidiol) und/oder 1,25-Dihydroxy-Vitamin D₃ (Calcitriol), vorzugsweise 25-Hydroxy-Vitamin D₃ (Calcidiol), zur Verwendung bei der prophylaktischen und/oder therapeutischen Behandlung von Entzündungen, insbesondere von allergischen, chronischen, infektiösen und/oder exazerbierten Lokal- und/oder Systemerkrankungen, insbesondere mit dem Ziel der Reduktion des Therapiebedarfs und/oder insbesondere zur Intensivierung und/oder Verbesserung der Wirksamkeit und/oder zur Wirkeffizienzsteigerung der Hauptwirkung von Leitlinientherapeutika, vorzugsweise unabhängig von einem Vitamin D-Mangelzustand.

Das Kombinationstherapeutikum nach der Erfindung eignet sich insbesondere zur Dosisreduktion des Monoterpens, insbesondere 1,8-Cineol, und/oder zur Dosisreduktion mindestens eines weiteren pharmakologischen Wirk- und/oder Inhaltsstoffs, insbesondere wie zuvor definiert, und/oder zur Dosisreduktion mindestens eines systemisch oder topisch applizierbaren pharmakologischen Wirkstoffs, insbesondere wie zuvor definiert, vorzugsweise eines Kortikosteroids. In diesem Zusammenhang können die zu verabreichenden Mengen und/oder Dosen des jeweiligen Wirkstoffs, unabhängig voneinander, insbesondere nach einem definierten Anwendungszeitraum des erfindungsgemäßen Kombinationstheraputikums, z. B. nach drei Wochen, insbesondere nach zwei Wochen, vorzugsweise nach einer Woche, um mindestens 5 %, insbesondere mindestens 10 %, vorzugsweise mindestens 20 %, bevorzugt mindestens 40 %, bezogen auf ein entsprechendes Therapeutikum ohne Vitamin D-Rezeptoragonist (VDA), verringert werden.

Gemäß einem noch weiteren Aspekt der vorliegenden Erfindung betrifft die vorliegende Erfindung auch einen Vitamin D-Rezeptoragonisten (VDA), insbesondere 25-Hydroxy-Vitamin D₃ (Calcidiol) und/oder 1,25-Dihydroxy-Vitamin D₃ (Calcitriol), vorzugsweise 25-Hydroxy-Vitamin D₃ (Calcidiol), zur Verwendung bei der prophylaktischen und/oder therapeutischen und/oder lokalen und/oder topischen und/oder inhalativen Behandlung von Erkrankungen der oberen und/oder unteren Atemwege und/oder zur Verwendung bei der prophylaktischen und/oder therapeutischen und/oder lokalen Behandlung von insbesondere chronisch entzündlicher Erkrankungen des Darms, vorzugsweise unabhängig von einem Vitamin D-Mangelzustand, insbesondere wobei der Vitamin D-Rezeptoragonist (VDA) in dünndarmlöslichen Kapseln eingebracht ist und/oder gelöst vorliegt und/oder insbesondere wobei der Vitamin D-Rezeptoragonist (VDA) in Form von Pellets vorliegt und in Pellets eingebracht ist.

Schließlich betrifft die vorliegende Erfindung - gemäß einem noch weiterführenden Aspekt der vorliegenden Erfindung - einen Vitamin D-Rezeptoragonisten (VDA), insbesondere 25-Hydroxy-Vitamin D₃ (Calcidiol) und/oder 1,25-Dihydroxy-Vitamin D₃ (Calcitriol), vorzugsweise 25-Hydroxy-Vitamin D₃ (Calcidiol), zur Verwendung bei der prophylaktischen und/oder therapeutischen und/oder lokalen und/oder topischen Behandlung von Erkältungserkrankungen, chronisch obstruktiven Atemwegserkrankungen und/oder chronisch entzündlichen Darmerkrankungen, insbesondere zur Inaktivierung oder Hemmung der Vermehrung von viralen und/oder bakteriellen Infekterregern, insbesondere mit dem Ziel, die Virulenz, Infektiosität, chronische persistierende Entzündungsreaktionen und somit den Therapiebedarf von Antibiotika, Kortikosteroiden und/oder anderen Immunsupressiva zu reduzieren und/oder zu verringern, vorzugsweise unabhängig von einem Vitamin D-Mangelzustand.

Im Rahmen der erfindungsgemäßen Konzeption kann Vitamin D, insbesondere Calcidiol und/oder Calcitriol, vorzugsweise Calcidiol, als spezielle Medikation eingesetzt werden, insbesondere auch in Form einer Co-Medikation zusammen mit einer Leitlinientherapie in verschiedenen Kombinationen, insbesondere mit dem Ziel, die eigenständige antiinflammatorische Wirkung von Vitamin D in Kombination mit verschiedenen Leitlinientherapeutika auch unabhängig von einem Vitamin D-Mangel zur Verstärkung der Steroidwirkung von Leitlinientherapeutika, wie SABA, LABA, ICS oder LAMA, einzusetzen. Insbesondere kann auf dieser Basis auch der Bedarf an Steroiden gesenkt werden bzw. kann eine Alternative zur Therapie mit Steroiden bereitgestellt werden.

Erfindungsgemäß können insbesondere folgende Behandlungskonzepte bereitgestellt werden: Vitamin D und Xylometazolin oder Oxymetazolin; Vitamin D und Xylometazolin oder Oxymetazolin und Dexpanthenol; Vitamin D und Xylometazolin oder Oxymetazolin und Cromoglicinsäure (DNCG); Vitamin D und Xylometazolin oder Oxymetazolin mit Antihistaminikum; Vitamin D und Xylometazolin oder Oxymetazolin mit topischen Steroiden und einem Antihistaminikum; Vitamin D und Xylometazolin oder Oxymetazolin mit Vitamin A und/oder E, Phenoxyethanol, Monoterpenen oder ätherischen Mischölen; Vitamin D und Xylometazolin oder Oxymetazolin mit Vagolytikum (insbesondere bei nasaler Kongestion).

Erfindungsgemäß können zudem insbesondere folgende Behandlungskonzepte bereitgestellt werden: Behandlung der unteren Atemwege durch Inhalation von Vitamin D und Cineol mit LABA, insbesondere Formoterol, oder entsprechende systemische Gabe; Vitamin D und Cineol mit ICS, insbesondere Budesonid; Vitamin D und Cineol mit LABA und ICS; Vitamin D und Cineol und LAMA; Vitamin D und Cineol mit LABA und/oder LAMA; Vitamin D und Cineol mit ICS und/oder LABA und/oder LAMA; insbesondere auch zur Verstärkung einer steroidartigen entzündungshemmenden Wirkung, mit dem Ziel, Steroide zu ersetzen, steroidale Nebenwirkungen und eine gewisse krankheitsassoziierte Steroidresistenz zu überwinden.

Erfindungsgemäß können zudem insbesondere folgende Behandlungskonzepte bereitgestellt werden: Systemische Behandlung der oberen und unteren Atemwege mit Vitamin D, Cineol und Montelukast oder einem anderen LeukotrienRezeptorantagonisten oder -Inhibitor; Vitamin D und Cineol mit Prednsiolon; Vitamin D und Cineol mit anti-Immunglobulin E (z. B. Omalizumab); Vitamin D und Cineol mit Immunsuppressiva; Vitamin D und Cineol mit Antirheumatika, insbesondere zur Verstärkung einer steroidartigen entzündungshemmenden Wirkung, mit dem Ziel, Steroide zu ersetzen, steroidale Nebenwirkungen und eine etwaige krankheitsassoziierte Steroidresistenz zu überwinden.

Erfindungsgemäß können zudem insbesondere weitere Behandlungskonzepte bereitgestellt werden: Behandlung von oberen und unteren Atemwegserkrankungen zur Intensivierung der Wirkung von Monterpenen (1,8-Cineol, L-Menthol) oder von ätherischen Mischölen (Eukalyptusöl, Pfefferminzöl), welche ein Monoterpen enthalten, oder auch allen anderen Formen von standardisierten Mischölen, welche ein Monoterpen enthalten.

Erfindungsgemäß können zudem insbesondere noch folgende Behandlungskonzepte bereitgestellt werden: Erzielung einer antibiotischen und entzündungshemmenden Wirkung durch die Kombination von Vitamin D und Cineol mit Antibiotika.

Erfindungsgemäß können zudem insbesondere auch folgende Behandlungskonzepte bereitgestellt werden: Intensivierung der antientzündlichen Leitlinientherapie bei entzündlichen Darmerkrankungen auf Basis einer Kombination von Vitamin D und Cineol; Vitamin D und Cineol mit Budesonid; Vitamin D und Cineol mit Pfefferminzöl; Vitamin D und Cineol mit weiteren Monoterpenen, z. B. L-Menthol.

Erfindungsgemäß können zudem insbesondere auch folgende Behandlungskonzepte bereitgestellt werden: Kombinationen mit Vitamin D und Cineol zur synergistischen Intensivierung der Prophylaxe und des Therapieeffekts der Leitlinienentherapie bei arterieller Hypertonie und koronarer Herzerkrankung (Diuretika, ACE-Hemmer, ACE-Rezeptorantagonisten, mit oder ohne Kombination mit Diuretika, Ca⁺⁺-Antagonisten und beta-2-Rezeptorblockern), Arterioskleorse, zur Vermeidung von Restentstenosen bei Angina pectoris und Prophylaxe kardialer Ischämien, Rhytmusstörungen, akuten Myokardinfarkten, Zigarettenrauch und Diabetesassoziierten Gefäßerkrankungen oder Komplikationen (z. B. Vitamin D und Statine und anderen Fettsenkern, Plättchenaggregationshemmern, wie Acetylsalicylsäure, Clopidogrel) sowie zur Verbesserung der diabetogenen Stoffwechsellage durch Ersatz oder Reduktion des Bedarfs von Antidiabetika (Insulin, Insulinanaloga sowie oralen Antidiabetika).

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne sie jedoch hierauf zu beschränken.

### Ausführungsbeispiele und weitere Ausführungen zur vorliegenden Erfindung:

### Methodik

Im Rahmen der vorliegenden Erfindung wurde der Einfluss der entzündungshemmenden Wirkung von Calcidiol und Calcitriol sowie gegebenenfalls verschiedenen weiteren Testsubstanzen in Kulturen von normalen humanen Monozyten *in vitro* untersucht. Als Maß zur Bestimmung der entzündungshemmenden Wirkung wurde die Stärke der Hemmung der Produktion des Tumornekrosefaktor-alpha (TNF-alpha), repräsentativ für die mit entzündlichen Prozessen assoziierte Zytokinproduktion, in humanen Monozyten bestimmt. Die Produktion von TNF-alpha kann in humanen Monozyten gezielt durch die Zugabe von Lipopolysacchariden (LPS) stimuliert werden. Je stärker die entzündungshemmende Wirkung einer Testsubstanz ist, desto weniger TNF-alpha wird durch die mit LPS stimulierten Monozyten produziert. Im Rahmen der erfindungsgemäßen Versuche wurde neben Calcidiol und Calcitriol als weitere Testsubstanz das Monoterpen 1,8-Cineol eingesetzt.

Im Rahmen der Untersuchungen wurden zunächst humane Monozyten durch standardisierte und dem Fachmann grundsätzlich bekannte Methoden (Dichtegradientenzentrifugation oder magnetische Zellseparation unter Einsatz von *MicroBeads*^{®}) aus venösem Blut von Spendern isoliert. Anschließend wurden die gereinigten und hochvitalen Monozyten (95 %) in 48-Loch Kulturplatten (10⁵ /ml) in Gegenwart der Testsubstanzen inkubiert. Als Kontrollen bzw. Vergleichsversuche wurden Inkubationen der Monozyten in Gegenwart von Calcidiol bzw. Calcitriol sowie der weiteren Testsubstanzen jeweils alleine durchgeführt. Die Inkubation der Monozyten mit Calcidiol bzw. Calcitriol diente darüber hinaus dem generellen Nachweis von deren entzündungshemmender Wirkung. Darüber hinaus wurden Co-Inkubationen von Calcitriol bzw. Calcidiol in Kombination mit weiteren Testsubstanz durchgeführt.

Während der Inkubation mit den Testsubstanzen erfolgte die Stimulation der Produktion von TNF-alpha (Tumornekrosefaktor-alpha) über einen Zeitraum von 20 Stunden mit Lipopolysacchariden (10 µg/ml LPS). Anschließend wurden die Überstände gewonnen und bis zur weiteren Analyse bei -80 °C gelagert. Zu Analysezwecken wurden die jeweiligen Kulturüberstände einem ELISA (*Enzyme-Linked Immunosorbent Assay*) unterzogen, dessen Durchführung dem Fachmann an sich bekannt ist, wobei die durch die Monozyten produzierte Menge an TNF-alpha gemessen wurde.

Bei den nachfolgend detailliert dargestellten Ergebnissen bzw. Untersuchungen sind die prozentualen Hemmwirkungen von Calcidiol bzw. Calcitriol, gegebenenfalls mit weiteren Testsubstanzen, auf die Produktion von TNF-alpha durch humane, mit LPS stimulierte Monozyten, im Vergleich zur Produktion von TNF-alpha durch Monozyten, welche durch LPS stimuliert, allerdings nicht in Gegenwart von Testsubstanzen inkubiert wurden, angegeben. Die Ergebnisse sind dargestellt als Hemmung in Prozent der LPS-induzierten Kontrolle ohne Wirkstoff aus gepoolten Messungen von mindestens zwei bis vier Experimenten (n = 8 bis 12). Für statistische Analysen wurde der nichtparametrische Mann & Whitney Test herangezogen, insbesondere vor dem Hintergrund der Ermittlung einer Signifikanz im Vergleich zur LPS-Kontrolle, dem alleinigen Einsatz von Vitamin D₃ bzw. von Calcidiol bzw. Calcitriol sowie dem alleinigen Einsatz der Testsubstanz. Ab einem p-Wert von < 0,05 gegenüber der jeweiligen Kontrolle bzw. dem jeweiligen Vergleichsansatz wird im Rahmen der vorliegenden Erfindung die Hemmwirkung als signifikant angesehen.

### a) Calcidiol bzw. Calcitriol als Entzündungshemmer

Zum Nachweis der entzündungshemmenden Wirkung von Calcidiol bzw. Calcitriol wurde der Einfluss auf die LPS-stimulierte Produktion von TNF-alpha durch humane Monozyten untersucht. Dazu wurde Calcidiol bzw. Calcitriol in jeweils separaten Experimentreihen in Konzentrationen von jeweils 0,1 ng/ml, 1 ng/ml, 10 ng/ml, 30 ng/ml, 50 ng/ml und 100 ng/ml eingesetzt. Die in Prozent angegebene Hemmung der TNF-alpha Produktion durch Calcitriol bzw. Calcidiol bezieht sich dabei auf die Menge an TNF-alpha, welche durch mit LPS stimulierte Monozyten ohne den Einsatz von Hemmstoffen produziert wurde.

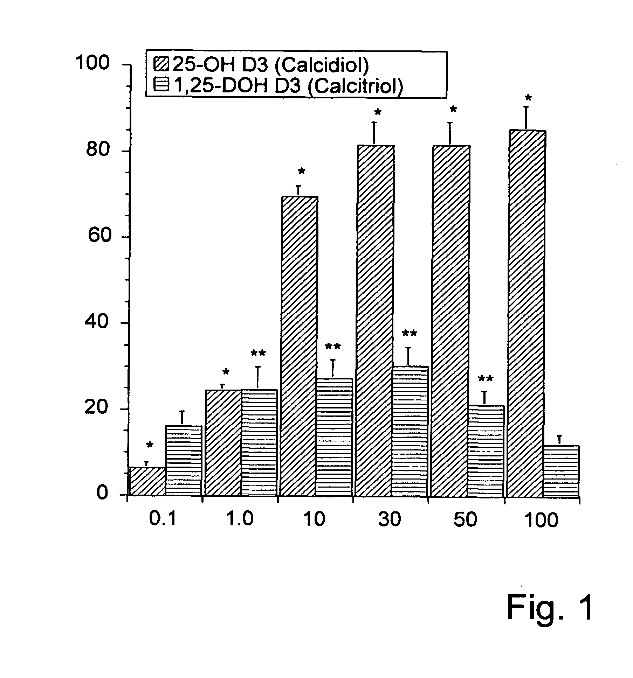

Die Ergebnisse zeigen überraschenderweise sowohl für Calcidiol als auch Calcitriol für Calcidiol eine signifikante starke Hemmung der Produktion von TNF-alpha, wobei die durch Calcidiol induzierte Hemmung diejenige von Calcitriol übersteigt. Die durch Calcidiol induzierte Hemmung von TNF-alpha variierte im Bereich von - 6,5 ± 1 % bei 0,1 ng/ml bis - 85,7 ± 5 % bei 100 ng/ml bei einem p-Wert von ≤ 0,0117. Somit kann die Hemmung der Produktion von TNF-alpha als signifikant angesehen werden. Mit Calcitriol konnte ebenfalls eine Hemmung von TNF-alpha erzielt werden, wobei diese mit Werten im Bereich von - 24,8 ± 5 % für 1 ng/ml und - 21,4 ± 3 % für 50 ng/ml mit einem Maximalwert der Hemmung von - 30,5 ± 4 % bei Einsatz von 30 ng/ml Calcitriol geringer ausfiel als die durch Calcidiol induzierte Hemmung. Die entsprechenden Ergebnisse sind Fig. 1 dargestellt, wobei die x-Achse die Konzentration an eingesetztem Calcidiol bzw. Calcitriol und die y-Achse den jeweiligen Mittelwert mit Angabe der Standardabweichung der prozentualen Hemmung im Vergleich zu der TNF-alpha-Kontrolle darstellt (mit *p = 0,0008 und **p < 0,017). Fig. 1 zeigt somit, dass im therapeutischen Konzentrationsbereich von Calcidiol (n = 8, 2 Experimente) und von Calcitriol (n = 8, 4 Experimente) die LPS-stimulierte Produktion von TNF-alpha in normalen Monozyten im Vergleich zur LPS-Kontrolle signifikant gehemmt wird.

Insgesamt konnte gezeigt werden, dass Vitamin D in seiner aktiven Form, insbesondere in Form von Calcidiol, aber auch in Form von Calcitriol, über eine eigenständige entzündungshemmende Wirkung verfügt. Im Gegensatz zu Calcidiol fällt die durch Calcitriol induzierte Hemmung von TNF-alpha bei normalen bzw. plasmarelevanten Konzentrationen, insbesondere im Bereich von 20 bis 55 ng/ml, wieder leicht ab. Steigende Calcitriol-Konzentrationen sind also insgesamt mit einem gewissen Rückgang der entzündungshemmenden Wirkung verbunden. Ohne sich hierbei auf diese Theorie beschränken zu wollen, spricht die abnehmende Entzündungshemmung bei höheren Konzentrationen von Calcitriol für eine Downregulation bzw. Herunterregulation des Vitamin D-Rezeptors durch höhere Konzentrationen an Calcitriol, so dass Calcitriol gewissermaßen imstande ist, seinen eigenen Rezeptor herunterzuregulieren.

Im Unterschied hierzu ist die Expression des Vitamin D-Rezeptors bei Vitamin D-Mangelsituationen induzierbar und wird durch eine Supplementierung oder Therapie mit Calcidiol nicht negativ beeinflusst.

Die obigen Ergebnisse erlauben die Schlussfolgerung, dass Vitamin D, insbesondere in seiner aktiven Form, für einen Einsatz im Rahmen der Behandlung von nicht-steroidpflichtigen, allergischen und entzündlichen Erkrankungen oder zur Steroidreduktion geeignet ist, wobei aufgrund der höheren antientzündlichen Wirkung Calcidiol bevorzugt wird. Calcidiol ist nämlich auch in höheren Konzentrationen in gesunden humanen Monozyten wirksam, was bislang im Stand der Technik bislang so nicht bekannt war.

### b) Vitamin D zur Intensivierung der Wirkung von Monoterpenen und ätherischen Mischölen

Hintergrund: Virale und bakterielle Infektionen der Atemwege sind häufig mit einem Vitamin D-Mangel assoziiert beschrieben worden, ohne dass es bislang gelungen ist, durch eine Therapie auf Basis von Vitamin D die Infektrate zu reduzieren.

Isolierte Monoterpene, wie z. B. 1,8-Cineol, und ätherische Mischöle werden im Rahmen der Behandlung von Infekten bzw. Erkrankungen der Atemwege üblicherweise primär zur Sekretolyse eingesetzt. Darüber hinaus sind sie aber auch für ihr entzündungshemmendes Wirkprofil bekannt, welches eine Reduktion der entzündlich bedingten Schleimproduktion bewirkt. Die Reduktion der entzündlich bedingten Schleimproduktion durch Monoterpene, insbesondere 1,8-Cineol, ist dabei insbesondere auf dessen entzündungshemmende Effekte und weniger auf die sekretolytischen bzw. mukolytischen Effekte zurückzuführen.

Erfindungsgemäßer Ansatz: Im Winter treten gehäuft respiratorische Infekte auf, was insbesondere auf eine erhöhte Aggressivität und Vermehrung von viralen und bakteriellen Infekterregern zurückzuführen ist. Gleichzeitig ist während der Wintermonate aufgrund der reduzierten körpereigenen lichtinduzierten Synthese von Calcitriol ausgehend von 7-Dehydrocholesterol der Vitamin D-Spiegel im Körper gesenkt. Diese Tatsache, insbesondere in Kombination mit der hohen Infektrate im Winter, lässt den Rückschluss zu, dass Vitamin D bzw. dessen Vorläufersubstanzen, wie beispielsweise Calcidiol, das Wachstum und die Aggressivität von Infekterregern, insbesondere in den oberen Atemwegen, kontrollieren.

Unter Therapie mit Monoterpenen und ätherischen Mischölen werden weniger Atemwegsinfektionen und ein schnelleres Abklingen von Infekten beobachtet, die mitunter mit einer Interaktion von Terpenen mit dem Vitamin D-Rezeptor oder dem lokalen Schleimhautspiegel von Vitamin D korrelieren.

Überraschenderweise wurde im Rahmen der vorliegenden Erfindung gefunden, dass es möglich ist, die entzündungshemmende Wirkung von Monoterpenen, insbesondere von 1,8-Cineol, durch den zusätzlichen Einsatz von Vitamin D bzw. dessen Analoga bzw. durch Calcidiol und/oder Calcitriol zu intensivieren. Gleichermaßen ist es auch möglich, durch den kombinierten Einsatz von Monoterpenen bzw. ätherischen Mischölen die entzündungshemmenden Effekte von Vitamin D bzw. dessen Analoga bzw. Calcidiol und/oder Calcitriol zu intensivieren.

Insbesondere ist es in völlig überraschender Weise möglich, durch eine Co-Medikation beispielsweise in dünndarmlöslichen Kapseln mit (i) Vitamin D bzw. Calcidiol und/oder Calcitriol einerseits sowie Monoterpenen andererseits oder mit (ii) Vitamin D bzw. Calcidiol und/oder Calcitriol einerseits und ätherischem Mischöl andererseits die entzündungshemmende bzw. sekretolytische Wirkung von Monoterpenen, wie z. B. 1,8-Cineol, oder von Mischterpenen zu verbessern, was insgesamt in einem verbesserten Schutz vor Atemwegsinfektionen und -entzündungen resultiert.

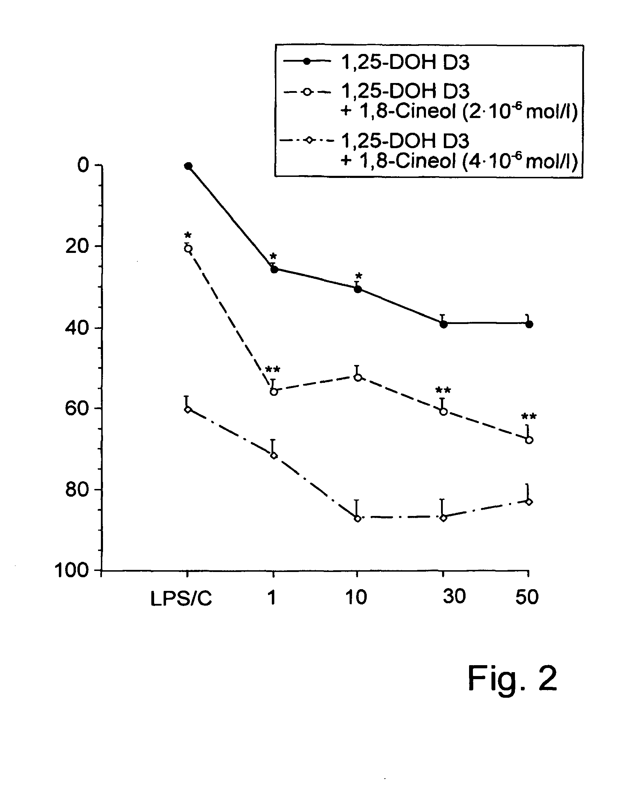

Dies wurde im Rahmen der vorliegenden Erfindung durch eine Co-Inkubation einer geringen (2 x 10⁻⁶ mol/l) und einer mittleren (4 x 10⁻⁶ mol/l) Konzentration von 1,8-Cineol und Calcitriol in aufsteigenden, plasmarelevanten Konzentrationen an humanen Monozyten nachgewiesen. In diesem Zusammenhang konnte in den humanen Monozyten für geringere Konzentrationen von 1,8-Cineol eine synergistische Zunahme der entzündungshemmenden Wirkung von ca. 300 %, bezogen auf die LPS-stimulierte Produktion von TNF-alpha, und für mittlere Konzentrationen eine synergistische Zunahme der entzündungshemmenden Wirkung von ca. 50 %, bezogen auf die LPS-stimulierte Produktion von TNF-alpha, nachgewiesen werden (Fig. 2).

Fig. 2 zeigt demnach, dass die LPS-stimulierte Produktion von TNF-alpha signifikant durch ansteigende Konzentrationen von Calcitriol gehemmt wird (durchgezogene Linie). Eine Co-Inkubation von Calcitriol mit einer niedrigeren Plasmakonzentration von 1,8-Cineol (C) (2 x 10⁻⁶ mol/l, gestrichelte Linie) führt zu einer signifikanten Zunahme der TNF- alpha-Hemmung im Vergleich zur Kontrolle (LPS+C), Vitamin D₃ bzw. Calcitriol oder Cineol alleine. Diese Hemmeffekte nehmen nach Co-Inkubation von Vitamin D₃ bzw. Calcitriol mit einer mittleren Konzentration von 1,8-Cineol zu (4 x 10⁻⁶ mol/l, strichpunktierte Linie).

In Fig. 2 zeigt die x-Achse die Konzentration an eingesetztem Calcitriol in ng/ml, und die y-Achse zeigt den jeweiligen Mittelwert mit Angabe der Standardabweichung der prozentualen Hemmung im Vergleich zu der TNF-alpha-Kontrolle (mit p < 0,004 für Vitamin D₃ bzw. Calcitriol, Cineol, Vitamin D₃ bzw. Calcitriol plus Cineol vs. Kontrolle; *p < 0.08 vs. LPS-Kontrolle; **p < 0,007 für Cineol 2 x 10⁻⁶ mol/l plus Vitamin D₃ bzw. Calcitriol vs. Vitamin D₃ bzw. Calcitriol alleine; **p < 0,007 für Cineol 4 x 10⁻⁶ mol/l plus Vitamin D₃ bzw. Calcitriol vs. Vitamin D₃ bzw. Calcitriol alleine).

Aus Fig. 2 geht im Ergebnis deutlich hervor, dass die LPS-stimulierte Produktion von TNF-alpha durch humane Monozyten im Rahmen einer Co-Inkubation von Calcitriol mit 1,8-Cineol im Vergleich zur alleinigen Inkubation mit Calcitriol deutlich stärker - und zwar synergistisch - gehemmt werden kann. Zudem hat sich gezeigt, dass eine Steigerung der (Plasma-)Konzentration von 1,8-Cineol zu einer weiteren Zunahme des Hemmeffekts führt. Die Co-Inkubation von Calcitriol und 1,8-Cineol mit einer mittleren Plasmakonzentration von 4 x 10⁻⁶ mol/l führt zu einer noch stärkeren Hemmung der Produktion von TNF-alpha in den humanen Monozyten im Vergleich zu der Co-Inkubation von Calcitriol und 1,8-Cineol mit einer niedrigen Plasmakonzentration von 2 x 10⁻⁶ mol/l.

Aus der beschriebenen antientzündlichen Interaktion von Vitamin D mit 1,8-Cineol resultiert insgesamt auch eine verstärkte antiinfektiöse Wirkung, wobei die Wirkeigenschaften von Monoterpenen und ätherischen Mischölen deutlich, und zwar synergistisch, gesteigert werden, ohne dass die Wirkstoffmenge des Monoterpens oder ätherischen Mischöls erhöht wird.

Im Rahmen der vorliegenden Erfindung hat es sich somit als besonders vorteilhaft erwiesen, zur Erzielung einer synergistisch gesteigerten Entzündungshemmung Vitamin D in Kombination mit Monoterpenen, insbesondere 1,8-Cineol, oder ätherischen Mischölen einzusetzen. Insbesondere aufgrund der starken Entzündungshemmung und der daraus resultierenden antiinfektiösen Wirkweise ist der kombinierte Einsatz von Vitamin D einerseits und Monoterpenen andererseits auch unabhängig von Vitamin D-Mangelzuständen, insbesondere in der Winterzeit, empfehlenswert.

Hieraus folgt, dass eine Vitamin D-Supplementierung oder -Therapie durch Zusatz insbesondere von 1,8-Cineol oder einem ätherischen Mischöl erfolgen sollte bzw. *vice versa,* da sich die jeweiligen Wirkstoffe hinsichtlich ihrer Therapie- bzw. Wirkeffizienz in synergistischer Weise ergänzen. Vorzugsweise wird Vitamin D direkt als Calcitriol oder in Form seiner Vorläufersubstanz Calcidiol gemeinsam mit 1,8-Cineol oder einem ätherischen Mischöl mit einem Monoterpen eingesetzt.

### c) Vitamin D zur Intensivierung der Wirkung von Kortikosteroiden

Insbesondere wurde im Rahmen der vorliegenden Erfindung die direkte entzündungshemmende Wirkung von Vitamin D₃ an isolierten Monozyten untersucht, welche den Vitamin D-Rezeptor exprimieren (Fig. 1).

Anschließend wurde die entzündungshemmende Wirkung einer Kombination auf Basis von Vitamin D, insbesondere Calcitriol, und Budesonid untersucht. Dazu wurde der hemmende Effekt von Calcitriol und Budesonid sowohl alleine als auch in Kombination auf die LPS-stimulierte Produktion von TNF-alpha in humanen Monozyten bestimmt. Die diesbezüglich erhaltenen Ergebnisse sind der nachfolgenden Tabelle 1 zu entnehmen.

**Tabelle 1: Effekt von Budesonid und Calcitriol auf die LPS-stimulierte Produktion von TNF-alpha in humanen Monozyten**

| **Konz.** | **n** | **TNF-alpha (pg/10⁵ Monozyten)** | **Vergleich zur Kontrolle (%)** | **p-Wert** |
|---|---|---|---|---|
| ohne Stimulation | 8 | 756,1 ± 192 | - | - |
| 10 µg/ml LPS | 8 | 889,1 ± 52 | - | - |

| **Calcitriol alleine** | | | | |
|---|---|---|---|---|
| 1 ng/ml | 8 | 712 ± 31 | -20,0 | 0,0117 |
| 10 ng/ml | 8 | 687,5 ± 33 | -22,7 | 0,0008 |
| 30 ng/ml | 8 | 588,8 ± 14 | -33,8 | 0,0008 |
| 50 ng/ml | 8 | 605,6 ± 21 | -31,9 | 0,0008 |

| **Budesonid alleine** | | | | |
|---|---|---|---|---|
| 10⁻¹⁰ mol | 8 | 606,4 ± 110 | -31,8 | 0,0460 |
| 10⁻⁹ mol | 8 | 412,2 ± 22 | -53,6 | 0,0008 |

| **Budesonid in Kombination mit Calcitriol** | | | | |
|---|---|---|---|---|
| Bud. 10⁻¹⁰ mol und Vit. D 1 ng/ml | 8 | 439,4 ± 53 | -50,6 | 0,0008 |
| Bud. 10⁻¹⁰ mol und Vit. D 10 ng/ml | 8 | 430,0 ± 73 | -51,6 | 0,0008 |
| Bud. 10⁻¹⁰ mol und Vit. D 30 ng/ml | 8 | 354,4 ± 57 | -60,1 | 0,0008 |
| Bud. 10⁻¹⁰ mol und Vit. D 50 ng/ml | 8 | 333,4 ± 54 | -62,5 | 0,0008 |
| Bud. 10⁻⁹ mol und Vit. D 1 ng/ml | 8 | 303,7 ± 12 | -65,5 | 0,0008 |
| Bud. 10⁻⁹ mol und Vit. D 10 ng/ml | 8 | 266,8 ± 22 | -70,0 | 0,0008 |
| Bud. 10⁻⁹ mol und Vit. D 30 ng/ml | 8 | 223.1 ± 18 | -73,8 | 0,0008 |
| Bud. 10⁻⁹ mol und Vit. D 50 ng/ml | 8 | 218,6 ± 27 | -75,5 | 0,0008 |

Die in Tabelle 1 aufgeführten Ergebnisse zeigen, dass sich überraschenderweise auf Basis einer Kombination von Budesonid und Calcitriol eine deutlich stärkere Hemmung der Produktion von TNF-alpha in humanen Monozyten erzielen lässt als mit Calcitriol bzw. Budesonid jeweils alleine, und zwar über die Wirkung der Einzelsubstanzen hinausgehend. Somit wurde im Rahmen der vorliegenden Erfindung eine synergistische Wirkung zwischen dem inhalativen Kortikosteroid Budesonid und Calcitriol gefunden, d. h. eine Zunahme der entzündungshemmenden Wirkung von Budesonid in Gegenwart von relevanten Konzentrationen von Calcitriol.

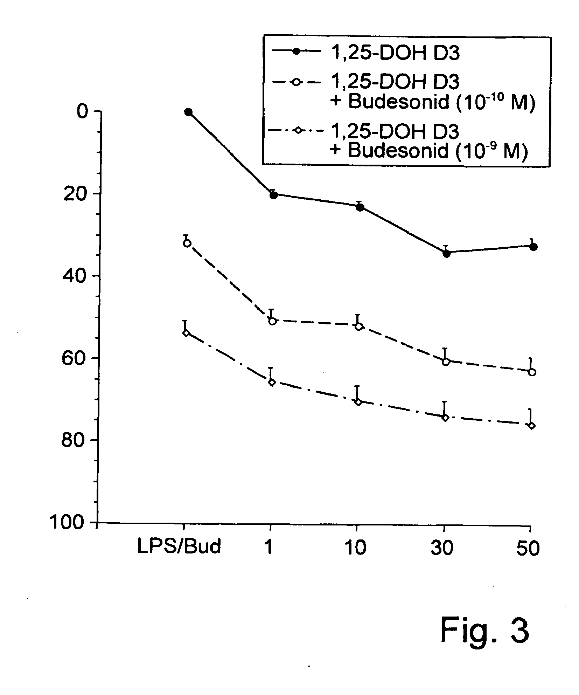

Fig. 3 zeigt, dass die LPS-stimulierte TNF-alpha-Produktion durch ansteigende Konzentrationen von Calcitriol signifikant gehemmt wird (durchgezogene Linie). Co-Inkubation von Calcitriol mit einer niedrigeren (Atemwegs-)Konzentration von Budesonid (10⁻¹⁰ mol/l, gestrichelte Linie) führt zu einer signifikanten Zunahme der TNF-alpha-Hemmung im Vergleich zur Kontrolle (LPS + Budesonid, Calcitriol oder Budesonid alleine). Diese Hemmeffekte nehmen weiter synergistisch zu nach Co-Inkubation von Calcitriol bei einer höheren Konzentration von Budesonid (10⁻⁹ mol/l, strichpunktierte Linie). In Fig. 3 zeigt die x-Achse die Konzentration an eingesetztem Calcitriol in ng/ml, und die y-Achse zeigt den jeweiligen Mittelwert mit Angabe der Standardabweichung der prozentualen Hemmung im Vergleich zu der TNF-alpha-Kontrolle (mit p < 0,045 für Calcitriol, Budesonid, Budesonid + Calcitriol vs. LPS-Kontrolle; p < 0.016 für Budesonid 10⁻¹⁰ mol/l + Calcitriol vs. Calcitriol alleine; p = 0.0008 für Budesonid 10⁻⁹ mol/l + Calcitriol vs. Calcitriol alleine; p < 0.0275 für Budesonid 10⁻¹⁰ mol/l + Calcitriol vs. Budesonid 10⁻¹⁰ mol/l alleine; p < 0.0035 für Budesonid 10⁻⁹ mol/l + Calcitriol vs. Budesonid alleine).

### d) Anwendungs- und Wirksamkeitsstudien

Im Rahmen eines ersten Untersuchungskomplexes erhielten acht Patienten im Alter von 38 bis 62 Jahren mit persistierendem Asthma bronchiale (GINA II), welche mit einer Kombinationstherapie aus inhalativem Glukokortikoid, (Beclometason, 2 x 200 µg/die inhalativ) und inhalativem langwirksamen beta-2-Sympathomimetika (LABA, Salmeterol) sowie Theophyllin peroral behandelt wurden, eine Woche lang 1,8-Cineol 4 x 200 mg/die peroral. Nach einwöchiger Therapie konnte bei 4 der 8 Probanden eine leichte Verbesserung der Lungenfunktion erreicht werden. Nach fortgesetzter zwölfwöchiger Dauertherapie hatte sich das persistierende Bronchialasthma weiter stabilisiert, so dass bei 5 der 8 Patienten der inhalative Glukokortikoidbedarf um 40 % reduziert werden konnte. Bei 3 der behandelten Patienten konnte auch der Betamimetikabedarf um 10 % reduziert werden. Eine weitere Gruppe auf Basis von 10 Patienten im Alter von 42 bis 71 Jahren mit identischer Indikation, d. h. persistierendem Asthma bonchiale (GINA II), welche gleichermaßen die oben angeführte Kombinationstherapie auf Basis des inhalativen Glukokortikoids und der inhalativen langwirksamen beta-2-Sympathomimetika sowie Theophyllin verabreicht bekamen, erhielten eine Woche lang eine Kombination von 1,8-Cineol 4 x 200 mg/die und Vitamin D in einer Tagesdosis von 80 µg. Nach einwöchiger Therapie mit der vorgenannten Dosis konnte bei 7 der 8 Probanden eine leicht- bis mittelgradige Verbesserung der Lungenfunktion erreicht werden. Nach fortgesetzter zwölfwöchiger Dauertherapie hatte sich das persistierende Bonchialasthma insofern stabilisiert, als bei 6 der 8 Patienten der inhalative Glukokortikoidbedarf um bis zu 70 % reduziert werden konnte und bei einem der 8 Patienten die inhalativen Glukokortikoide zeitweise sogar ganz abgesetzt werden konnten. Die Therapie wurde ohne Nebenwirkungen gut vertragen. Bei 6 der behandelten Patienten konnte auch der Betamimetikabedarf um bis zu 60 % reduziert werden.

### e) Weitere Anwendungs- und Wirksamkeitsstudien:

Die zuvor beschriebene, 1,8-Cineol und Vitamin D als Wirkstoffe enthaltende Darreichungsform nach der vorliegenden Erfindung wurde gleichermaßen im Rahmen einer Wirksamkeitsuntersuchung bei der Behandlung von *Morbus Crohn* und *Colitis ulcerosa* in Co-Medikation zu Kortikosteroiden eingesetzt.

Als Vergleich dienen wiederum Monopräparate auf Basis von 1,8-Cineol.

Im Rahmen der klinischen Anwendungsbeobachtungen wurden jeweils 10 Probanden (zum einen im Alter von 25 bis 52 Jahren und zum anderen im Alter von 22 bis 61 Jahren) einerseits mit dem Monopräparat auf Basis von 1,8-Cineol mit dreimaliger Gabe von 200 mg/die und andererseits mit der erfindungsgemäßen Darreichungsform, wie zuvor bei der Behandlung von Asthma bronchiale definiert, behandelt. Aufgrund der entzündlichen Komponenten der zugrundeliegenden Erkrankungen lassen sich im Rahmen der klinischen Anwendung in Analogie zur Therapie von Asthma bronchiale auch *Morbus Crohn* bzw. *Colitis ulcerosa* mit dem gleichen Therapieansatz behandeln.

Auch hier kann mit der erfindungsgemäßen Darreichungsform auf Basis der Kombination von 1,8-Cineol und Vitamin D ein deutlich verbesserter klinischer Effekt hervorgerufen werden, welcher sich dadurch manifestiert, dass die Stuhlfrequenz im Vergleich zu der Gruppe mit dem Monopräparat, bei welcher die Frequenz um etwa 40 % nachgelassen hat, nochmals deutlich auf eine Gesamtreduktion von über 60 % verbessert werden konnte. Ebenfalls kann auch im Vergleich zu der Behandlung mit dem Monopräparat die systemische Kortikosteroidgabe nochmals deutlich reduziert werden.

Die vorliegenden Wirksamkeitsstudien belegen insgesamt die hervorragende und synergistische Wirkung der erfindungsgemäß angeführten Kombination eines Vitamin D-Rezeptoragonisten (VDA) einerseits und eines Monoterpens andererseits.

### Zusammenfassung:

Die im Rahmen der vorliegenden Erfindung durchgeführten Untersuchungen zeigen, dass die Stimulation des Vitamin D-Rezeptors mit den Agonisten Calcitriol und Calcidiol eine eigenständige, antientzündliche Wirkung auch an der unteren Vitamin D-Normgrenze von 30 ng/ml besitzt. Insbesondere vermitteln Calcitriol bzw. Calcidiol im Vergleich zur Kontrolle, d. h. von lediglich mit LPS-stimulierten huma-nen Monozyten ohne Inkubation mit Calcitriol bzw. Calcidiol, eine Hemmung der LPS-stimulierten Produktion von TNF-alpha von 30 bzw. 82 %.

Infolge von Interaktionen von Vitamin D bzw. dessen Metaboliten mit verschiedenen, überwiegend entzündungshemmenden Leitlinientherapeutika, wird im Rahmen der vorliegenden Erfindung die Entwicklung neuer entzündungshemmender Kombinationen, bevorzugt auf Basis von Calcidiol mit mindestens einem Leilinientherapeutikum, ermöglicht. Insbesondere sind diese neuartigen Kombinationen zur Intensivierung der Leitlinientherapie geeignet. Dieser Effekt ist insbesondere auf die überraschend festgestellte, synergistisch zunehmende Entzündungshemmung durch den zusätzlichen Einsatz von Vitamin D-Metaboliten zurückzuführen. Insgesamt erlauben die erfindungsgemäßen Therapiekonzepte die Behandlung unterschiedlicher, mit oder ohne Vitamin D-Mangel assoziierter chronisch-entzündlicher Darmerkrankungen und Atemwegserkrankungen.

Die vorliegende Erfindung wird durch die folgenden Aspekte näher beschrieben:

### Aspekt 1:

Vitamin D-Rezeptoragonisten (VDA), insbesondere 25-OH D₃ (Calcidiol) oder Analoga, zur Prophylaxe und Therapie der Entzündungshemmung bei allergischen, chronischen, infektiösen, exazerbierten Lokal- und Systemerkrankungen, mit dem Ziel der Reduktion des Therapiebedarfs bzw. Intensivierung der Hauptwirkung von anerkannten Leitlinientherapeutika, insbesondere unabhängig von einem Vit. D-Mangelzustand.

### Aspekt 2:

Vitamin D-Rezeptoragonisten (VDA), insbesondere 25-OH D₃ (Calcidiol) oder Analoga, bevorzugt zur prophylaktischen, lokalen, topischen und inhalativen Therapie von oberen und unteren Atemwegserkrankungen sowie zur lokalen Therapie chronisch entzündlicher Darmerkrankungen, insbesondere gelöst in dünndarmlöslichen Kapseln oder in Form von Pellets, insbesondere unabhängig von einem Vit. D-Mangelzustand.

### Aspekt 3:

Vitamin D-Rezeptoragonisten (VDA), insbesondere 25-OH D₃ (Calcidiol), 1,25-DOH D₃ (Calcitriol) oder deren Analoga, bevorzugt in lokaler, topischer Form bei Erkältungskrankheiten, chronisch obstruktiven Atemwegserkrankungen und/oder chronisch entzündlichen Darmerkrankungen zur Inaktivierung oder Hemmung der Vermehrung von viralen und bakteriellen Infekterregern, insbesondere mit dem Ziel, die Virulenz, Infektiosität, chronische persistierende Entzündungsreaktionen und somit den Therapiebedarf von Antibiotika, Kortikosteroiden und anderen Immunsupressiva, unabhängig von Vit. D-Mangelzuständen, zu reduzieren.

### Aspekt 4:

25-OH D₃ (Calcidiol) in Kombination mit 1,25-DOH D₃ (Calcitriol) zur Intensivierung der Wirkungen nach Aspekt 1 bis 3 durch eine Zunahme der Entzündungshemmung und einer Wirkverstärkung durch Reduktion der 1,25-DOH D₃ vermittelten Downregulation des Vitamin D₃-Rezeptors.

### Aspekt 5:

Vitamin D₃ wird eingesetzt als Tablette / dünndarmlösliche Kapsel (2 mal täglich 800 IE bis 2.000 IE), zur topisch-nasalen Therapie (50 bis 100 µg/Hub, 1-2 mal täglich) oder zur Inhalation in Suspension- oder Pulverform (250 µg/Hub bzw. 250 bis 500 µg/Pulver).

### Aspekt 6:

Verwendung von 25-OH D₃ und 1,25-DOH D₃ oder deren Analoga als Supplement in Verbindung mit 1,8-Cineol zur gegenseitigen Wirkverstärkung für die Prophylaxe und Behandlung saisonaler, d.h. typischer winterlicher Vitamin D₃-Mangelzustände, insbesondere zur Besserung der Infektabwehr und zur verbesserten entzündungshemmenden Therapie der Atemwege, mit dem Ziel, akute und chronische Exazerbationen bei chronisch obstruktiver Bronchitis, Asthma und chronisch entzündlichen und allergischen Erkrankungen, insbesondere auch bei chronischer Rhinosinusitis und allergischer Rhinitis, der oberen Atemwege zu reduzieren.

### Aspekt 7:

Verwendung von 25-OH D₃ und 1,25-DOH D₃ oder Analoga zur synergistischen Intensivierung der antiinfektiösen, antiallergischen, entzündungshemmenden und antioxidativen Wirkung von Monoterpenen, insbesondere von 1,8-Cineol (C) und L-Menthol in peroraler Darreichungsform, vorzugsweise in Form von dünndarmlöslichen Kapseln (z. B. VD₃ 400 bis 600 IE + C 200 bis 300 mg).

### Aspekt 8:

Verwendung von 25-OH D₃ und 1,25-DOH D₃ oder deren Analoga zur lokalen-nasalen, inhalativen oder systemischen Therapie zur Prophylaxe und Therapie von viralen und bakteriellen Erkältungskrankheiten, insbesondere auch in Kombination mit 1,8-Cineol, Vitamin C, Vitamin E und Acetylsalicylsäure oder einem anderen nicht-steroidalen Antiphlogistikum (aus der Gruppe der NSAIDS) oder von Paracetamol zur Verstärkung von deren Wirksamkeit mit Besserung und Reduktion des Krankheitsverlaufs von Erkältungskrankheiten.

### Aspekt 9:

Verwendung von 25-OH D₃ und 1,25-DOH D₃ oder deren Analoga zur synergistischen Intensivierung der antiinfektiösen, antiallergischen, entzündungshemmenden und antioxidativen Wirkung von Mischterpenen, insbesondere von Pfefferminzölen (z. B. Colpermin^{®}) und standardisierten Mischterpenen (z. B. Gelomyrtol^{®}).

### Aspekt 10:

Verwendung von 25-OH D₃ oder Analoga, als Kombinationstherapeutikum, insbesondere in Form eines Kit als dünndarmlösliche Kapsel, mit einem Monoterpen, bevorzugt von 1,8-Cineol, einem Pfefferminzöl (z. B. Colpermin^{®}), oder einem standardisierten Mischterpen (z. B. Gelomyrtol^{®}) als Co-Medikation mit nasal-topischen Glukokortikosteroiden zur Intensivierung der Leitlinientherapie für obere Atemwegserkrankungen, insbesondere der akuten, allergischen, chronischen und chronisch rezidivierend polypoiden Rhinitis bzw. Rhinosinusitis.

### Aspekt 11:

Verwendung von 25-OH D₃ oder Analoga, als Kombinationstherapeutikum, insbesondere in Form eines Kit als Nasenspray, mit nasal-topischen Glukokortikosteroiden zur Intensivierung der Steroidwirkung bei oberen Atemwegserkrankungen, insbesondere der akuten, allergischen, chronischen und chronisch rezidivierenden, polypoiden Rhinitis bzw. Rhinosinusitis.

### Aspekt 12:

Verwendung von 1,25-DOH D₃, insbesondere von 25-OH D₃, oder deren Analoga als Kombinationstherapeutikum, insbesondere in Form eines Kit als Spray, bzw. Augentropfen, mit alpha-1-Sympathomimetika, insbesondere Xylometazolin, Xylometazolin+Dexpanthenol, Dexpanthenol, Oxymetazolin; Chromoglicerinsäure; Nedocromil; Vagolytika und/oder Antihistaminika für die lokale Therapie von Nase, Nasennebenhöhlen bzw. der Augen, und/oder für die Vermittlung bzw. Intensivierung abschwellender, antiviraler, antibakterieller und antientzündlicher, antipolypoider und antiallergischer Wirkungen.

### Aspekt 13:

Verwendung von 1,25-DOH D₃, insbesondere von 25-OH D₃, oder deren Analoga als Kombinationstherapeutikum, insbesondere in Form eines Kit als Spray in Form von alpha-1-Sympathomimetika, insbesondere Xylometazolin und Dexpanthenol oder Dexpanthenol alleine mit einer Erhöhung des Anteils von Dexpanthenol auf ≥ 20% mit dem Ziel einer ausreichenden entzündungshemmenden Wirkung für den Einsatz bei Rhinitis, allergischer Rhinitis und/oder chronischer Rhinosinusitis, gegebenenfalls mit *Polyposis nasi.*

### Aspekt 14:

Verwendung von 1,25-DOH D₃, insbesondere von 25-OH D₃, oder deren Analoga als Kombinationstherapeutikum, insbesondere in Form eines Kit als Spray für die lokale Therapie von Nase und Nasennebenhöhlen mit einem alpha-1-Sympathomimetikum, mit oder ohne Dexpanthenol, oder einem Glukokortikosteroid, zur Verhinderung oder Reduktion von Wirkverlusten durch Gewöhnungseffekte (Previnismus) mit anhaltender nasaler Kongestion mit Flußminderung.

### Aspekt 15:

Verwendung von 1,25-DOH D₃, insbesondere von 25-OH D₃, oder deren Analoga als Kombinationstherapeutikum, insbesondere in Form eines Kits als Spray mit einem alpha-1-Sympathomimetikum, insbesondere Xylometazolin, mit oder ohne Dexpanthenol oder Oxymetazolin, zur Behandlung von oberen Atemwegsobstruktionen sowie profibrotischen Entzündungsreaktionen bei schlafbezogenen Atmungsstörungen, insbesondere des obstruktiven und zentralen Schlafapnoesyndroms.

### Aspekt 16:

Verwendung von 1,25-DOH D₃, insbesondere von 25-OH D₃, oder deren Analoga als Kombinationstherapeutikum, insbesondere in Form eines Kits als dünndarmlösliche Kapsel, mit einem Monoterpen, bevorzugt von 1,8-Cineol, einem Pfefferminzöl (z. B. Colpermin^{®}) oder einem standardisierten Mischterpen (z. B. Gelomyrtol^{®}) als Co-Medikation der Leitlinientherapie (topischnasale Steroide, alpha-1-Sympathomimetika) für obere (akute, allergische und chronische Rhinitis bzw. Rhinosinusitis) und als Co-Medikation zur Intensivierung der Leitlinientherapie (LABA, ICS, LAMA, LABA+ICS, LABA+LAMA, LABA+ICS+LAMA) für untere Atemwegserkrankungen (Asthma, COPD, chron. Lungenemphysem), insbesondere zur Induktion und/oder Verstärkung des steroidpermissiven Effekts, den Progress von Obstruktion und Emphysem, Exazerbationen und den Steroidbedarf zu reduzieren, sowie durch Zigarettenrauch oder Feinpartikel und andere Umweltnoxen assoziierte Atemwegserkrankungen.

### Aspekt 17:

Verwendung von 1,25-DOH D₃, insbesondere von 25-OH D₃, oder deren Analoga als Kombinationstherapeutikum, insbesondere in Form eines Kits zur Zusatztherapie, mit einem Monoterpen, bevorzugt 1,8-Cineol, systemischen Glukokortikosteroid (SCS), einer intravenösen Substitutionstherapie mit alpha-1-Antitrypsin (mit Vitamin D₃ 10.000 bis 15.000 IE / Woche) oder IgG-Immunglobulinen bei AK-Mangelsyndrom, sowie einem Phosphodiesteraseinhibitor (Roflumilast, Theophyllin) zur Behandlung von oberen bzw. unteren Atemwegserkrankungen (insbesondere chronischer Rhinosinusitis, allergischer Rhinitis, COPD und/oder Asthma), insbesondere mit der Indikation, die Therapieeffekte und Ziele der Leitlinientherapie zu intensivieren, insbesondere einer Induktion und/oder Verstärkung des steroidpermissiven Effekts, den Progress von Obstruktion und Emphysem, Exazerbationen und/oder den Steroidbedarf zu reduzieren und/oder zur Prophylaxe von mit Zigarettenrauch oder Feinpartikeln sowie mit anderen Umweltnoxen assoziierten Atemwegserkrankungen.

### Aspekt 18:

Verwendung von 1,25-DOH D₃, insbesondere von 25-OH D₃, oder Analoga als Kombinationstherapeutikum, insbesondere in Form eines Kits als Tablette oder Kapsel, in Verbindung mit Leukotrienrezeptorantagonisten, insbesondere Montelukast, Pranlukast, Zaforlukast, 5-Lipoxygenase-Hemmstoffen, oder Antiallergika bzw. Antihistaminika zur Intensivierung der Leitlinientherapieempfehlungen bei allergischen Erkrankungen, insbesondere des allergischen Asthmas, und/oder der allergischen Rhinitis und Konjunktivitis, Neurodermitis und von Nahrungsmittelallergien.

### Aspekt 19:

Verwendung von 1,25-DOH D₃, insbesondere von 25-OH D₃, oder deren Analoga als Kombinationstherapeutikum, insbesondere in Form eines Kits zur Inhalation, in Verbindung mit ICS, LABAs, LAMAs, LABA+ICS sowie LABA+ICS+LAMA, insbesondere mit dem Ziel der Verbesserung des Therapieeffekts der topisch eingesetzten Leitlinientherapie für untere Atemwegserkrankungen (siehe Aspekt 16).

### Aspekt 20:

Verwendung von 1,25-DOH D₃, insbesondere von 25-OH D₃, oder deren Analoga als Kombinationstherapeutikum, insbesondere in Form eines Kits als Zusatztherapie (+Vitamin D₃ 20 bis 30.000 IE / 2 Wochen), zusammen mit einer Anti-IgE-Therapie, insbesondere mit Omalizumab, zur Hemmung der Produktion von IgE-Antikörpern, vorzugsweise zum Schutz vor allergischen Sensibilisierungen bei gleichzeitiger Wirkverstärkung bei besserer Verträglichkeit mit eventueller Option zur Dosisreduktion kostenaufwendiger Therapien mit Anti-IgE.

### Aspekt 21:

Verwendung von Monoterpenen, insbesondere von 1,8-Cineol, mit oder ohne 25-OH D₃ oder 1,25-DOH D₃, zur Kompensation niedriger Wirkstoffspiegel von Vitamin D₃ oder auch Monoterpenen durch Co-Medikation beider Wirkstoffe als Kit zur Erhöhung der körpereigenen Vitamin D-Synthese oder durch Erhöhung der oralen Dosierung von dünndarmlöslichen Kapseln (3 x 400 mg) oder als intramuskuläre Depot-Injektion alle 2 bis 4 Wochen einer Kombination aus Vit.D3 (30.000/60.000 IE) und 1,8-Cineol (5/10 g) für die Prophylaxe, Therapie und Retardierung des Progress bei schweren chronisch-aktiven entzündlichen Darmerkrankungen sowie zur Reduktion von Exazerbationen, schwerer COPD, insbesondere des Lungenemphysems mit oder ohne respiratorischer Insuffizienz, schwerem Asthma, schweren Infektionserkrankungen, allergischen Reaktionen, zur Entzündungshemmung und Steroideinsparung, Osteoporose, Herzkreislauferkrankungen, Arteriosklerose, malignen Erkrankungen und Retardierung von Alterungsprozessen.

### Aspekt 22:

Verwendung nach einem der vorangehenden Aspekte zur Hemmung oder Modulation COPD-assoziierter und COPD-unabhängiger Alterungsprozesse, insbesondere mit dem Ziel, die Erkrankungshäufigkeit und -schwere zu reduzieren und die Lebenserwartung und -qualität zu bessern und den gesamten Alterungsprozess zu retardieren.

### Aspekt 23:

Verwendung von 1,25-DOH D₃, insbesondere von 25-OH D₃, oder deren Analoga als Kombinationstherapeutikum, insbesondere in Form eines Kits als dünndarmlösliche Kapsel mit einem Monoterpen, bevorzugt von 1,8-Cineol, oder einem ätherischen Mischöl zur Behandlung von oberen Atemwegsobstruktionen sowie profibrotischen Entzündungsreaktionen bei schlafbezogenen Atmungsstörungen, insbesondere des obstruktiven und zentralen Schlafapnoesyndroms.

### Aspekt 24:

Verwendung von 1,25-DOH D₃, insbesondere von 25-OH D₃, oder deren Analoga als Kombinationstherapeutikum, insbesondere in Form eines Kits als dünndarmlösliche Kapsel, in Verbindung mit einem Glukokortikosteroid, insbesondere Budesonid (Vitamin D₃+Budesonid), und einem Zytokininhibitor- oder Rezeptorantagonisten) oder anderen Biologika zur Intensivierung der Leitlinientherapie von chronisch entzündlichen Darmerkrankungen (IBD) mit Befall des Dünn- und Dickdarms, insbesondere von *Morbus Crohn* (CD) und *Colitis ulcerosa* (CU), hepatischen Autoimmunerkrankungen, wie bei primärbilärer Zirrhose, zur Prophylaxe und Erhöhung der Steroidwirkung bei IBD.

### Aspekt 25:

Verwendung von 1,25-DOH D₃, insbesondere von 25-OH D₃, oder Analoga als Kombinationstherapeutikum, insbesondere in Form eines Kits als dünndarmlösliche Kapsel, in Verbindung mit einem Monoterpen, bevorzugt von 1,8-Cineol (Vitamin D₃+1,8-Cineol), einem Pfefferminzöl, insbesondere Colpermin^{®} (Vitamin D₃+Colpermin^{®}), oder einem standardisiertem Mischöl (z. B. Vitamin D₃+Gelomyrtol^{®}) zur Intensivierung der Leitlinientherapie von chronisch entzündlichen Darmerkrankungen (IBD), einschließlich des Reizdarmsyndroms (IBS), *Morbus Crohn* (CD) und *Colitis ulcerosa* (CU) zur Prophylaxe, Remissionserhaltung bzw. Exazerbationsprophylaxe und Erhöhung der Entzündungshemmung bei Kindern unter 14 Jahren und Erwachsenen.

### Aspekt 26:

Prophylaxe chronisch entzündlicher Darmerkrankungen durch antioxidative Agentien, insbesondere eine Kombination von Vitamin D₃, Vitamin C, und/oder Vitamin E mit entweder Pfefferminzölen (z.B. Colpermin^{®}), Monoterpenen (1,8-Cineol, L-Menthol) oder Budesonid in dünndarmlöslichen Kapseln und in Form als Nahrungsmittelzusatz.

### Aspekt 27:

Verwendung von 1.25-DOH D₃, insbesondere von 25-OH D₃, oder deren Analoga als Kombinationstherapeutikum, insbesondere in Form eines Kits als dünndarmlösliche Kapsel, in Verbindung mit Budesonid oder anderen Glukokortikosteroiden, Monoterpenen, insbesondere von 1,8-Cineol, Misch- oder standardisierten Terpenen, insbesondere von Colpermin^{®} und Gelomyrtol^{®}, zur lokalen, systemischen und synergistischen Verstärkung der Leitlinientherapie der unter Aspekt 24 genannten Darmerkrankungen mit besonderem Fokus auf die klinische Überlegenheit durch Reduktion akuter, infektiöser, allergisch oder chronisch bedingter Rezidivhäufungen mit Besserung der Therapierefraktion durch einen neuen kausalen Ansatz zur kombinierten Behandlung der Lokal- und Systementzündung bei IBD.

## Patentansprüche

1. Pharmazeutisches Kombinationstherapeutikum zur Verwendung bei der prophylaktischen und/oder therapeutischen Behandlung von entzündlichen Atemwegserkrankungen und/oder bronchopulmonalen Erkrankungen oder zur Verwendung bei der prophylaktischen und/oder therapeutischen Behandlung von entzündlichen Erkrankungen des Darms,
wobei das Kombinationstherapeutikum - in jeweils pharmazeutisch wirksamen Mengen - umfasst:
(a) mindestens einen Vitamin D-Rezeptoragonisten (VDA) und
(b) 1,8-Cineol.

2. Kombinationstherapeutikum zur Verwendung nach Anspruch 1, wobei (a) der Vitamin D-Rezeptoragonist (VDA) ausgewählt ist aus der Gruppe von 25-Hydroxy-Vitamin D₃ (Calcidiol) und/oder 1,25-Dihydroxy-Vitamin D₃ (Calcitriol), vorzugsweise 25-Hydroxy-Vitamin D₃ (Calcidiol), und/oder wobei (a) der Vitamin D-Rezeptoragonist (VDA) 25-Hydroxy-Vitamin D₃ (Calcidiol) ist und/oder wobei (a) der Vitamin D-Rezeptoragonist (VDA) eine Kombination von 25-Hydroxy-Vitamin D₃ (Calcidiol) und 1,25-Dihydroxy-Vitamin D₃ (Calcitriol) ist, insbesondere wobei das Kombinationstherapeutikum (i) 25-Hydroxy-Vitamin D₃ und (ii) 1,25-Dihydroxy-Vitamin D₃ in einem gewichtsbezogenen Mengenverhältnis von [(i): (ii)] im Bereich von 1.000: 1 bis 1 : 100, insbesondere im Bereich von 500 : 1 bis 1 : 10, vorzugsweise im Bereich von 100 : 1 bis 1 : 5, bevorzugt im Bereich von 50 : 1 bis 1 : 1, besonders bevorzugt im Bereich von 10 : 1 bis 1 : 1, aufweist; und/oder wobei der Vitamin D-Rezeptoragonist (VDA) 25-Hydroxy-Vitamin D₃ (Calcidiol) ist.

3. Kombinationstherapeutikum zur Verwendung nach Anspruch 1 oder 2, wobei das Kombinationstherapeutikum (a) den Vitamin D-Rezeptoragonisten (VDA), insbesondere 25-Hydroxy-Vitamin D₃ (Calcidiol) und/oder 1,25-Dihydroxy-Vitamin D₃ (Calcitriol), in einer Menge im Bereich von 0,0001 Gew.-% bis 5 Gew.-%, insbesondere im Bereich von 0,0005 Gew.-% bis 2 Gew.-%, vorzugsweise im Bereich von 0,001 Gew.-% bis 1,5 Gew.-%, bevorzugt im Bereich von 0,005 Gew.-% bis 1 Gew.-%, besonders bevorzugt im Bereich von 0,01 Gew.-% bis 0,75 Gew.-%, ganz besonders bevorzugt im Bereich von 0,05 Gew.-% bis 0,5 Gew.-%, bezogen auf das Kombinationstherapeutikum, enthält.

4. Kombinationstherapeutikum zur Verwendung nach einem der vorangehenden Ansprüche,
wobei das Kombinationstherapeutikum (b) 1,8-Cineol in einer Menge im Bereich von 0,1 Gew.-% bis 95 Gew.-%, insbesondere im Bereich von 0,5 Gew.-% bis 90 Gew.-%, vorzugsweise im Bereich von 1 Gew.-% bis 85 Gew.-%, bevorzugt im Bereich von 2 Gew.-% bis 80 Gew.-%, besonders bevorzugt im Bereich von 5 Gew.-% bis 75 Gew.-%, bezogen auf das Kombinationstherapeutikum, enthält; und/oder
wobei das Kombinationstherapeutikum mindestens einen mit (a) dem Vitamin D-Rezeptoragonisten (VDA) und/oder (b) 1,8-Cineol mischbaren und/oder hierin löslichen, insbesondere bei 20 °C und Atmosphärendruck flüssigen, physiologisch unbedenklichen Träger (Exzipienten) enthält, insbesondere wobei der Träger ausgewählt ist aus der Gruppe von fetten Ölen, bevorzugt Triglyceriden, besonders bevorzugt mittelkettigen Triglyceriden (*Medium Chain Triglycerides,* MCT), ganz besonders bevorzugt Triglyceriden mit C₆-C₁₂-Fettsäureresten, und/oder insbesondere wobei der Träger in einem (a) Vitamin D-Rezeptoragonist (VDA)/Träger-Mengenverhältnis und/oder in einem (b) 1,8-Cineol/Träger-Mengenverhältnis, unabhängig voneinander, im Bereich von 1.000 : 1 bis 1 : 1.000, insbesondere im Bereich von 100 : 1 bis 1 : 100, eingesetzt ist.

5. Kombinationstherapeutikum zur Verwendung nach einem der vorangehenden Ansprüche,
wobei das Kombinationstherapeutikum (c) mindestens einen weiteren pharmakologisch wirksamen Inhaltsstoff und/oder Wirkstoff umfasst,
insbesondere wobei (c) der pharmakologisch wirksame Inhaltsstoff und/oder Wirkstoff ein insbesondere systemisch applizierbares und/oder systemisches, vorzugsweise peroral applizierbares Kortikosteroid, insbesondere Glukokortikosteroid, ist, und/oder
insbesondere wobei (c) der pharmakologisch wirksame Inhaltsstoff und/oder Wirkstoff ausgewählt ist aus der Gruppe von Antiphlogistika, insbesondere nichtsteroidalen Antiphlogistika, vorzugsweise Acetylsalicylsäure und/oder Paracetamol; beta-Sympathomimetika, vorzugsweise beta-2-Sympathomimetika; Parasympatholytika und/oder Vagolytika; und Anticholinergika; sowie Mischungen und Kombinationen der vorgenannten Verbindungen, und/oder
insbesondere wobei das Kombinationstherapeutikum den (c) weiteren pharmakologisch wirksamen Inhaltsstoff und/oder Wirkstoff in einer Menge im Bereich von 0,001 Gew.-% bis 10 Gew.-%, insbesondere im Bereich von 0,01 Gew.-% bis 5 Gew.-%, vorzugsweise im Bereich von 0,1 Gew.-% bis 2 Gew.-%, bezogen auf das Kombinationstherapeutikum, enthält.

6. Kombinationstherapeutikum zur Verwendung nach einem der vorangehenden Ansprüche,
wobei das Kombinationstherapeutikum (d) mindestens ein weiteres Vitamin, insbesondere ausgewählt aus der Gruppe von Vitamin A, Vitamin C und Vitamin E, insbesondere Vitamin C und Vitamin E, enthält; und/oder
wobei das Kombinationstherapeutikum mindestens einen weiteren Inhaltsstoff aufweist, ausgewählt aus der Gruppe von Verarbeitungshilfsstoffen, Stabilisatoren, insbesondere organischen Lösemitteln, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen; und/oder
wobei das Kombinationstherapeutikum topisch applizierbar ist und/oder appliziert wird oder wobei das Kombinationstherapeutikum systemisch, insbesondere peroral applizierbar ist und/oder appliziert wird.

7. Kombinationstherapeutikum zur Verwendung nach einem der vorangehenden Ansprüche zur prophylaktischen und/oder therapeutischen Kombinationstherapie und/oder Co-Medikation von Atemwegserkrankungen und/oder bronchopulmonalen Erkrankungen und zur prophylaktischen und/oder therapeutischen Kombinationstherapie und/oder Co-Medikation von entzündlichen Erkrankungen des Darms, wobei das Kombinationstherapeutikum (a) den Vitamin D-Rezeptoragonisten (VDA) einerseits und (b) 1,8-Cineol andererseits, gegebenenfalls gemeinsam mit (c) dem weiteren pharmakologisch wirksamen Inhaltsstoff und/oder Wirkstoff und/oder (d) dem weiteren Vitamin und/oder dem weiteren Inhaltsstoff, in einer gemeinsamen und/oder einzigen Zusammensetzung aufweist.

8. Kombinationstherapeutikum zur Verwendung nach einem der vorangehenden Ansprüche zur Verwendung bei der prophylaktischen und/oder therapeutischen Behandlung von entzündlichen Atemwegserkrankungen und/oder bronchopulmonalen Erkrankungen oder zur Verwendung bei der prophylaktischen und/oder therapeutischen Behandlung von entzündlichen Erkrankungen des Darms, umfassend - in jeweils pharmazeutisch wirksamen Mengen-mindestens einen Vitamin D-Rezeptoragonisten (VDA) einerseits und 1,8-Cineol andererseits, wobei das Kombinationstherapeutikum in Form eines Kit ("*Kit of Parts*") vorliegt und/oder wobei das Kombinationstherapeutikum (a) den Vitamin D-Rezeptoragonisten (VDA) einerseits und (b) 1,8-Cineol andererseits räumlich voneinander getrennt, in voneinander verschiedenen Zusammensetzungen, aufweist.

9. Kombinationstherapeutikum zur Verwendung nach einem der Ansprüche 1 bis 8,
wobei die Atemwegserkrankung, insbesondere die bronchopulmonale Erkrankung, eine entzündliche Erkrankung der oberen oder unteren Atemwege ist; und/oder wobei die Atemwegserkrankung, insbesondere die bronchopulmonale Erkrankung, eine entzündliche, insbesondere infektexazerbierte und/oder steroidpflichtige Atemwegserkrankung ist; und/oder
wobei die Atemwegserkrankung, insbesondere die bronchopulmonale Erkrankung, Asthma bronchiale oder Bronchitis ist; und/oder
wobei die Atemwegserkrankung, insbesondere die bronchopulmonale Erkrankung, eine chronische obstruktive Lungenerkrankung (COPD) ist, insbesondere eine chronische obstruktive Bronchitis oder ein Lungenemphysem; und/oder
wobei die Atemwegserkrankung, insbesondere die bronchopulmonale Erkrankung, eine tabakrauchinduzierte, insbesondere nikotininduzierte akute oder chronische Atemwegserkrankung, insbesondere entzündliche Atemwegserkrankung, ist; und/oder
wobei die Atemwegserkrankung, insbesondere die bronchopulmonale Erkrankung, eine Frühform von COPD, insbesondere im Stadium nach GOLD 0 oder I, oder eine Frühform des Asthma bronchiale, insbesondere Stadium nach GINA 0 oder I, ist; und/oder wobei die Atemwegserkrankung eine Erkältungserkrankung und/oder ein grippaler Infekt ist und/oder wobei die Atemwegserkrankung Rhinitis und/oder Sinusitis ist; und/oder wobei die insbesondere entzündliche Erkrankung des Darms *Morbus Crohn* und/oder *Colitis ulcerosa* und/oder eine chronisch entzündliche Darmerkrankung (*Inflammatory Bowel Disease,* IBD) ist.

10. Kombinationstherapeutikum zur Verwendung nach einem der Ansprüche 1 bis 8
zur insbesondere synergistischen Steigerung der antiinflammatorischen und/oder antioxidativen Wirkung topischer, insbesondere inhalativer Kortikosteroide, insbesondere Glukokortikoide; und/oder
zur Dosisreduktion von topisch applizierbaren, insbesondere inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen; und/oder
zur Induktion und/oder Verstärkung des steroidpermissiven Effektes von topisch applizierbaren, insbesondere inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen; und/oder
zur Vermeidung oder Reduktion eines Gewöhnungseffektes von beta-Sympathomimetika bei der prophylaktischen und/oder therapeutischen Behandlung von Atemwegserkrankungen, insbesondere bronchopulmonalen Erkrankungen, insbesondere unter Dauertherapie bei allen Schweregraden von COPD und Asthma bronchiale.

11. Kombinationstherapeutikum zur Verwendung nach einem der Ansprüche 1 bis 8
in Kombination mit mindestens einem topisch applizierbaren, insbesondere inhalativen Atemwegstherapeutikum, insbesondere in Kombination mit einem inhalativen Kortikosteroid, zur Reduktion des Bedarfs oder zum Ersatz systemischer Kortikosteroide oder anderer antiinflammatorisch und/oder immunsuppressiv wirkender systemischer Substanzen bei der prophylaktischen und/oder therapeutischen Behandlung von Atemwegserkrankungen, insbesondere bronchopulmonalen Erkrankungen.

12. Kombinationstherapeutikum zur Verwendung nach einem der Ansprüche 1 bis 8
zur gegenseitigen Wirkverstärkung (a) des Vitamin D-Rezeptoragonisten (VDA) einerseits und (b) 1,8-Cineol andererseits, insbesondere für die prophylaktische und/oder therapeutische Behandlung saisonaler Vitamin D₃-Mangelzustände, insbesondere winterlicher Vitamin D₃-Mangelzustände, vorzugsweise zur Besserung der Infektabwehr und zur verbesserten entzündungshemmenden Therapie der Atemwege, insbesondere mit dem Ziel, akute und chronische Exazerbationen bei chronisch obstruktiver Bronchitis, Asthma und/oder chronisch entzündlichen und/oder allergischen Erkrankungen, insbesondere bei chronischer Rhinosinusitis und/oder allergischer Rhinitis, der oberen Atemwege zu reduzieren; und/oder
zur insbesondere synergistischen Intensivierung der antiinfektiösen, antiallergischen, entzündungshemmenden und/oder antioxidativen Wirkung von 1,8-Cineol vorzugsweise in peroraler Darreichungsform, bevorzugt in Form von dünndarmlöslichen Kapseln; und/oder
zur lokalen Applikation und/oder Verabreichung, insbesondere nasalen und/oder inhalativen Applikation und/oder Verabreichung, oder zur systemischen Applikation und/oder Verabreichung zur prophylaktischen und/oder therapeutischen Behandlung von viralen und/oder bakteriellen Erkältungserkrankungen, insbesondere in Kombination mit mindestens einer weiteren Wirksubstanz, vorzugsweise mit Vitaminen, insbesondere Vitamin C und oder Vitamin E, Acetylsalicylsäure und/oder mindestens einem weiteren nichtsteroidalen Antiphlogistikum, insbesondere ausgewählt aus der Gruppe der NSAIDS oder Paracetamol, insbesondere zur Verstärkung der Wirksamkeit der in Kombination eingesetzten Wirksubstanzen, insbesondere mit dem Ziel der Verbesserung und Reduktion des Krankheitsverlaufs von Erkältungskrankheiten.

13. Kombinationstherapeutikum zur Verwendung nach einem der Ansprüche 1 bis 8
zur Co-Medikation und/oder als Zusatztherapie insbesondere in Form eines Kits, mit mindestens einem weiteren Wirkstoff, wobei der Wirkstoff ausgewählt ist aus der Gruppe von systemischen Glukokortikosteroiden (SCS), intravenösen Substitutionstherapien mit α₁-Antitrypsin, IgG-Immunglobulinen, insbesondere bei AK-Mangelsyndrom, Phosphodiesteraseinhibitoren, insbesondere Roflumilast und/oder Theophyllin, zur prophylaktischen und/oder therapeutischen Behandlung von Erkrankungen der oberen Atemwege, insbesondere akuter, allergischer und/oder chronischer Rhinitis und/oder Rhinosinusitis, und/oder zur prophylaktischen und/oder therapeutischen Behandlung von Erkrankungen der unteren Atemwege, insbesondere Asthma, COPD und/oder chronischen Lungenemphysemen, und/oder zur prophylaktischen und/oder therapeutischen Behandlung von durch Zigarettenrauch, Feinpartikel und/oder durch andere Umweltnoxen induzierten und/oder assoziierten Atemwegserkrankungen, insbesondere mit dem Ziel, die Therapieeffekte von Leitlinientherapien, wie zuvor definiert, zu intensivieren und/oder zu verbessern, und/oder mit dem Ziel einer Induktion und/oder Verstärkung von steroidpermissiven Effekten, insbesondere um den progressiven Verlauf von Obstruktionen und/oder Emphysemen, Exazerbationen und/oder den Steroidbedarf zu reduzieren; und/oder
zur Kompensation niedriger Wirkstoffspiegel von Vitamin D₃ und/oder niedriger Wirkstoffspiegel von 1,8-Cineol, insbesondere wobei das Kombinationstherapeutikum als Kit vorliegt, für die Prophylaxe, Therapie und/oder Retardierung des progressiven Verlaufs bei insbesondere schweren entzündlichen Darmerkrankungen, insbesondere chronisch-aktiven entzündlichen Darmerkrankungen, und/oder für die Reduktion von Exazerbationen, schwerer COPD, insbesondere des Lungenemphysems mit oder ohne respiratorischer Insuffizienz, schwerem Asthma, schweren Infektionserkrankungen, allergischen Reaktionen, zur Entzündungshemmung und Steroideinsparung, Osteoporose, Herzkreislauferkrankungen, Arteriosklerose, malignen Erkrankungen und Retardierung von Alterungsprozessen.

14. Kombinationstherapeutikum zur Verwendung nach einem der Ansprüche 1 bis 8, insbesondere wobei das Kombinationstherapeutikum als Kit vorliegt,
zur prophylaktischen und/oder therapeutischen Behandlung von oberen Atemwegsobstruktionen sowie von profibrotischen Entzündungsreaktionen, insbesondere bei schlafbezogenen Atmungsstörungen, insbesondere des obstruktiven und zentralen Schlafapnoesyndroms; und/oder
zur Intensivierung und/oder Verbesserung der Wirksamkeit und/oder zur Wirkeffizienzsteigerung von Leitlinientherapien von chronisch entzündlichen Darmerkrankungen, insbesondere des Reizdarmsyndroms, Morbus Crohn und Colitis ulcerosa, insbesondere mit dem Ziel der Prophylaxe, insbesondere Exazerbationsprophylaxe, Remissionserhaltung und Erhöhung der Entzündungshemmung bei Kindern und Erwachsenen.

15. Kombinationstherapeutikum zur Verwendung nach einem der Ansprüche 1 bis 8, zur Dosisreduktion von 1,8-Cineol und/oder zur Dosisreduktion mindestens eines weiteren pharmakologischen Wirk- und/oder Inhaltsstoffs, insbesondere wie in Anspruch 4 definiert, und/oder zur Dosisreduktion mindestens eines systemisch oder topisch applizierbaren pharmakologischen Wirkstoffs, vorzugsweise eines Kortikosteroids, insbesondere wobei die zu verabreichende Menge und/oder Dosis des jeweiligen Wirkstoffs, unabhängig voneinander, um mindestens 5 %, insbesondere mindestens 10 %, vorzugsweise mindestens 20 %, bevorzugt mindestens 40 %, bezogen auf ein entsprechendes Therapeutikum ohne Vitamin D-Rezeptoragonist (VDA), verringert wird.

## Claims

1. A pharmaceutical combination therapeutic for use in the prophylactic and/or therapeutic treatment of inflammatory respiratory diseases and/or bronchopulmonary diseases, or for use in the prophylactic and/or therapeutic treatment of inflammatory diseases of the intestines,
wherein the combination therapeutic comprises the following - each in pharmaceutically effective amounts:
(a) at least one vitamin D receptor agonist (VDA), and
(b) 1,8-cineol.

2. A combination therapeutic for use according to claim 1, wherein (a) the vitamin D receptor agonist (VDA) is selected from the group of 25-hydroxy vitamin D₃ (calcidiol), and/or 1,25-dihydroxy vitamin D₃ (calcitriol), preferably 25-hydroxy vitamin D₃ (calcidiol), and/or wherein (a) the vitamin D receptor agonist (VDA) is 25-hydroxy vitamin D₃ (calcidiol), and/or wherein (a) the vitamin D receptor agonist (VDA) is a combination of 25-hydroxy vitamin D₃ (calcidiol) and 1,25-dihydroxy vitamin D₃ (calcitriol), in particular, wherein the combination therapeutic comprises (i) 25-hydroxy vitamin D₃ and (ii) 1,25-dihydroxy vitamin D₃ at a weight-based proportion of [(i):(ii)] in the range of 1,000:1 to 1:100, in particular in the range of 500:1 to 1:10, preferably in the range of 100:1 to 1:5, preferably in the range of 50:1 to 1:1, particularly preferred in the range of 10:1 to 1:1 and/or wherein the vitamin D receptor agonist (VDA) is 25-hydroxy vitamin D₃ (calcidiol).

3. The combination therapeutic for use according to claims 1 or 2, wherein the combination therapeutic (a) comprises the vitamin D receptor agonist (VDA), in particular 25-hydroxy vitamin D₃ (calcidiol) and/or 1,25-dihydroxy vitamin D₃ (calcitriol), at an amount in the range of 0.0001% by weight to 5% by weight, in particular in the range of 0.0005% by weight to 2% by weight, preferably in the range of 0.001% by weight to 1.5% by weight, preferably in the range of 0.005% by weight to 1% by weight, particularly preferred in the range of 0.01% by weight to 0.75% by weight, most particularly preferred in the range of 0.05% by weight to 0.5% by weight, based on the combination therapeutic.

4. The combination therapeutic for use according to one of the previous claims, wherein the combination therapeutic (b) comprises 1,8-cineole at an amount in the range of 0.1% by weight to 95% by weight, in particular in the range of 0.5% by weight to 90% by weight, preferably in the range of 1% by weight to 85% by weight, preferably in the range of 2% by weight to 80% by weight, particularly preferred in the range of 5% by weight to 75% by weight, based on the combination therapeutic, and/or
wherein the combination therapeutic comprises at least one physiologically acceptable carrier (excipient) that is (a) miscible with the vitamin D receptor agonist (VDA), and/or (b) miscible with 1,8-cineole, and/or soluble therein, being liquid, in particular, at 20 °C and at atmospheric pressure, in particular, wherein the carrier is selected from the group of fatty oils, preferably triglycerides, particularly preferred medium-chain triglycerides (MCT), most particularly preferred triglycerides having C₆-C₁₂ fatty acid residues, and/or in particular, wherein the carrier is independently utilized at a (a) proportion of vitamin D receptor agonist (VDA)/carrier, and/or a (b) proportion of 1,8-cineole/carrier in the range of 1,000:1 to 1:1,000, in particular in the range of 100:1 to 1:100.

5. The combination therapeutic for use according to one of the previous claims, wherein the combination therapeutic comprises (c) at least one additional pharmacologically effective ingredient and/or active agent,
in particular, wherein (c) the pharmacologically effective ingredient and/or active agent is, in particular, a systemically applicable, and/or systemically, preferably perorally applicable corticosteroid, in particular glucocorticosteroid, and/or
in particular, wherein (c) the pharmacologically effective ingredient and/or active agent is selected from the group of antiphlogistics, in particular non-steroidal antiphlogistics, preferably acetyl salicylic acid, and/or paracetamol; beta-sympathomimetics, in particular beta-2-sympathomimetics; parasympatholytics, and/or vagolytics; and anticholinergics; as well as mixtures and combinations of the compounds mentioned above, and/or
in particular, wherein the combination therapeutic comprises (c) the additional pharmacologically effective ingredient and/or active agent at an amount in the range of 0.001% by weight to 10% by weight, in particular in the range of 0.01% by weight to 5% by weight, preferably in the range of 0.1% by weight to 2% by weight, based on the combination therapeutic.

6. The combination therapeutic for use according to one of the previous claims, wherein the combination therapeutic comprises (d) at least one additional vitamin, in particular selected from the group of vitamin A, vitamin C, and vitamin E, in particular vitamin C and vitamin E; and/or
wherein the combination therapeutic comprises at least one additional ingredient, selected from the group of processing adjuvants, stabilizers, in particular organic solvents, emulsifiers, antioxidants, wetting agents, thickeners, antiseptics, dyes, aromatics, scents, flavors, diluents, binders, surfactants, vitamins, trace elements, minerals, micronutrients, and/or essential oils, as well as the combinations thereof;
and/or
wherein the combination therapeutic is applicable topically, and/or is applied, topically, or wherein the combination therapeutic is applicable, and/or is applied, systemically, in particular perorally.

7. The combination therapeutic for use according to one of the previous claims for the prophylactic and/or therapeutic combination therapy, and/or co-medication of respiratory diseases and/or bronchopulmonary diseases, and for the prophylactic and/or therapeutic combination therapy, and/or co-medication of inflammatory diseases of the intestines, wherein the combination therapeutic comprises both (a) the vitamin D receptor agonist (VDA) and (b) 1,8-cineole, optionally together with (c) the additional pharmacologically acceptable ingredient and/or active agent, and/or (d) the additional vitamin, and/or the additional ingredient, in a mutual and/or single composition.

8. The combination therapeutic for use according to one of the previous claims for use with the prophylactic and/or therapeutic treatment of inflammatory respiratory diseases and/or bronchopulmonary disease, or for use with the prophylactic and/or therapeutic treatment of inflammatory diseases of the intestines, comprising - each in pharmaceutically effective amounts - at least one vitamin D receptor agonist (VDA) and 1,8-cineole, wherein the combination therapeutic is present in the form of a kit ("kit of parts"), and/or wherein the combination therapeutic comprises both (a) the vitamin D receptor agonist (VDA) and (b) 1,8-cineole, each being spatially separated from each other, in compositions that differ from each other.

9. The combination therapeutic for use according to claims 1 to 8,
wherein the respiratory disease, in particular the bronchopulmonary disease, is an inflammatory disease of the upper and lower respiratory systems; and/or
wherein the respiratory disease, in particular the bronchopulmonary disease, is an inflammatory, in particular infection-exacerbating and/or steroid-obligatory respiratory disease; and/or
wherein the respiratory disease, in particular the bronchopulmonary disease, is bronchial asthma or bronchitis; and/or
wherein the respiratory disease, in particular the bronchopulmonary disease, is chronic obstructive pulmonary disease (COPD), in particular chronic obstructive bronchitis, or pulmonary emphysema; and/or
wherein the respiratory disease, in particular the bronchopulmonary disease, is tobacco smoke induced, in particular nicotine induced acute or chronic respiratory disease, in particular inflammatory respiratory disease; and/or
wherein the respiratory disease, in particular the bronchopulmonary disease, is the early onset form of COPD, in particular stage 0 or I according to GOLD, or an early onset form of bronchial asthma, in particular stage 0 or I according to GINA; and/or
wherein the respiratory disease is a cold, and/or flue, and/or wherein the respiratory disease is rhinitis and/or sinusitis; and/or
wherein, in particular, the inflammatory disease of the intestines is *Morbus Crohn,* and/or *Colitis ulcerosa,* and/or a chronic *inflammatory bowel disease* (IBD).

10. The combination therapeutic for use according to one of the claims 1 to 8 in particular, for synergistically increasing the anti-inflammatory and/or anti-oxidative effect of topical, in particular, inhalative corticosteroids, in particular glucocorticoids;
and/or
for the reduction of the dosage of topically applicable, in particular inhalative respiratory therapeutics, preferably inhalative corticosteroids, with the prophylactic and/or therapeutic treatment of respiratory diseases, in particular bronchopulmonary diseases; and/or
for the induction and/or enhancement of the steroid-permissive effect of topically applicable, in particular inhalative respiratory therapeutics, preferably inhalative corticosteroids, with the prophylactic and/or therapeutic treatment of respiratory diseases, in particular bronchopulmonary diseases; and/or
for avoiding or reducing the habitual effect of beta-sympathomimetics with the prophylactic and/or therapeutic treatment of respiratory diseases, in particular bronchopulmonary diseases, in particular with a long-term therapy with all degrees of severity of COPD and bronchial asthma.

11. The combination therapeutic for use according to one of the claims 1 to 8 in combination with at least one topically applicable, in particular inhalative respiratory therapeutic, in particular in combination with an inhalative corticosteroid for the reduction of the requirement of, or for the replacement of, systemic corticosteroids, or other anti-inflammatory and/or immunosuppressive acting systemic substances with the prophylactic and/or therapeutic treatment of respiratory diseases, in particular bronchopulmonary diseases.

12. The combination therapeutic for use according to one of the claims 1 to 8 for the coadjutant enhancement of effectiveness of both (a) the vitamin D receptor agonist (VDA) and (b) 1,8-cineole, in particular for the prophylactic and/or therapeutic treatment of seasonal vitamin D₃ deficiencies, in particular of vitamin D₃ deficiencies in winter, preferably for improving the defense against infection and for improving the anti-inflammatory therapy of respiratory diseases, in particular with the aim of reducing acute and chronic exacerbations with chronic obstructive bronchitis, asthma, and/or chronic inflammatory and/or allergic diseases, in particular with chronic rhinosinusitis, and/or allergic rhinitis, of the upper respiratory systems; and/or
in particular, for the synergistic intensifying of the anti-infections, anti-allergenic, anti-inflammatory, and/or anti-oxidative effect of 1,8-cineole, preferably in peroral pharmaceutical form, preferably in the form of capsules soluble in the small intestines;
and/or
for local application and/or administration, in particular nasal and/or inhalative application and/or administration, or for systemic application and/or administration for the prophylactic and/or therapeutic treatment of virus and/or bacterial colds, in particular in combination with at least one additional active ingredient, preferably with vitamins, in particular vitamin C and/or vitamin E, acetyl salicylic acid, and/or at least one additional non-steroidal antichloristic, in particular selected from the group of NSAIDS, or of Paracetamol, in particular for enhancing the effectiveness of the active ingredients utilized in combination, in particular with the aim of improving and reducing the etiopathology of common colds.

13. The combination therapeutic for use according to one of the claims 1 to 8 for co-medication and/or as additional therapy, in particular in the form of a kit, comprising at least one additional active agent, wherein the active agent is selected from the group of systemic glucocorticosteroids (SCS), intravenous substitution therapies using α₁-antitrypsin, IgG-immunoglobulins, in particular with AK deficiency syndrome, phosphodiesterase inhibitors, in particular Roflumilast and/or Theophylline, for the prophylactic and/or therapeutic treatment of diseases of the upper respiratory system, in particular of acute, allergic, and/or chronic rhinitis and/or rhinosinusitis, and/or for the prophylactic and/or therapeutic treatment of diseases of the lower respiratory system, in particular asthma, COPD, and/or chronic pulmonary emphysema, and/or for the prophylactic and/or therapeutic treatment of respiratory diseases induced by, and/or associated with, cigarette smoke, fine particles, and/or other environmental toxins, in particular with the aim of intensifying and/or improving the therapeutic effects of guideline therapies, as defined above, and/or with the aim of inducing and/or enhancing steroid-permissive effects, in particular in order to reduce the progressive course of obstructions and/or emphysemas, exacerbations, and/or the need for steroids; and/or for the compensation of a low active agent level of vitamin D₃, and/or the low active agent level of 1,8-cineole, in particular, wherein the combination therapeutic is present as a kit, for the prophylaxis, therapy, and/or retardation of the progressive course of, in particular severe inflammatory bowel diseases, in particular chronic-active inflammatory bowel diseases, and/or for the reduction of exacerbations, severe COPD, in particular of the pulmonary emphysema with or without respiratory insufficiency, severe asthma, severe infectious diseases, allergic reactions, for inhibiting infections and saving on steroids, osteoporosis, cardiovascular diseases, arteriosclerosis, malignant diseases, and retardation of aging processes.

14. The combination therapeutic for use according to one of the claims 1 to 8, wherein the combination therapeutic is present as a kit,
for the prophylactic and/or therapeutic treatment of upper respiratory obstructions, as well as of profibrotic inflammation reactions, in particular with sleep-related breathing disturbances, in particular of the obstructive and central sleep apnea syndrome; and/or for intensifying and/or improving the effectiveness, and/or for increasing the active efficiency of guideline therapies for chronic inflammatory bowel diseases, in particular of irritable bowel syndrome, Morbus Crohn, and Colitis ulcerosa, in particular with the aim of prophylaxis, in particular exacerbation prophylaxis, remission retention, and increase of inflammation inhibition with children and adults.

15. The combination therapeutic for use according to one of the claims 1 to 8 for the dosage reduction of 1,8-cineole, and/or for the dosage reduction of at least one additional pharmacological active agent and/or ingredient, in particular as defined in claim 4, and/or for the dosage reduction of at least one systemically or topically applicable pharmacological active agent, preferably a corticosteroid, in particular, wherein the amount and/or dosage of the respective active agent to be administered is independently reduced by at least 5%, in particular by at least 10%, preferably by at least 20%, preferably by at least 40%, based on a respective therapeutic without any vitamin D receptor agonist (VDA).

## Revendications

1. Agent thérapeutique pharmaceutique en association pour utilisation lors du traitement prophylactique et/ou thérapeutique de maladies inflammatoires des voies respiratoires et/ou de bronchopneumopathies, ou pour utilisation lors du traitement prophylactique et/ou thérapeutique de maladies inflammatoires de l'intestin,
l'agent thérapeutique en association comprenant, dans chaque cas en des quantités pharmaceutiquement efficace :
(a) au moins un agoniste des récepteurs de la vitamine D (VDA) et
(b) du 1,8-cinéol.

2. Agent thérapeutique en association pour utilisation selon la revendication 1, dans lequel (a) l'agoniste des récepteurs de la vitamine D (VDA) est choisi dans le groupe consistant en la 25-hydroxy-vitamine D₃ (calcidiol) et/ou la 1,25-dihydroxy-vitamine D₃ (calcitriol), de préférence la 25-hydroxy-vitamine D₃ (calcidiol) et/ou dans lequel (a) l'agoniste des récepteurs de la vitamine D (VDA) est la 25-hydroxy-vitamine D₃ (calcidiol) et/ou dans lequel (a) l'agoniste des récepteurs de la vitamine D (VDA) est une association de 25-hydroxy-vitamine D₃ (calcidiol) et de 1,25-dihydroxy-vitamine D₃ (calcitriol), en particulier dans lequel l'agent thérapeutique en association comprend (i) la 25-hydroxy-vitamine D₃ et (ii) la 1,25-dihydroxy-vitamine D₃ selon une proportion pondérale de [(i):(ii)] comprise dans la plage de 1000:1 à 1:100, en particulier dans la plage de 500:1 à 1:10, de préférence dans la plage de 100:1 à 1:5, préférentiellement dans la plage de 50:1 à 1:1, d'une manière particulièrement préférée dans la plage de 10:1 à 1:1 ; et/ou dans lequel l'agoniste des récepteurs de la vitamine D (VDA) est la 25-hydroxy-vitamine D₃ (calcidiol).

3. Agent thérapeutique en association pour utilisation selon la revendication 1 ou 2, dans lequel l'agent thérapeutique en association contient (a) l'agoniste des récepteurs de la vitamine D (VDA), en particulier la 25-hydroxy-vitamine D₃ (calcidiol) et/ou la 1,25-dihydroxy-vitamine D₃ (calcitriol), en une quantité comprise dans la plage de 0,0001 % en poids à 5 % en poids, en particulier dans la plage de 0,0005 % en poids à 2 % en poids, de préférence dans la plage de 0,001 % en poids à 1,5 % en poids, préférentiellement dans la plage de 0,005 % en poids à 1 % en poids, d'une manière particulièrement préférée dans la plage de 0,01 % en poids à 0,75 % en poids, d'une manière tout particulièrement préférée dans la plage de 0,05 % en poids à 0,5 % en poids, par rapport à l'agent thérapeutique en association.

4. Agent thérapeutique en association pour utilisation selon l'une des revendications précédentes,
dans lequel l'agent thérapeutique en association contient (b) le 1,8-cinéol en une quantité comprise dans la plage de 0,1 % en poids à 95 % en poids, en particulier dans la plage de 0,5 % en poids à 90 % en poids, de préférence dans la plage de 1 % en poids à 85 % en poids, préférentiellement dans la plage de 2 % en poids à 80 % en poids, d'une manière particulièrement préférée dans la plage de 5 % en poids à 75 % en poids, par rapport à l'agent thérapeutique en association ; et/ou
dans lequel l'agent thérapeutique en association contient au moins un support (excipient), physiologiquement inoffensif, miscible (a) à l'agoniste des récepteurs de la vitamine D (VDA) et/ou (b) au 1,8-cinéol, et/ou soluble dans ces derniers, en particulier liquide à 20°C et sous la pression atmosphérique, en particulier dans lequel le support est choisi dans le groupe des huiles grasses, préférentiellement des triglycérides, d'une manière particulièrement préférée des triglycérides à chaîne moyenne (*Medium Chain Triglycerides,* MCT), d'une manière tout particulièrement préférée des triglycérides ayant des résidus d'acides gras en C₆-C₁₂, et/ou en particulier dans lequel le support est utilisé selon une proportion pondérale (a) agoniste des récepteurs de la vitamine D (VDA)/support et/ou selon une proportion pondérale (b) 1,8-cinéol/support, indépendamment l'une de l'autre, comprises dans la plage de 1000:1 à 1:1000, en particulier dans la plage de 100:1 à 1:100.

5. Agent thérapeutique en association pour utilisation selon l'une des revendications précédentes,
dans lequel l'agent thérapeutique en association (c) comprend au moins un autre ingrédient et/ou principe actif pharmacologiquement efficace,
en particulier dans lequel (c) l'ingrédient et/ou le principe actif pharmacologiquement efficace est un corticostéroïde, pouvant être administré en particulier par voie systémique et/ou pouvant être administré par voie systémique, de préférence par voie orale, et/ou
en particulier dans lequel (c) l'ingrédient et/ou le principe actif pharmacologiquement efficace sont choisis dans le groupe consistant en les antiphlogistiques, en particulier les antiphlogistiques non stéroïdiens, de préférence l'acide acétylsalicylique et/ou le paracétamol, les bêta-sympathicomimétiques, de préférence les bêta-2-sympathicomimétiques ; les parasympathicolytiques et/ou les vagolytiques ; et les anticholinergiques ; ainsi que les mélanges et associations des composés mentionnés ci-dessus, et/ou
en particulier dans lequel l'agent thérapeutique en association contient (c) l'autre ingrédient et/ou principe actif pharmacologiquement efficace en une quantité comprise dans la plage de 0,001 % en poids à 10 % en poids, en particulier dans la plage de 0,01 % en poids à 5 % en poids, de préférence dans la plage de 0,1 % en poids à 2 % en poids, par rapport à l'agent thérapeutique en association.

6. Agent thérapeutique en association pour utilisation selon l'une des revendications précédentes,
dans lequel l'agent thérapeutique en association (d) contient au moins une autre vitamine, en particulier choisie dans le groupe consistant en la vitamine A, la vitamine C et la vitamine E, en particulier la vitamine C et la vitamine E ; et/ou
dans lequel l'agent thérapeutique en association comprend au moins un autre ingrédient, choisi dans le groupe consistant en les auxiliaires de mise en oeuvre, les stabilisants, en particulier les solvants organiques, les émulsifiants, les antioxydants, les humectants, les épaississants, les antiseptiques, les colorants, les aromatisants, les substances odorantes, les parfums, les diluants, les liants, les mouillants, les vitamines, les oligoéléments, les substances minérales, les micronutriments et/ou les huiles essentielles, ainsi que leurs combinaisons ; et/ou
dans lequel l'agent thérapeutique en association peut être administré et/ou est administré par voie topique, ou dans lequel l'agent thérapeutique en association peut être administré ou est administré par voie systémique, en particulier par voie orale.

7. Agent thérapeutique en association pour utilisation selon l'une des revendications précédentes, pour un traitement en association et/ou une co-médication, prophylactique et/ou thérapeutique, de maladies des voies respiratoires et/ou des bronchopneumopathies et/ou pour le traitement en association et/ou la co-médication prophylactique et/ou thérapeutique de maladies inflammatoires de l'intestin, dans lequel l'agent thérapeutique en association comprend d'une part (a) l'agoniste des récepteurs de la vitamine D (VDA) et (b) d'autre part du 1,8-cinéol, éventuellement en même temps que (c) l'autre ingrédient et/ou principe actif pharmacologiquement efficace et/ou (d) l'autre vitamine et/ou l'autre ingrédient, selon une composition commune et/ou unique.

8. Agent thérapeutique en association pour utilisation selon l'une des revendications précédentes, pour utilisation lors du traitement prophylactique et/ou thérapeutique de maladies inflammatoires des voies aériennes et/ou des bronchopneumopathies et/ou pour utilisation lors du traitement prophylactique et/ou thérapeutique de maladies inflammatoires de l'intestin, comprenant - dans chaque cas en des quantités pharmaceutiquement efficaces, d'une part au moins un agoniste des récepteurs de la vitamine D (VDA) et d'autre part du 1,8-cinéol, dans lequel l'agent thérapeutique en association se présente sous forme d'un kit *("Kit of Parts")* et/ou dans lequel l'agent thérapeutique en association comprend d'une part (a) l'agoniste des récepteurs de la vitamine D (VDA) et d'autre part (b) du 1,8-cinéol, spatialement séparés l'un de l'autre, selon des compositions différentes l'une de l'autre.

9. Agent thérapeutique en association pour utilisation selon l'une des revendications 1 à 8,
dans lequel la maladie des voies respiratoires, en particulier la bronchopneumopathie, est une maladie inflammatoire des voies respiratoires supérieures ou inférieures et/ou
dans lequel la maladie des voies respiratoires, en particulier la bronchopneumopathie, est une maladie des voies respiratoires inflammatoire, en particulier exacerbée par une infection, et/ou justifiant d'un traitement par des stéroïdes ; et/ou
dans lequel la maladie des voies respiratoires, en particulier la bronchopneumopathie, est l'asthme bronchique ou la bronchite ; et/ou
dans lequel la maladie des voies respiratoires, en particulier la bronchopneumopathie, est une bronchopneumopathie chronique obstructive (BPCO), en particulier une bronchite obstructive chronique ou un emphysème pulmonaire ; et/ou
dans lequel la maladie des voies respiratoires, en particulier la bronchopneumopathie, est une maladie des voies respiratoires aiguë ou chronique, provoquée par la fumée du tabac, en particulier provoquée par la nicotine, en particulier une maladie inflammatoire des voies respiratoires ; et/ou
dans lequel la maladie des voies respiratoires, en particulier la bronchopneumopathie, est une forme précoce de BPCO, en particulier au stade 0 ou 1 selon GOLD, ou une forme précoce de l'asthme bronchique, en particulier au stade 0 ou 1 selon GINA ; et/ou
dans lequel la maladie des voies respiratoires est une maladie de refroidissement et/ou une infection grippale, et/ou dans lequel la maladie des voies respiratoires est une rhinite et/ou une sinusite ; et/ou
dans lequel la maladie en particulier inflammatoire de l'intestin est la maladie de Crohn ou la rectocolite hémorragique, et/ou une affection intestinale inflammatoire chronique *(Inflammatory Bowel Disease, IBD).*

10. Agent thérapeutique en association pour utilisation selon l'une des revendications 1 à 8,
pour une augmentation, en particulier synergique, de l'action anti-inflammatoire et/ou anti-oxydative de corticostéroïdes, en particulier de glucocorticoïdes en administration topique, en particulier en inhalation ; et/ou
pour la réduction de la dose d'agent thérapeutique des voies respiratoires pouvant être administrée par voie topique, en particulier par inhalation, de préférence de corticostéroïdes en inhalation, lors du traitement prophylactique et/ou thérapeutique de maladies des voies respiratoires, en particulier des bronchopneumopathies ; et/ou
pour l'induction et/ou le renforcement de l'effet permissif des stéroïdes d'agents thérapeutiques des voies respiratoires pouvant être administrés par voie topique, en particulier en inhalation, de préférence de corticostéroïdes en inhalation, lors du traitement prophylactique et/ou thérapeutique de maladies des voies respiratoires, en particulier des bronchopneumopathies ; et/ou
pour éviter ou réduire un effet d'accoutumance de bêta-sympathicomimétique lors du traitement prophylactique et/ou thérapeutique de maladies des voies respiratoires, en particulier des bronchopneumopathies, en particulier en traitement de longue durée pour tous les grades de sévérité de la BPCO et de l'asthme bronchique.

11. Agent thérapeutique en association pour utilisation selon l'une des revendications 1 à 8,
en association avec au moins un agent thérapeutique des voies respiratoires pouvant être administré par voie topique, en particulier en inhalation, en particulier en association avec un corticostéroïde en inhalation, pour la réduction du besoin ou pour remplacer des corticostéroïdes systémiques à effet anti-inflammatoire et/ou immunosuppresseur, lors du traitement prophylactique et/ou thérapeutique de maladies des voies respiratoires, en particulier des bronchopneumopathies.

12. Agent thérapeutique en association pour utilisation selon l'une des revendications 1 à 8,
pour le renforcement réciproque de l'action, d'une part (a) de l'agoniste des récepteurs de la vitamine D (VDA), et d'autre part (b) du 1,8-cinéol, en particulier pour le traitement prophylactique et/ou thérapeutique d'états de carence saisonnière en vitamine D₃, en particulier d'états hivernaux de carence en vitamine D₃, de préférence pour améliorer la défense contre les infections et pour le traitement anti-inflammatoire amélioré des voies respiratoires, en particulier avec pour objectif une réduction des exacerbations aiguës et chroniques lors d'une bronchite obstructive chronique, d'un asthme et/ou de maladies inflammatoires et/ou allergiques chroniques, en particulier lors d'une rhino-sinusite chronique et/ou une rhinite allergique, des voies respiratoires supérieures ; et/ou
pour l'intensification en particulier synergique de l'action anti-infectieuse, antiallergique, anti-inflammatoire et/ou anti-oxydative du 1,8-cinéol, de préférence sous une forme posologique par voie orale, préférentiellement sous forme de gélules solubles dans l'intestin grêle ; et/ou
pour l'application et/ou l'administration locale, en particulier pour l'application et/ou l'administration nasale et/ou en inhalation, ou pour l'application et/ou l'administration systémique pour le traitement prophylactique et/ou thérapeutique de maladies de refroidissement virales et/ou bactériennes, en particulier en association avec au moins un autre principe actif, de préférence avec des vitamines, en particulier la vitamine C et/ou la vitamine E, l'acide acétylsalicylique et/ou au moins un autre antiphlogistique non-stéroïdien, choisi en particulier dans le groupe des AINS ou le paracétamol, en particulier pour renforcer l'efficacité des principes actifs utilisés en association, en particulier avec pour objectif l'amélioration et la réduction de l'évolution de maladies de refroidissement.

13. Agent thérapeutique en association pour utilisation selon l'une des revendications 1 à 8,
pour la co-médication et/ou en tant que traitement d'appoint, en particulier sous forme d'un kit, avec au moins un autre principe actif, le principe actif étant choisi dans le groupe consistant en les glucocorticostéroïdes systémiques (SCS), les traitements substitutifs intraveineux avec de l'α₁-antitrypsine, des immunoglobulines IgG, en particulier lors d'un syndrome de carence en anticorps, les inhibiteurs de la phosphodiestérase, en particulier le roflumilast et/ou la théophylline, pour le traitement prophylactique et/ou thérapeutique de maladies des voies respiratoires supérieures, en particulier la rhinite et/ou la rhino-sinusite aiguë, allergique et/ou chronique, et/ou pour le traitement prophylactique et/ou thérapeutique de maladies des voies respiratoires inférieures, en particulier l'asthme, la BPCO et/ou l'emphysème pulmonaire chronique, et/ou pour le traitement prophylactique et/ou thérapeutique de maladies des voies respiratoires induites par la fumée de cigarette, les particules fines et/ou d'autres polluants environnementaux, et/ou qui y sont associées, en particulier avec pour objectif d'intensifier et/ou d'améliorer les effets de traitements par lignes directrices, tels que définis antérieurement, et/ou avec pour objectif une induction et/ou un renforcement d'effets permissifs des stéroïdes, en particulier pour la réduction de l'évolution progressive des obstructions et/ou des emphysèmes, des exacerbations et/ou du besoin en stéroïdes ; et/ou
pour la compensation de faible niveau de principe actif de vitamine D₃ et/ou d'un faible niveau de principe actif du 1,8-cinéol, en particulier dans lequel l'agent thérapeutique en association se présente sous forme d'un kit, pour la prophylaxie, le traitement et/ou le ralentissement de l'évolution progressive, en particulier lors de maladies inflammatoires de l'intestin, en particulier graves, en particulier les maladies inflammatoires à activité chronique de l'intestin et/ou pour la réduction de l'exacerbation, de la BPCO sévère, en particulier de l'emphysème pulmonaire avec ou sans insuffisance respiratoire, de l'asthme sévère, des maladies infectieuses sévères, des réactions allergiques, pour inhiber l'inflammation et économiser les stéroïdes, l'ostéoporose, les maladies cardiovasculaires, l'artériosclérose, les maladies malignes et le ralentissement des processus de vieillissement.

14. Agent thérapeutique en association pour utilisation selon l'une des revendications 1 à 8, en particulier dans lequel l'agent thérapeutique en association se présente sous forme d'un kit,
pour le traitement prophylactique et/ou thérapeutique des obstructions des voies respiratoires supérieures, ainsi que des réactions inflammatoires profibrotiques, en particulier lors de troubles de la respiration liés au sommeil, en particulier du syndrome d'apnée obstructive et centrale du sommeil ; et/ou
pour l'intensification et/ou l'amélioration de l'efficacité et/ou pour augmenter l'action de thérapies en ligne directrice de maladies inflammatoires chroniques de l'intestin, en particulier du syndrome de l'intestin irritable, de la maladie de Crohn et de la rectocolite hémorragique, en particulier avec pour objectif la prophylaxie, en particulier la prophylaxie de l'exacerbation, le maintien des rémissions et l'augmentation de l'effet anti-inflammatoire chez les enfants et les adultes.

15. Agent thérapeutique en association pour utilisation selon l'une des revendications 1 à 8, pour la réduction de la dose du 1,8-cinéol et/ou pour la réduction de la dose d'au moins un autre principe actif et/ou ingrédient pharmacologique, en particulier comme défini dans la revendication 4, et/ou pour la réduction de la dose d'au moins un principe actif pharmacologique pouvant être administré par voie systémique ou topique, en particulier d'un corticostéroïde, en particulier dans lequel la quantité et/ou la dose à administrer du principe actif considéré sont, indépendamment l'un de l'autre, diminuées d'au moins 5 %, en particulier d'au moins 10 %, de préférence d'au moins 20 %, préférentiellement d'au moins 40 %, par rapport à un agent thérapeutique correspondant sans antagoniste des récepteurs de la vitamine D (VDA).
